(19) **Europäisches Patentamt / European Patent Office / Office européen des brevets**

(11) **EP 4 084 609 B1**

(12) # EUROPEAN PATENT SPECIFICATION

(45) Date of publication and mention
of the grant of the patent:
**10.12.2025 Bulletin 2025/50**

(21) Application number: **20838029.5**

(22) Date of filing: **18.12.2020**

(51) International Patent Classification (IPC):
*A01K 67/027* (2024.01)  *A61K 9/16* (2006.01)
*A61K 31/573* (2006.01)  *C07K 14/78* (2006.01)
*A61K 38/39* (2006.01)  *A61P 27/06* (2006.01)

(52) Cooperative Patent Classification (CPC):
(C-Sets available)
**A01K 67/027; A61K 31/573; A61K 38/39;
A61P 27/06; C07K 14/78;** A01K 2207/20;
A01K 2227/105; A01K 2267/035; A61K 9/1647
(Cont.)

(86) International application number:
**PCT/EP2020/087153**

(87) International publication number:
**WO 2021/136697 (08.07.2021 Gazette 2021/27)**

(54) **RODENT MODEL OF CHRONIC GLAUCOMA**

NAGETIERMODELL VON CHRONISCHEM GLAUKOM

MODÈLE DE RONGEUR DE GLAUCOME CHRONIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.12.2019 EP 19383213**

(43) Date of publication of application:
**09.11.2022 Bulletin 2022/45**

(73) Proprietors:
• **Universidad Complutense De Madrid**
**28040 Madrid (ES)**
• **Universidad De Zaragoza**
**50009 Zaragoza (ES)**
• **Instituto de Investigación Sanitaria Aragón**
**50009 Zaragoza (ES)**

(72) Inventors:
• **HERRERO VANRELL, María, Rocío**
**28040 Madrid (ES)**
• **GARCÍA MARTÍN, Elena**
**50009 Zaragoza (ES)**
• **RODRIGO SANJUÁN, Maria, Jesus**
**50009 Zaragoza (ES)**

• **GARCÍA HERRANZ, David**
**28040 Madrid (ES)**
• **ARAGÓN NAVAS, Alba**
**28040 Madrid (ES)**
• **BRAVO OSUNA, Irene**
**28040 Madrid (ES)**
• **GARCÍA FEIJOO, Julian**
**28040 Madrid (ES)**
• **PABLO JÚLVEZ, Luis**
**50009 Zaragoza (ES)**

(74) Representative: **Herrero & Asociados, S.L.
Edificio Aqua - Calle Agustín de Foxá, 4-10, 2ºA
28036 Madrid (ES)**

(56) References cited:
**EP-B1- 2 329 811    WO-A2-2005/107705**

• **BUNKER SHANNON ET AL: "Experimental
Glaucoma Induced by Ocular Injection of
Magnetic Microspheres", JOURNAL OF
VISUALIZED EXPERIMENTS, no. 96, 2 February
2015 (2015-02-02), XP055781640, Retrieved from
the Internet <URL:https://www.ncbi.nlm.nih.gov/
pmc/articles/PMC4354616/pdf/jove-96-52400.
pdf> DOI: 10.3791/52400**

- BARCIA E ET AL: "Downregulation of endotoxin-induced uveitis by intravitreal injection of polylactic-glycolic acid (PLGA) microspheres loaded with dexamethasone", EXPERIMENTAL EYE RESEARCH, ACADEMIC PRESS LTD, LONDON, vol. 89, no. 2, 1 August 2009 (2009-08-01), pages 238 - 245, XP026336939, ISSN: 0014-4835, [retrieved on 20090331], DOI: 10.1016/J.EXER.2009.03.012
- A. BOUHENNI RACHIDA ET AL: "Animal Models of Glaucoma", JOURNAL OF BIOMEDICINE AND BIOTECHNOLOGY, vol. 2012, 2012, pages 1 - 11, XP055781714, ISSN: 1110-7243, DOI: 10.1155/2012/692609
- SMEDOWSKI ADRIAN ET AL: "A rat experimental model of glaucoma incorporating rapid-onset elevation of intraocular pressure", SCIENTIFIC REPORTS, vol. 4, no. 1, August 2014 (2014-08-01), XP055781648, Retrieved from the Internet <URL:http://www.nature.com/articles/srep05910.pdf> DOI: 10.1038/srep05910
- OVERBY DARRYL R ET AL: "Animal models of glucocorticoid-induced glaucoma", EXPERIMENTAL EYE RESEARCH, ACADEMIC PRESS LTD, LONDON, vol. 141, 4 June 2015 (2015-06-04), pages 15 - 22, XP029318488, ISSN: 0014-4835, DOI: 10.1016/J.EXER.2015.06.002
- WHITLOCK N. ANDREW ET AL: "Increased Intraocular Pressure in Mice Treated with Dexamethasone", INVESTIGATIVE OPTHALMOLOGY & VISUAL SCIENCE, vol. 51, no. 12, December 2010 (2010-12-01), pages 6496, XP055781519, ISSN: 1552-5783, Retrieved from the Internet <URL:http://dx.doi.org/10.1167/iovs.10-5430> DOI: 10.1167/iovs.10-5430
- ZAREI-GHANAVATI SIAMAK ET AL: "Preparation, Characterization, and In Vivo Evaluation of Triamcinolone Acetonide Microspheres After Intravitreal Administration", JOURNAL OF OCULAR PHARMACOLOGY AND THERAPEUTICS., vol. 28, no. 5, October 2012 (2012-10-01), US, pages 502 - 506, XP055781744, ISSN: 1080-7683, DOI: 10.1089/jop.2011.0215
- TURTURRO SANJA ET AL: "Sustained Release of Matrix Metalloproteinase-3 to Trabecular Meshwork Cells Using Biodegradable PLGA Microparticles", MOLECULAR PHARMACEUTICS, vol. 10, no. 8, 18 July 2013 (2013-07-18), US, pages 3023 - 3032, XP055781722, ISSN: 1543-8384, Retrieved from the Internet <URL:https://pubs.acs.org/doi/pdf/10.1021/mp4001052> DOI: 10.1021/mp4001052
- THACKABERRY EVAN A. ET AL: "Evaluation of the Toxicity of Intravitreally Injected PLGA Microspheres and Rods in Monkeys and Rabbits: Effects of Depot Size on Inflammatory Response", INVESTIGATIVE OPTHALMOLOGY & VISUAL SCIENCE, vol. 58, no. 10, 29 August 2017 (2017-08-29), pages 4274, XP055781596, ISSN: 1552-5783, DOI: 10.1167/iovs.16-21334
- RODRÍGUEZ VILLANUEVA JAVIER ET AL: "Optimising the controlled release of dexamethasone from a new generation of PLGA-based microspheres intended for intravitreal administration", EUROPEAN JOURNAL OF PHARMACEUTICAL SCIENCES, ELSEVIER AMSTERDAM, NL, vol. 92, 14 March 2016 (2016-03-14), pages 287 - 297, XP029666062, ISSN: 0928-0987, DOI: 10.1016/J.EJPS.2016.03.012
- FARALLI JENNIFER A. ET AL: "Role of Fibronectin in Primary Open Angle Glaucoma", CELLS, vol. 8, no. 12, 1 December 2019 (2019-12-01), CH, pages 1518, XP055781711, ISSN: 2073-4409, DOI: 10.3390/cells8121518

(52) Cooperative Patent Classification (CPC): (Cont.)

C-Sets
A61K 31/573, A61K 2300/00;
A61K 38/39, A61K 2300/00

**Description**

## FIELD OF THE INVENTION

The present invention belongs to the field of research tools for glaucoma, more specifically it relates to a non-human animal model of chronic glaucoma wherein the animal has intraocular Polylactic-co-glycolic acid (PLGA), Polylactic acid (PLA) or Polyglicolic acid (PGA) microparticles, having a particle size between 5 $\mu$m and 40 $\mu$m in the anterior chamber of the eye of the animal in order to induce an increase in intraocular pressure resulting in a chronic gradual progressive neuroretinal degeneration from a functional-structural point of view and wherein the animal is a rodent. In addition, the invention refers to a method for the preparation of said animal model, as well as to the use of thereof.

## BACKGROUND OF THE INVENTION

Glaucoma is a degenerative optic neuropathy in which irreversible vision loss is produced by the gradual death of retinal ganglion cells (RGC), although affectation in other retinal layers has also been observed in recent studies (Vidal-Sanz et al., Prog. Brain. Res. 2015; 220:1-35). According to the World Health Organization (WHO), it is the second leading cause of irreversible blindness in the world and the first in developed countries, with over 61 million people affected, although it is estimated that this prevalence is actually 20-25% higher due to frequent undiagnosed cases, as this pathology is asymptomatic until its late stages. It is believed that 7 million glaucoma patients have already lost their vision. Over 2 million cases are registered in the world every year, and the pathology is expected to affect 80 million people (according to "World Glaucoma Association" data). In response to the growing disease burden derived from chronic ocular conditions, the WHO coordinates a worldwide research attempt focused on identifying services and policies to fight neurodegenerative pathologies, being glaucoma among them.

The main modifiable risk factor which is currently known is intraocular pressure (IOP) increase, which hinders blood supply to the retina, compromising it by the pressure excess. This damages neural structures with optic nerve atrophy. Furthermore, it is argued that the pressure increase in the optic nerve connective tissues interrupts the axoplasmatic flow, blocking the arrival of endogenous neurotrophic factors to the neuronal body from the axons (Pease et al., Invest. Ophtalmol. Vis. Sci., 2000, 41(3):764-774). Although it is considered there is risk of suffering from glaucoma when IOP is high, not every patient with high IOP develops glaucoma, nor a decrease in IOP assures protection against the development of the disease (Ritch et al., Ophtalmol. Clin. North Am., 2005, 18(4):597-609). Therefore, in the last decade other important factors in the genesis and the development of neuronal degeneration in the retina have been studied, proving that the neurodegeneration process can be described, chronologically, in three steps:

1. Primary axonal damage;
2. Death of damaged neurons;
3. Damage and subsequent death of adjacent neurons, which is known as 'secondary degeneration'. This degeneration occurs in neurons which are not damaged initially, but they end up dying due to exposure to cytotoxic agents released by the death of neurons with primary axonal damage.

There are different types of glaucoma, being primary open-angle glaucoma (OAG) the most frequent as well as one of the most usual causes of blindness in the world. It is characterized by slow and progressive clinical process due to the fact that gradual and chronic IOP increase does not produce pain or discomfort and, at the first stages, loss of visual field is not perceptible by patients, although as it develops it causes malfunctions in the visual field and progressive vision loss. Once these symptoms appear, they are irreversible and might imply the disease is in an advanced stage of its evolution. The main therapy is based on reducing IOP with hypotensive eyedrops, drainage implants or surgery, depending on stage and severity.

In addition, physiopathological studies of OAG showed that once neuronal death starts, even when patients present IOP between limits considered normal, a flood of damaging proinflammatory and proapoptotic substances of RGC is unleashed, which causes the death of the adjacent neurons, known, as mentioned before, as secondary degeneration (Ritch et al., Ophthalmol. Clin. North Am., 2005, 18(4):597-609). Therefore, the use of neuroprotective therapies in the treatment of glaucoma results in an alternative way to therapies based on IOP control, which are sometimes deficient in many glaucomatous pathologies and in patients with normal IOP values.

The main problem to evaluate the efficacy of hypotensive treatments or to develop new therapies resides in the absence of a chronic and slow glaucoma animal model that simulates a human one. Nowadays most animal models in retinal degeneration are acute models, either by genetic failure or induced damage, obtaining abrupt deterioration of the tissue in few weeks. On the one hand, these models do not reproduce the reality of retinal pathologies in human beings, whose nature is chronic and where retinal damage usually takes years to appear (Dey et al Cell Transplant. 2018 Feb;27(2):213-229, Mukai et al PLoS ONE. 2019. 14(1): e02087132019). On the other hand, acute models of degeneration are not useful to assess modified release systems, which present as great potential their capacity to extend the release of active substances in small quantities for months (Nadal-Nicolás et al., Invest. Ophtalmol.Vis. Sci. 2016; 57(3):1183-92). Furthermore, these acute glaucoma animal models do not allow testing the efficacy of new therapies (hypotensive, neuronal protective, etc.) because in few days the optic nerve of the animal is completely and irreversibly atrophied and therefore no treatment has enough time to stop the disease progression.

In order to broaden and improve the knowledge of glaucomatous pathologies, several animal models have been developed in the last decades (Dey et al Cell Transplant. 2018 Feb;27(2):213-229), in which it has been resorted to an increase in IOP secondary to a decrease in the flow of aqueous humor, either through cauterization, ligature and/or sclerosis of episcleral veins, either by mechanical blockage of the trabecular meshwork with non-biodegradable particles injected in the anterior chamber, or through the use of corticoids (which reduce aqueous humor outflow when inhibiting cellular phagocytosis in the trabecular meshwork, thus avoiding the cleaning of the waste channels (Zeng et al. Current Eye Research 2019). The model of episcleral sclerosis using a hypertonic saline solution has proved to increase IOP in a sustained manner with retinotopic death of RGC (Morrison et al., Exp. Eye Res. 1997, 64:84-96; Vecino et al., Glaucoma Basic and Clinical Concepts 2011, First edition, Croatia Intech:319-334; Chen et al., Invest. Ophtalmol.Vis. Sci.2011, 52(1):5-16), albeit acute optic nerve atrophy appears in the animal in merely few weeks.

Further animal models of glaucoma have been reported in the prior art so far (Struebing el al Prog. Mol. Biol. Trans. Sci. 2015; Bouhenni et al. J. Biomed. Biotech., 2012; Agarwal et al Expert Opin Drug Discov. 2017; Biswas and Wan Acta Ophthalmol. 2019) but no research group has achieved the creation of an animal model which mimics OAG and allows assessing new reliable therapies.

The present invention represents a new model of chronic animal glaucoma based on the injection of biodegradable microspheres (Ms) of PLGA, PLA or PGA (poly-lactic-co-glycolic-acid, polylactic acid or polyglycolic acid) in the anterior chamber of the eye in a mammal, preferably a rat, which produces a progressive and chronic increase of IOP and consequently, a chronic neurodegenerative process which simulates the conditions which appear in chronic glaucoma patients. Until now, biodegradable microparticles had never been used for this purpose. Biodegradable microparticles produce mechanical blockage in the trabecular meshwork which can be accompanied by a pharmacological effect if the particles are loaded with pharmacological agents able to provide damage at this level. All these events cause a slow, persistent and progressive rise of IOP with slow retinal degeneration of the ganglion cell layer and the optic nerve, simulating OAG physiopathology.

This invention allows to use biodegradable microspheres not only as active substance administration systems (Garcia-Caballero et al. Eur J Pharm Sci. 2017a;103:19-26) but also as a useful tool to create ocular hypertensive animal models which are able to reproduce chronic human pathologies.

## BRIEF DESCRIPTION OF THE FIGURES

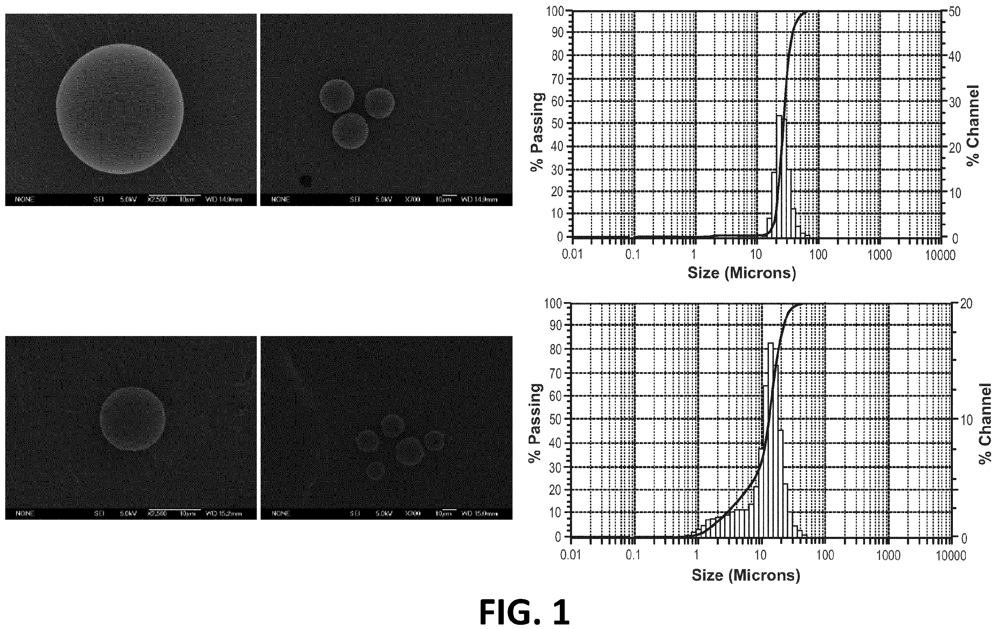

**Figure 1: Scanning electron microscopy picture and particle size distribution of PLGA microspheres.** 38-20 $\mu$m Ms (up); 20-10 $\mu$m Ms (botton).

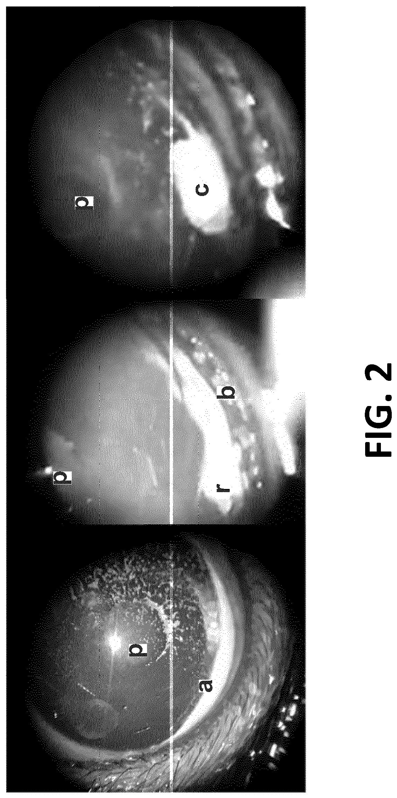

**Figure 2: PLGA microspheres behavior in the anterior chamber of rat eye over follow-up.** P: pupil; r: light reflex; a: microspheres at inferior iridocorneal angle; b: remnants of microspheres; c: pellet of microspheres.

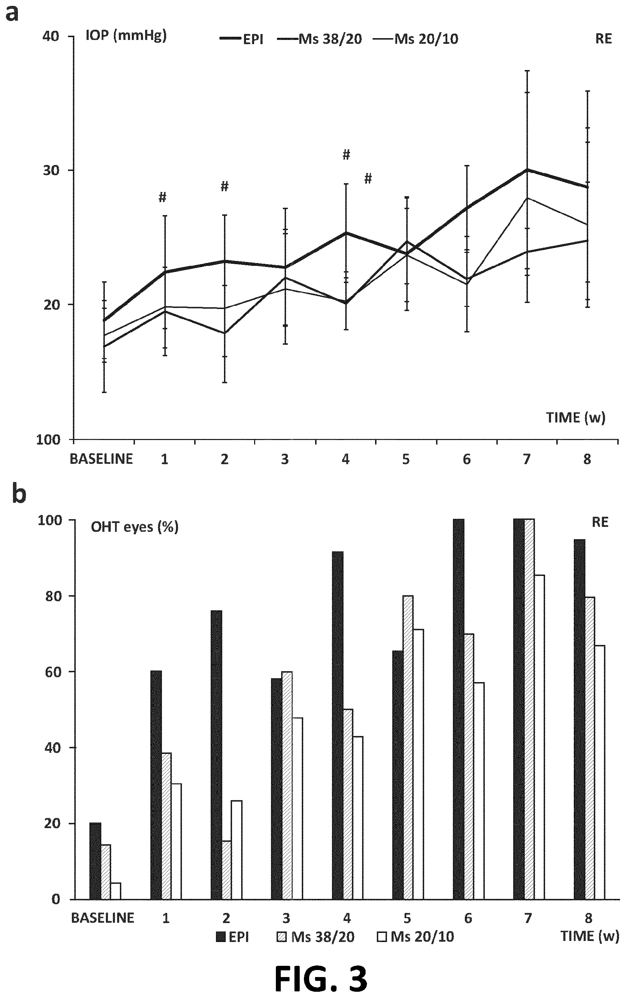

**Figure 3: a: Right eye intraocular pressure curve in the ocular hypertensive models over follow-up. b: Right eye percentage of ocular hypertensive eyes (>20mmHg) in the three ocular hypertensive models over follow-up.** EPI: epiescleral sclerosis model; Ms 38/20: microsphere sized 38/20 model, Ms 20/10: microsphere sized 20/10 model; RE: right eye; IOP: intraocular pressure; w: weeks, OHT: ocular hypertension; (%): percentage; *: statistical significance p<0.050; #: statistical significance p<0.020, for Bonferroni correction for multiple comparisons.

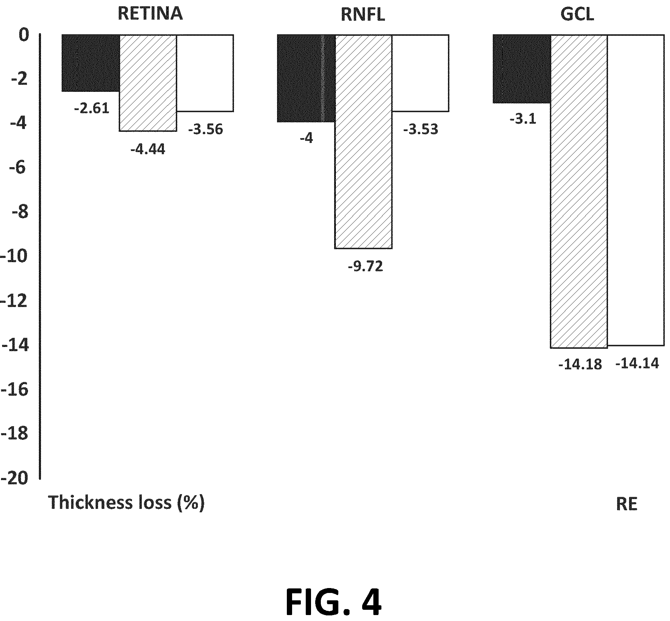

**Figure 4: Percentage thickness loss of neuro-retinal structure by optical coherence tomography (OCT) in the ocular hypertensive models. EPI:** epiescleral sclerosis model; Ms 38/20: microsphere sized 38/20 model, Ms 20/10: microsphere sized 20/10 model RNFL: Retinal Nerve Fiber Layer; GCL: Ganglion cell layer complex; RE: right eye; (%): percentage; OCT: optical coherence tomography.

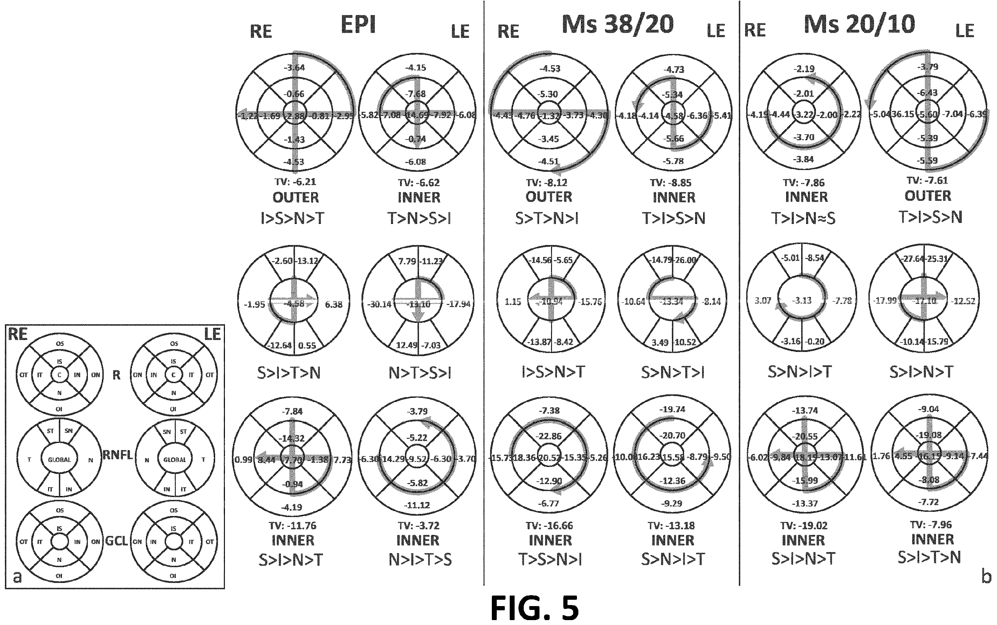

**Figure 5: a: Nomenclature of the neuro-retinal sectors by optical coherence tomography (OCT). b: Neuro-retinal percentage thickness loss in OCT sectors, and loss trend at 8 week follow-up.** Abbreviations: R: retina; C: central, II: inner inferior, OI: outer inferior; IS: inner superior; OS: outer superior; IN. inner nasal, ON: outer nasal; IT: inner temporal; OT: outer temporal; RNFL: Retinal Nerve Fiber Layer; IT: inferior temporal; IN: inferior nasal; ST: superior temporal; SN: superior nasal; N: nasal, T: temporal; GCL: Ganglion cell layer complex, OD: right eye; LE: left eye. EPI: epiescleral sclerosis model; Ms 38/20: microsphere sized 38/20 model, Ms 20/10: microsphere sized 20/10 model; TV: total volume; I: inferior; S. superior; N: nasal, T: temporal; >: higher loss than.

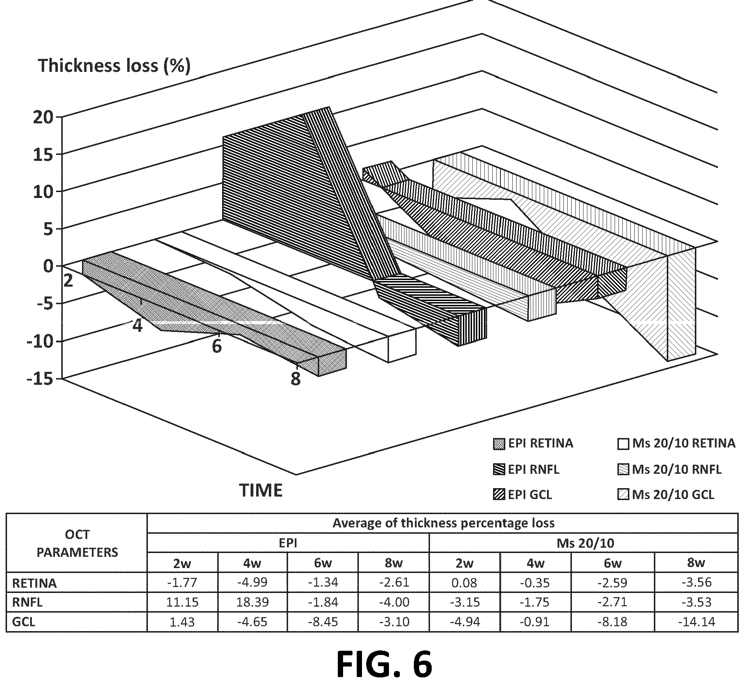

**Figure 6: Thickness percentage loss changes by optical coherence tomography (OCT) in two ocular hypertensive models.** EPI: epiescleral sclerosis model. Ms20/10: Microspheres sized 20/10 model. RNFL: Retinal Nerve Fiber Layer; GCL: Ganglion cell layer complex; w: week; %: percentage.

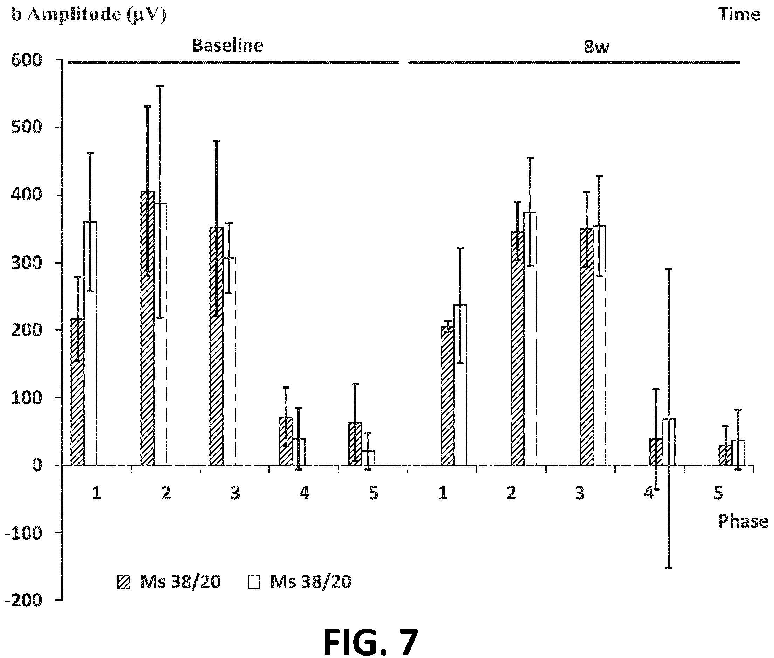

**Figure 7: Comparison of the functional neuro-retinal measurements of b-wave amplitude by scotopic electroretinogram (ERG) between both Ms ocular hypertensive models.** Ms 38/20: microsphere sized 38/20 model, Ms 20/10: microsphere sized 20/10 model w: week; $\mu$V: microvolts; b: b-wave expresses the functionality of intermediate bipolar cells.

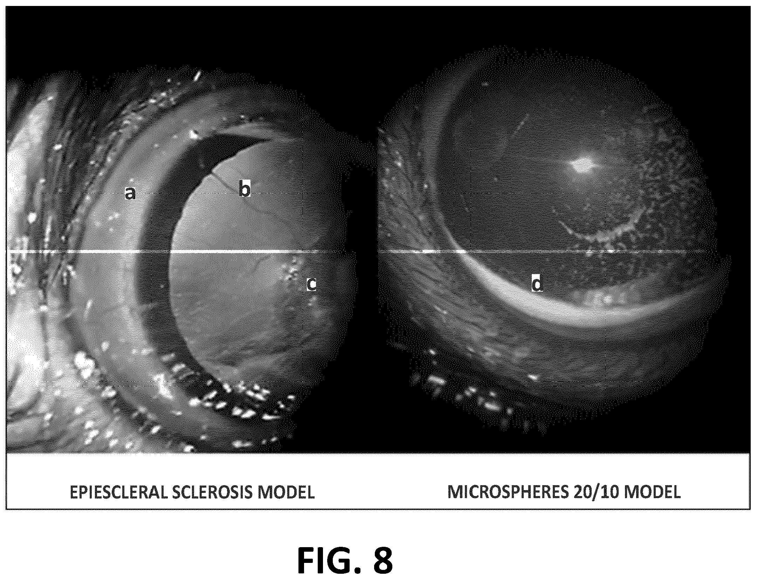

**Figure 8: Ocular surface of rat eye in ocular hypertensive models.** Episcleral sclerosis model. a: sclerosis of espiescleral veins, b: corneal neovascularization, c: corneal leucoma. Microsphere 20/10 model. d: microspheres of poly lactic-co-glycolic acid (PLGA) in the anterior chamber of the rat eye.

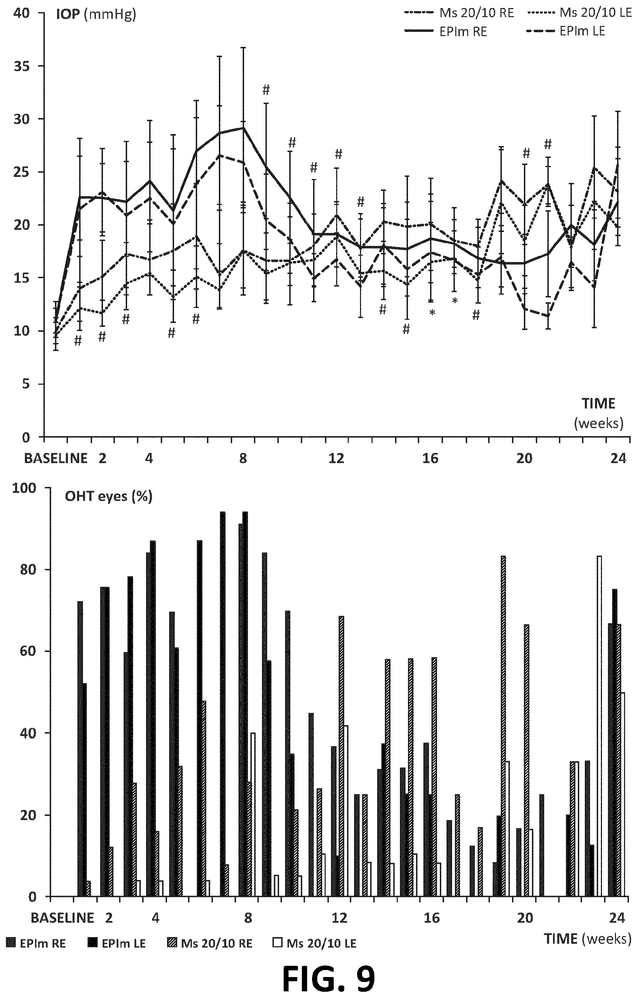

**Figure 9: a: Intraocular pressure comparison over 6 months, in both ocular hypertensive models.** Ms20/10: microspheres model; EPIm: epieslceral sclerosis model; RE: right eye; LE: left eye; IOP: intraocular pressure; *: statistical significance p<0.05 (EPIm in black and Ms20/10 in grey); #: statistical significance p<0.02, for Bonferroni correction for multiple comparisons (EPIm in black and Ms20/10 in grey). **b: Percentage of ocular hypertensive eyes (>20mmHg) in EPIm vs Ms20/10 models over6 months' follow-up.** Ms20/10: microspheres model; EPIm: epieslceral sclerosis model; RE: right eye; LE: left eye; OHT: ocular hypertension; %: percentage.

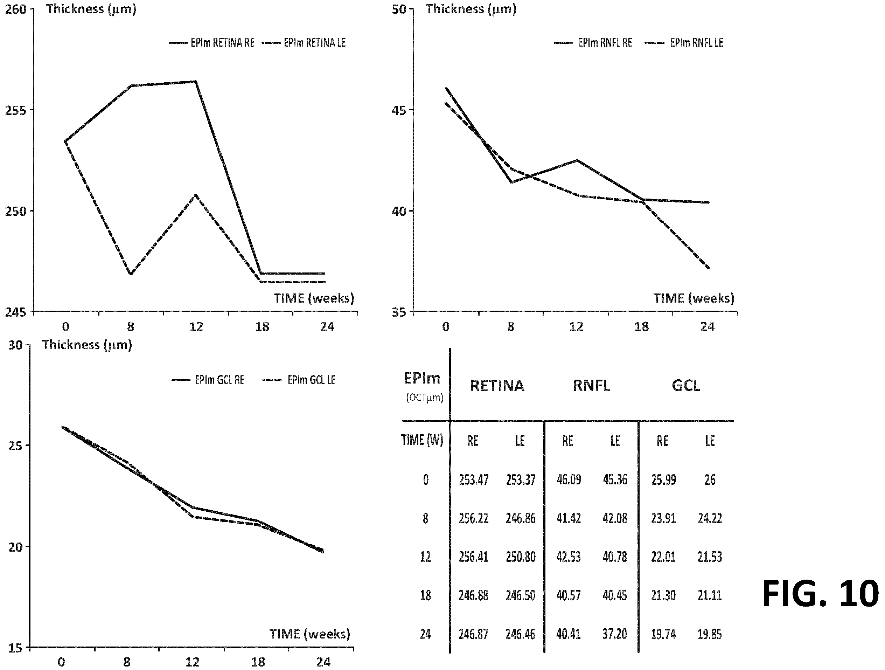

**Figure 10: Structural analysis of neuro-retina by optical coherence tomography in both eyes of the epiescleral model.** EPIm: epieslceral sclerosis model; RE: right eye; LE: left eye; OCT: optical coherence tomography; RNFL: Retinal Nerve Fiber Layer; GCL: Ganglion Cell Layer complex; average thickness in microns ($\mu$m).

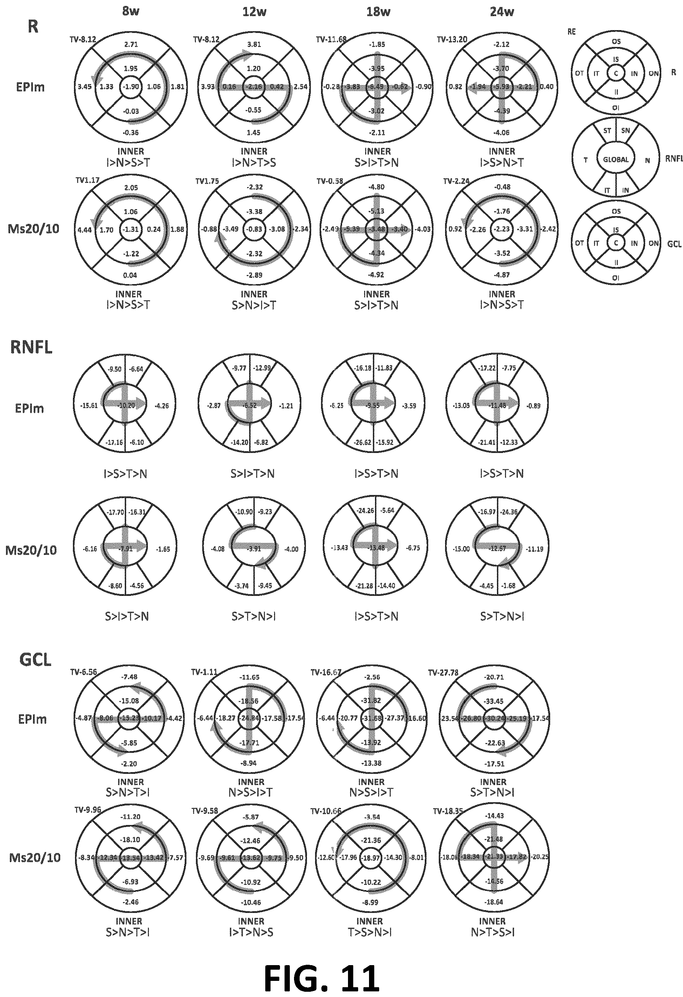

**Figure 11: Neuro-retinal percentage thickness loss by optical coherence tomography (OCT) sectors and loss trend in both ocular hypertensive models.** EPIm: epieslceral sclerosis model; Ms20/10: microspheres model; RE: right eye; R: retina; C: central, II: inner inferior, OI: outer inferior; IS: inner superior; OS: outer superior; IN. inner nasal, ON: outer nasal; IT: inner temporal; OT: outer temporal; RNFL: Retinal Nerve Fiber Layer; IT: inferior temporal; IN: inferior nasal; ST: superior temporal; SN: superior nasal; N: nasal, T: temporal; GCL: Ganglion Cell Layer complex; TV: total volume; I: inferior; S. superior; N: nasal, T: temporal; >: higher loss than.

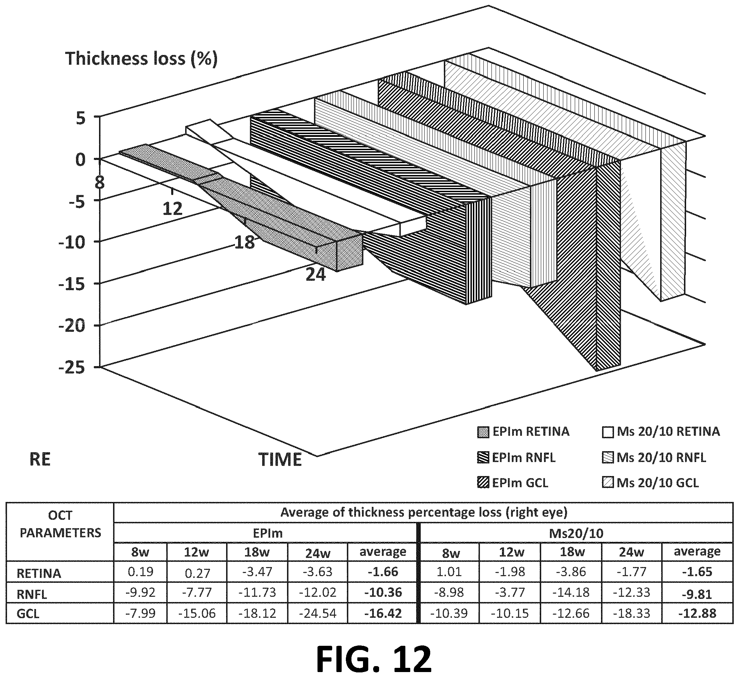

**Figure 12: Thickness percentage loss by optical coherence tomography (OCT) in both OHT models over 6 months follow-up.** Right eye (RE) analysis. EPIm: Episcleral sclerosis model. Ms20/10: Microspheres sized 20/10 model. RNFL: Retinal Nerve Fiber Layer; GCL: Ganglion cell layer complex; w: week; %: percentage.

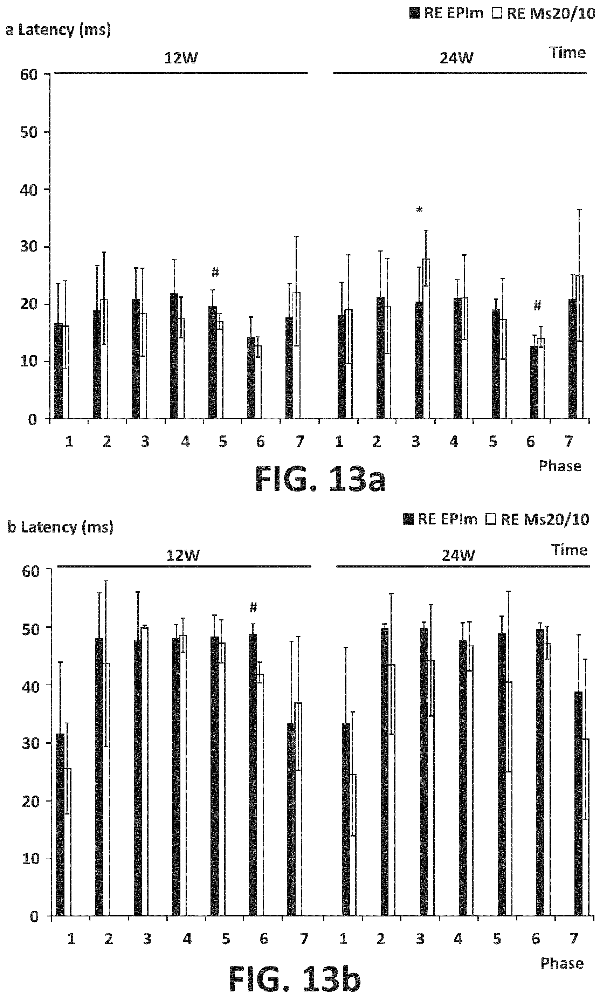

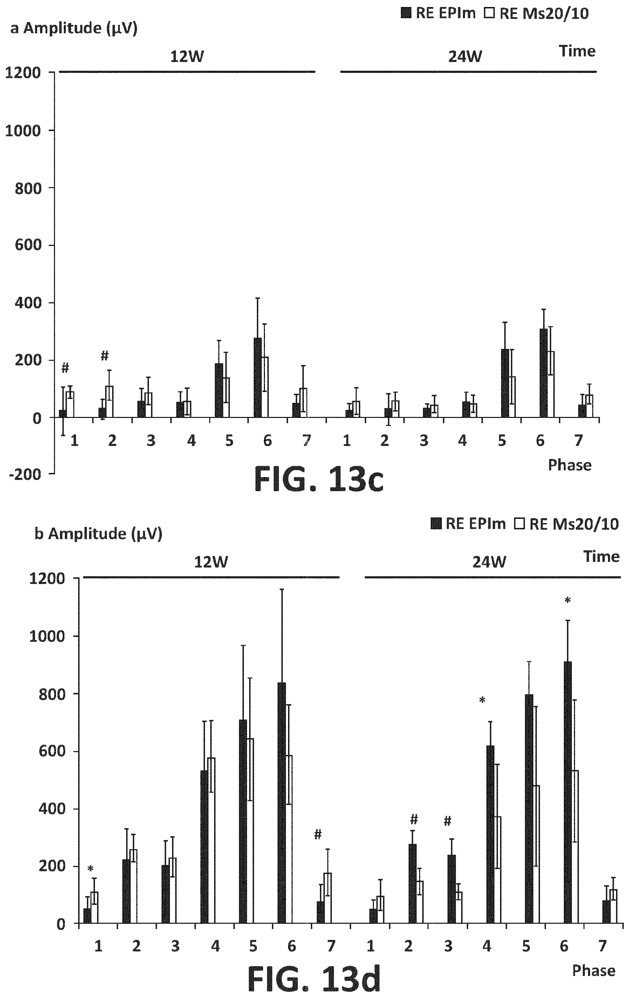

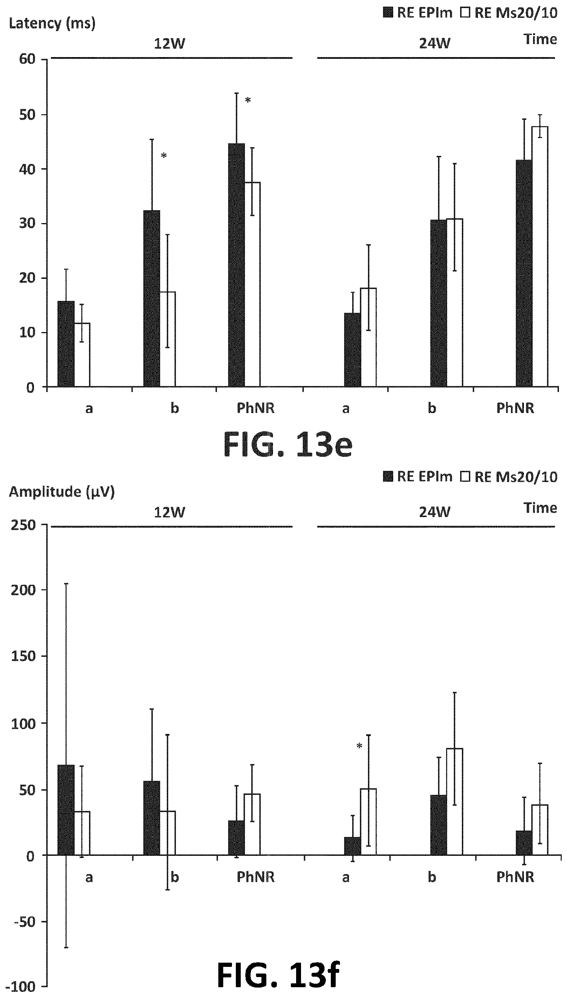

**Figure 13: Comparison of the functional neuro-retinal measurements by electroretinogram (ERG) between both ocular hypertensive models.** Scotopic electroretinogram (ERG); PhNR: Photopic Negative Response; w: week; ms: milliseconds; $\mu$V: microvolts; a: a-wave expresses the functionality of photoreceptors; b: b-wave expresses the functionality of intermediate cells; PhNR: PhNR-wave expresses the functionality of retinal ganglion cells. Phase 1: 0.0003 cds/m$^2$, 0.2Hz/s; phase 2: 0.003 cds/m$^2$, 0.125Hz/s; phase 3: 0.03 cds/m$^2$, 8.929Hz/s; phase 4: 0.03 cds/m$^2$, 0.111 Hz/s; phase 5:0.3 cds/m$^2$, 0.077Hz/s; phase 6: 3.0 cds/m$^2$, 0.067Hz/s; and phase 7: 3.0 cds/m$^2$, 29.412Hz/s explored by dark adaptation. PhNR explored by blue light; *: statistical significance p<0.05; #: statistical significance p<0.02, for Bonferroni correction for multiple comparisons.

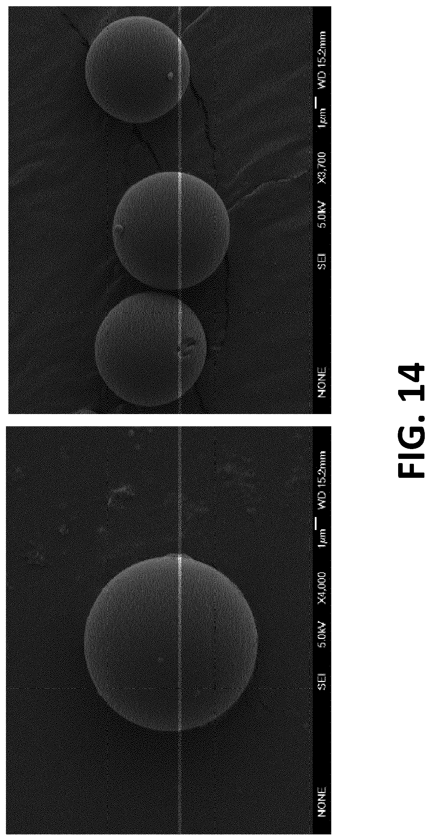

**Figure 14: Scanning electron microscopy (SEM) pictures of Dexamethasone-loaded poly lactic-co-glycolic acid microspheres (PLGA Ms).**

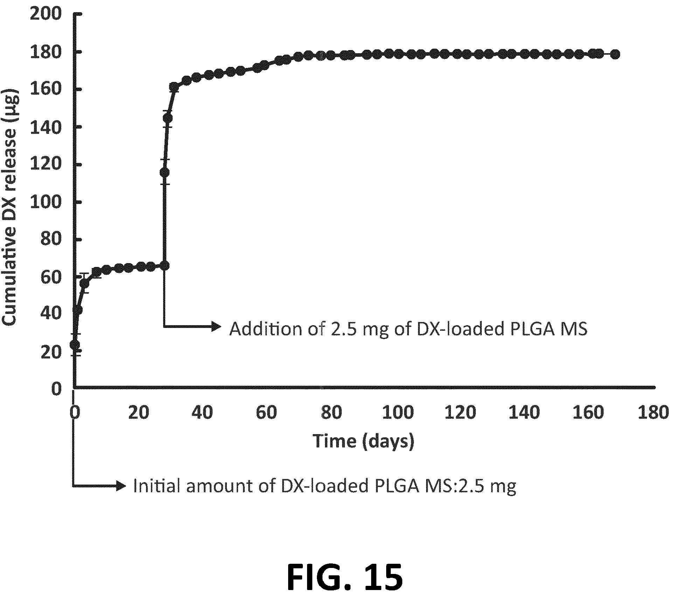

**Figure 15: Dexamethasone (DX)** *in vitro* **release profile from Dexamethasone-loaded poly lactic-co-glycolic acid microspheres (PLGA Ms).**

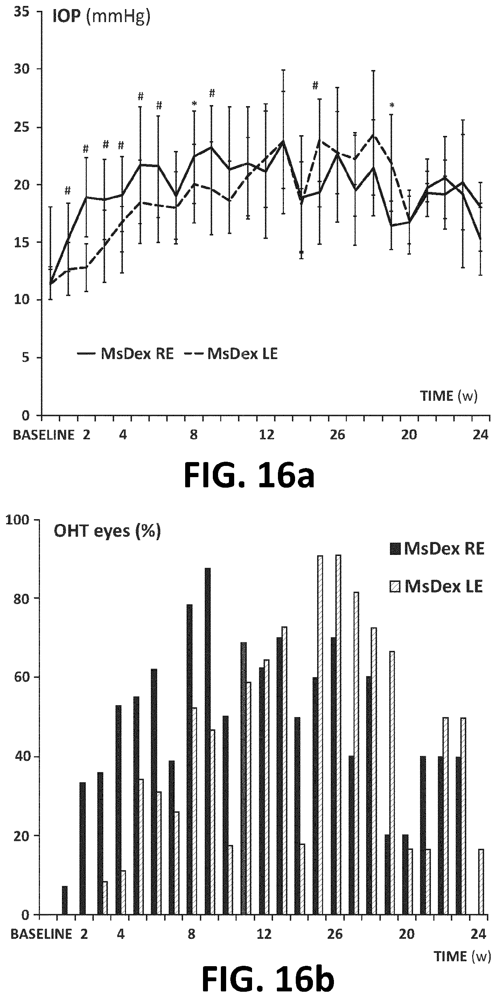

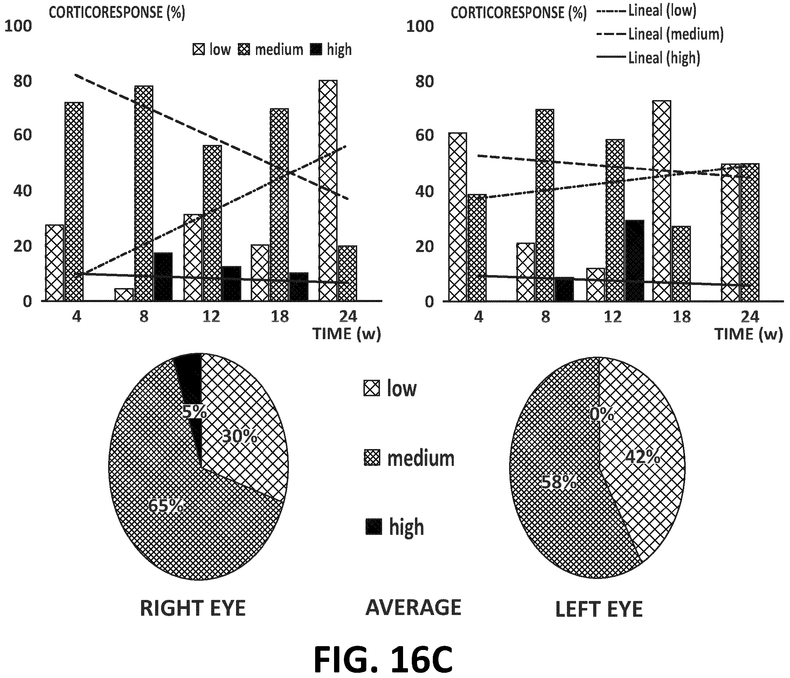

**Figure 16: a: Intraocular pressure curve over 6 months in microspheres loaded with dexamethasone (MsDex) model.** IOP: intraocular pressure; MsDex: microspheres loaded with dexamethasone; RE: right eye; LE: left eye; w: week; *: p<0.05; #: p<0.02. **b: Percentage of ocular hypertensive eyes (>20mmHg) MsDex model over 6 months' follow-up.** OHT: ocular hypertension; %: percentage; MsDex: microspheres loaded with dexamethasone; RE: right eye; LE: left eye; w: week. c: **Percentage of corticosteroid response in right and left eyes.** Low: <6 mmHg increase; medium: 6-15mmHg increase; high: >15mmHg increase.

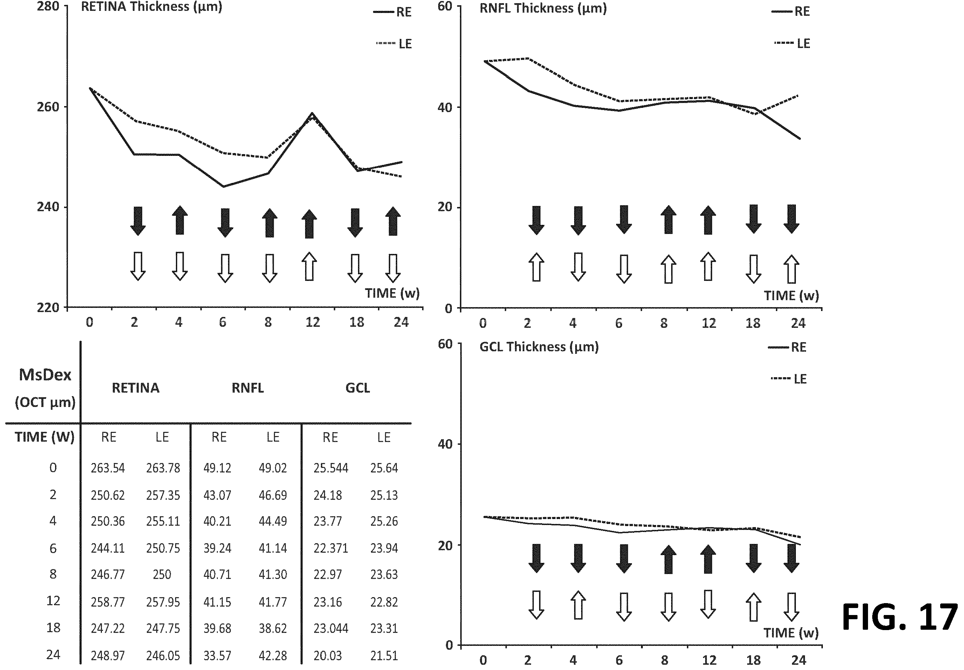

**Figure 17: Structural analysis of neuro-retina by optical coherence tomography (OCT) in both eyes of the microspheres loaded with dexamethasone (MsDex) model.** MsDex: microspheres loaded with dexamethasone; RE: right eye; LE: left eye; w: week; OCT: optical coherence tomography; RNFL: Retinal Nerve Fiber Layer; GCL: Ganglion Cell Layer complex; average thickness in microns ($\mu$m). Black arrows: from right eye; grey arrows from left eye; down arrow: decreasing thickness; up arrow: increasing thickness.

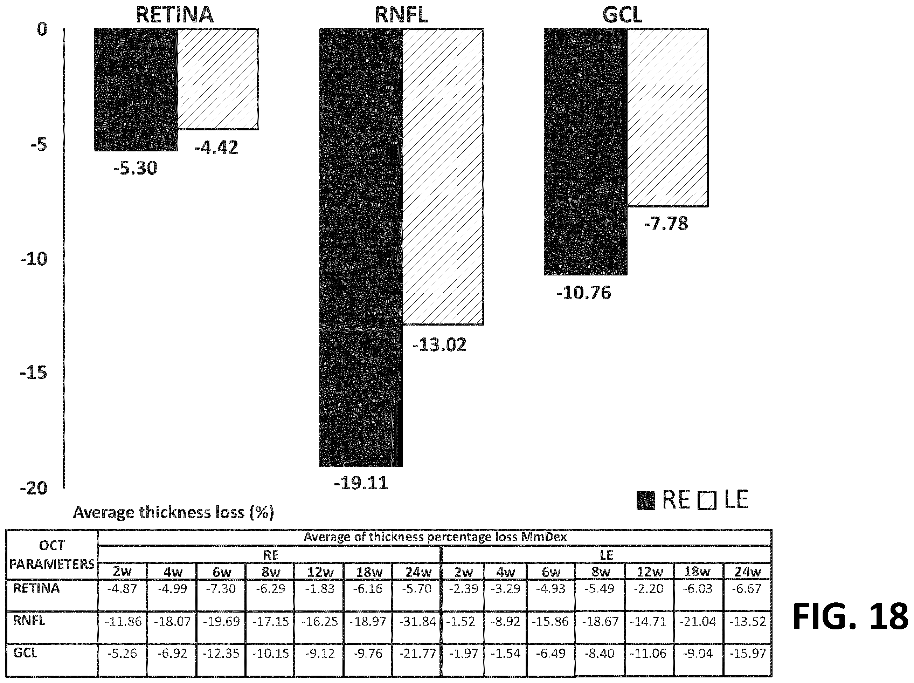

**Figure 18: Thickness percentage loss by optical coherence tomography (OCT) in microspheres loaded with dexamethasone (MsDex) model over 6 month follow-up.** MsDex: microspheres loaded with dexamethasone; RE: right eye; LE: left eye; w: week; OCT: optical coherence tomography; RNFL: Retinal Nerve Fiber Layer; GCL: Ganglion Cell Layer complex; %: percentage.

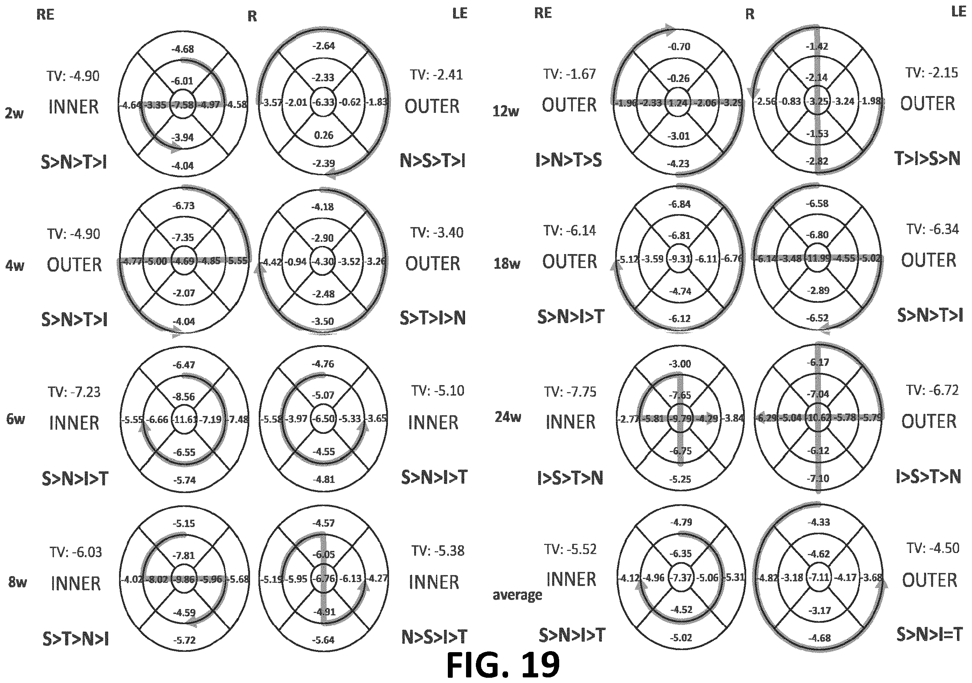

**Figure 19: Retinal percentage loss by optical coherence tomography (OCT) sectors and loss trend in microspheres loaded with dexamethasone (MsDex) model over 6 month follow-up.** R: retina; MsDex: microspheres loaded with dexamethasone; RE: right eye; LE: left eye; w: week; TV: total volume; S: superior; I: inferior; N: nasal; T: temporal; >: higher loss than.

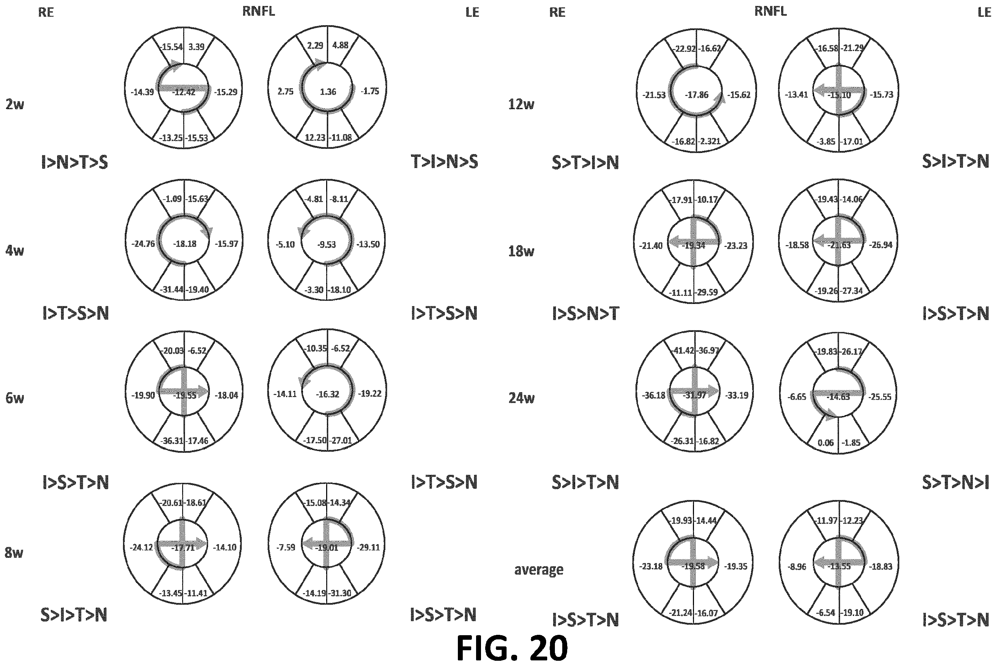

**Figure 20: Retinal nerve fiber layer percentage thickness loss by optical coherence tomography (OCT)**

**sectors and loss trend in microspheres loaded with dexamethasone (MsDex) model over 6 month follow-up.** RNFL: retinal nerve fiber layer; MsDex: microspheres loaded with dexamethasone; RE: right eye; LE: left eye; w: week; S: superior; I: inferior; N: nasal; T: temporal; >: higher loss than.

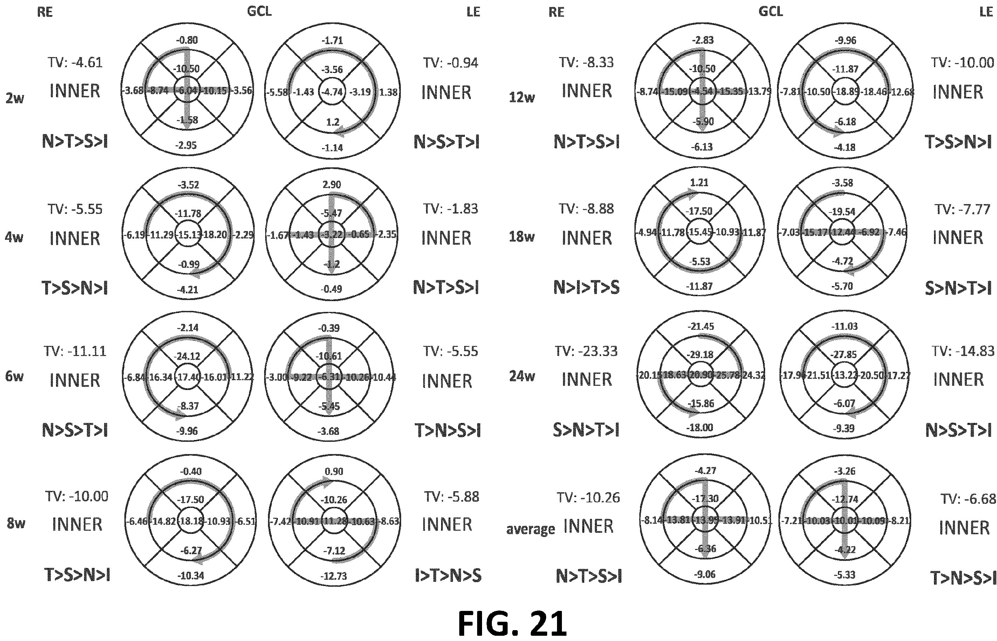

**Figure 21: Ganglion cell layer percentage thickness loss by optical coherence tomography (OCT) sectors and loss trend in microspheres loaded with dexamethasone (MsDex) model over 6 month follow-up.** GCL: ganglion cell layer complex; MsDex: microspheres loaded with dexamethasone; RE: right eye; LE: left eye; w: week; TV: total volume; S: superior; I: inferior; N: nasal; T: temporal; >: higher loss than.

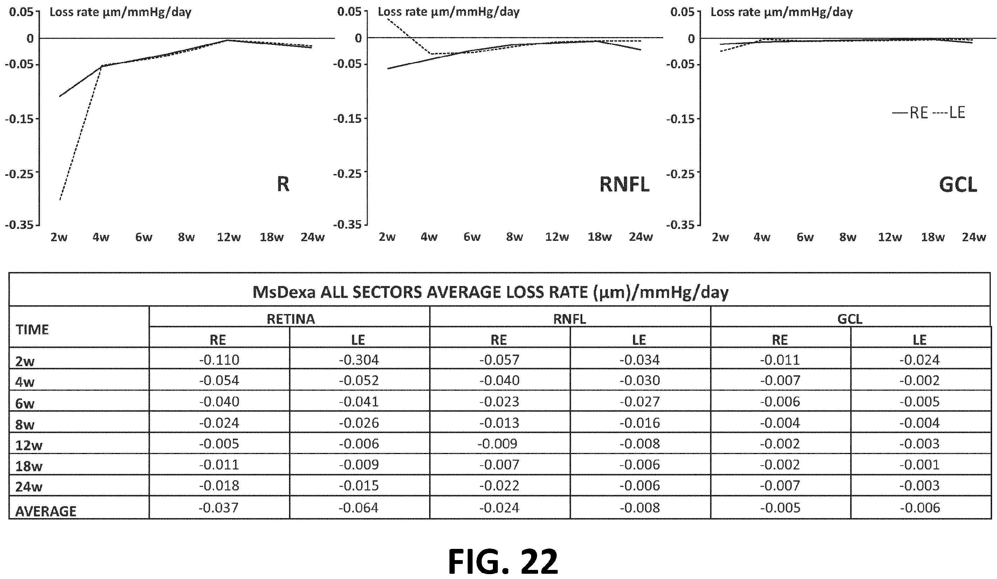

**Figure 22: Neuro-retinal thickness loss rate measured by optical coherence tomography (OCT) in microspheres loaded with dexamethasone (MsDex) model.** MsDex: microspheres loaded with dexamethasone; RE: right eye; LE: left eye; w: week; RNFL: Retinal Nerve Fiber Layer; GCL: Ganglion Cell Layer complex.

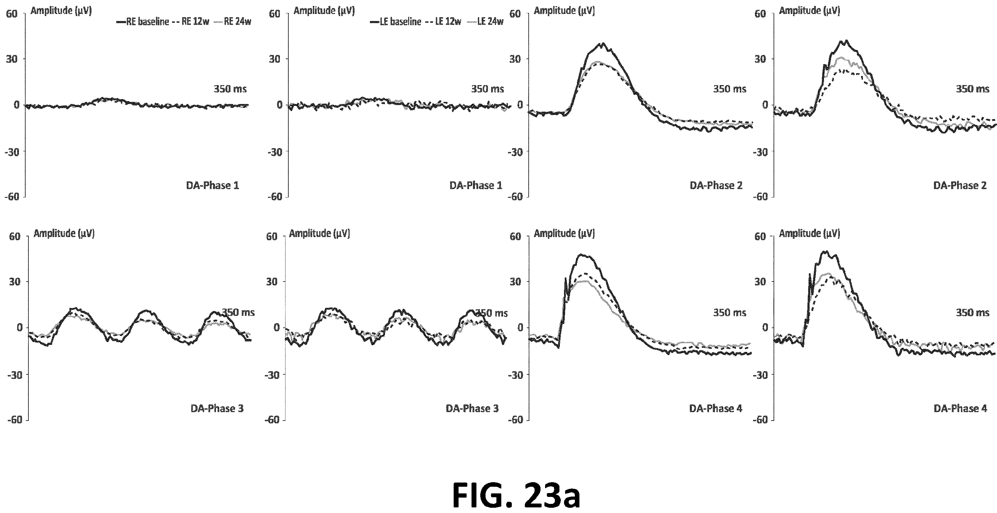

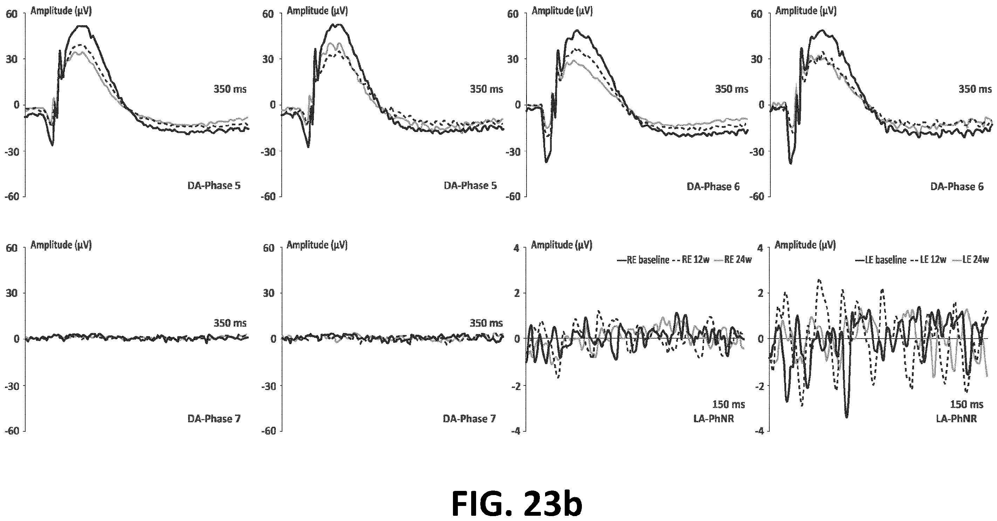

**Figure 23: Functionality of neuroretina by dark and light adapted electroretinography (ERG) in microspheres loaded with dexamethasone (MsDex) model over 6 month follow up.** MsDex: microspheres loaded with dexamethasone; RE: right eye; LE: left eye; w: week; DA: dark adapted; LA: Light adapted; $\mu$V: microvolts; ms: milliseconds.

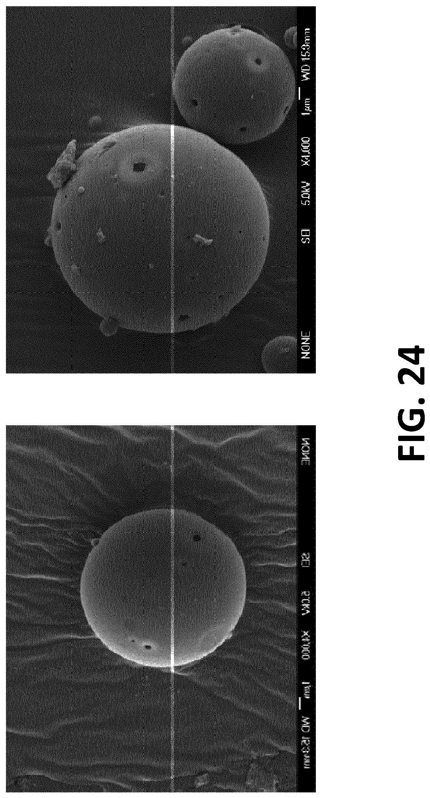

**Figure 24: Dexamethasone/fibronectine-loaded poly lactic-co-glycolic acid microspheres (MsDexaFibro) scanning electron microscopy images.**

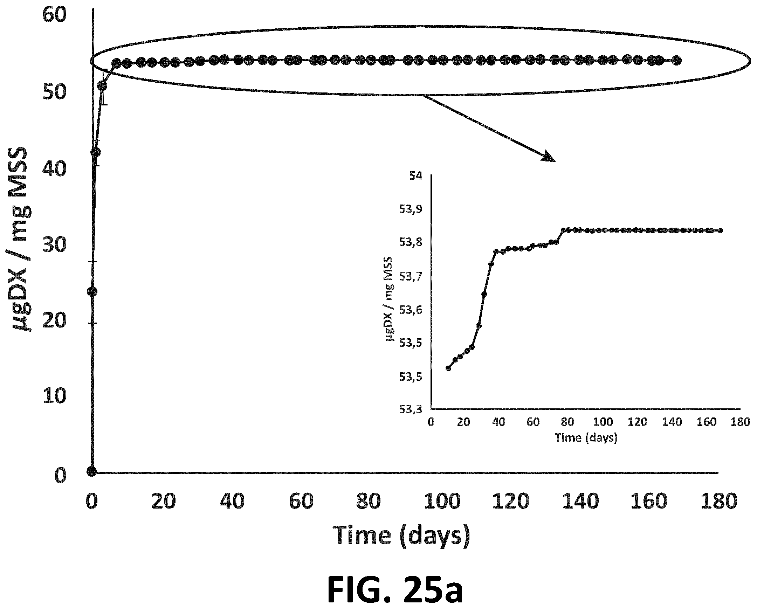

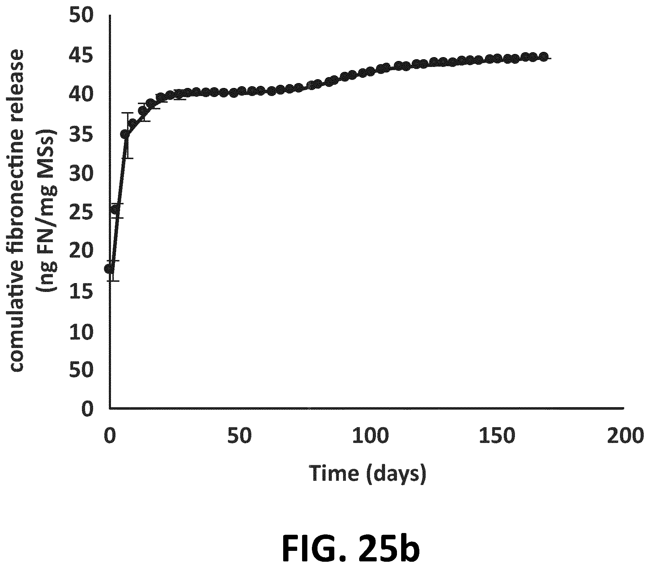

*Figure 25:* **Cumulative** *in vitro* **release of dexamethasone (Figure 25a) and fibronectine (Figure 25b) from Dexamethasone/fibronectine-loaded poly lactic-co-glycolic acid microspheres (MsDexaFibro).**

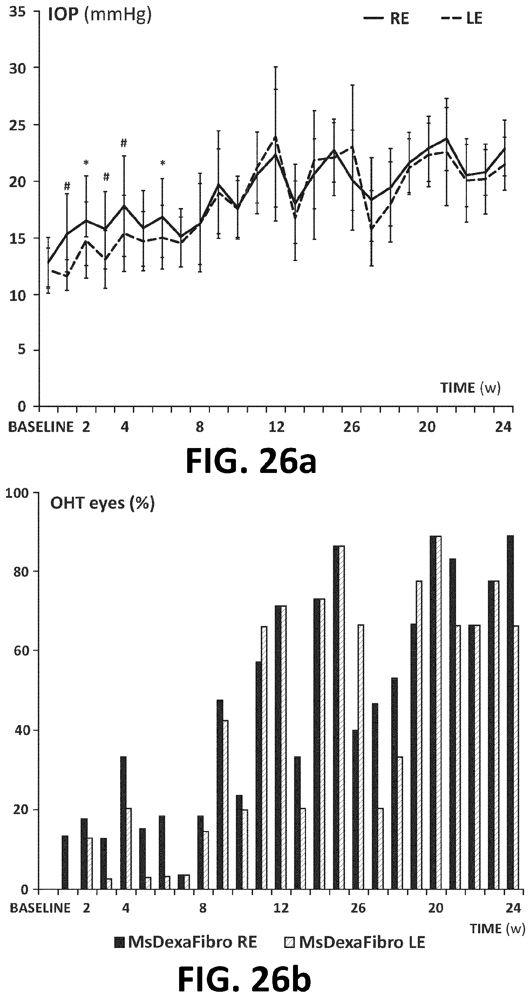

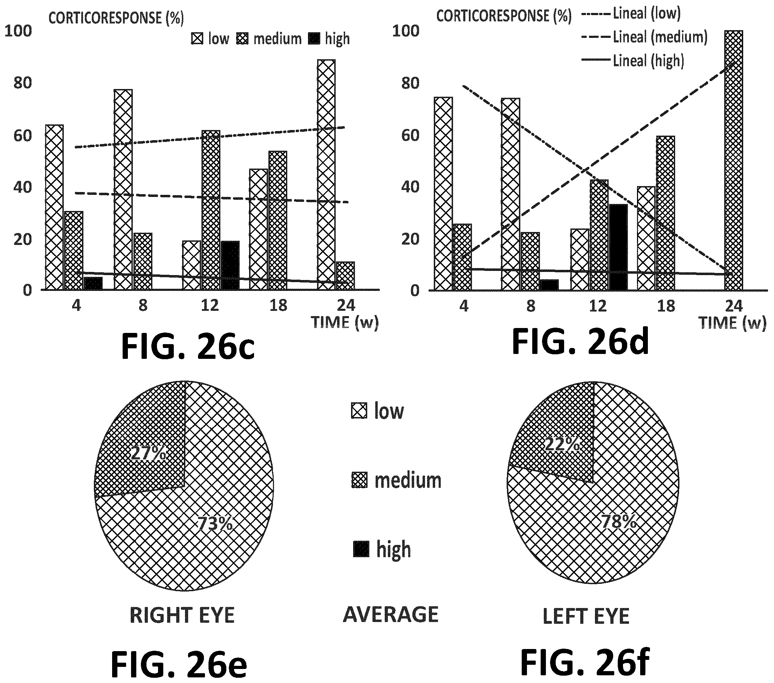

**Figure 26: a: Intraocular pressure curve over 6 months in microspheres co-loaded with dexamethasone and fibronectine (MsDexaFibro) model. IOP:** intraocular pressure; MsDexaFibro: microspheres co-loaded with dexamethasone and fibronectine; RE: right eye; LE: left eye; w: week; *: p<0.05; #: p<0.02 (Bonferroni Correction for multiple comparisons). **b: Percentage of ocular hypertensive eyes (>20mmHg) in MsDexafibro model over 6 months' follow-up.** OHT: ocular hypertension; %: percentage; MsDexaFibro: microspheres co-loaded with dexamethasone and fibronectine; RE: right eye; LE: left eye; w: week. **c: Percentage of corticosteroid response and tendency in right eyes over the study. d: Percentage of corticosteroid response and tendency in left eyes over the study e: Averaged percentage of corticosteroid response in right eyes. f: Averaged percentage of corticosteroid response in left eyes.** Low: <6 mmHg increase; medium: 6-15mmHg increase; high: >15mmHg increase.

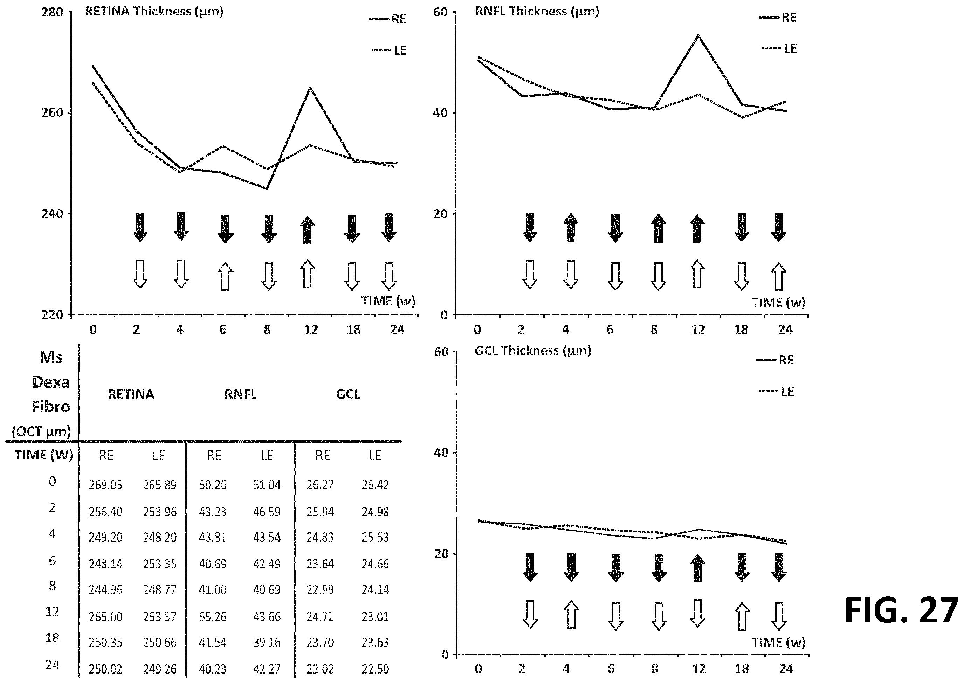

**Figure 27: Structural analysis of neuro-retina by optical coherence tomography (OCT) in both eyes of the microspheres co-loaded with dexamethasone and fibronectine (MsDexaFibro) model.** MsDexaFibro: microspheres co-loaded with dexamethasone and fibronectine; RE: right eye; LE: left eye; OCT: optical coherence tomography; RNFL: Retinal Nerve Fiber Layer; GCL: Ganglion Cell Layer complex; average thickness in microns ($\mu$m); w: week; down arrow: decreasing thickness; up arrow: increasing thickness.

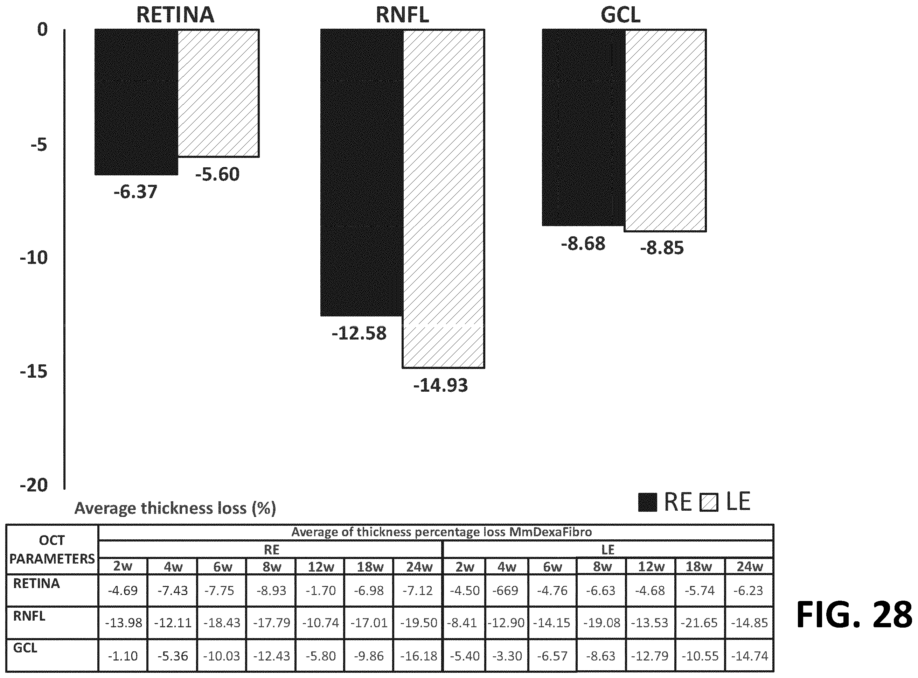

**Figure 28: Thickness percentage loss by optical coherence tomography (OCT) in microspheres co-loaded with dexamethasone and fibronectine (MsDexaFibro) model over 6 month follow-up.** MsDexaFibro: microspheres co-loaded with dexamethasone and fibronectine; RE: right eye; LE: left eye; w: week; OCT: optical coherence tomography; RNFL: Retinal Nerve Fiber Layer; GCL: Ganglion Cell Layer complex; %: percentage.

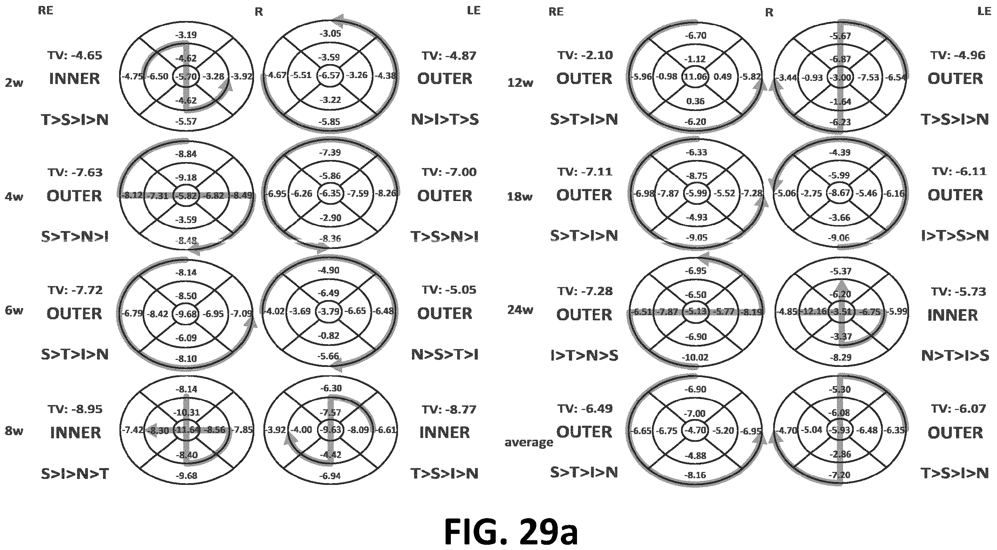

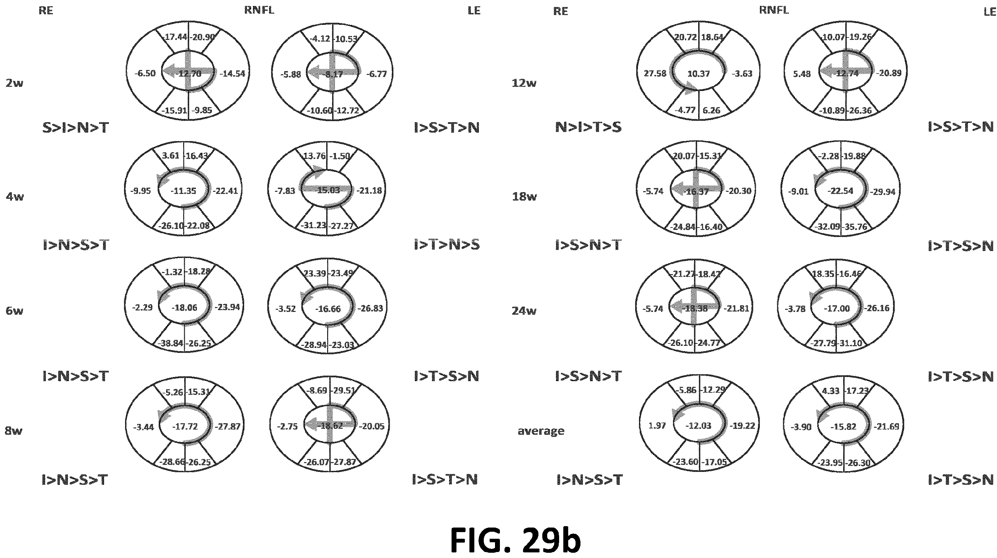

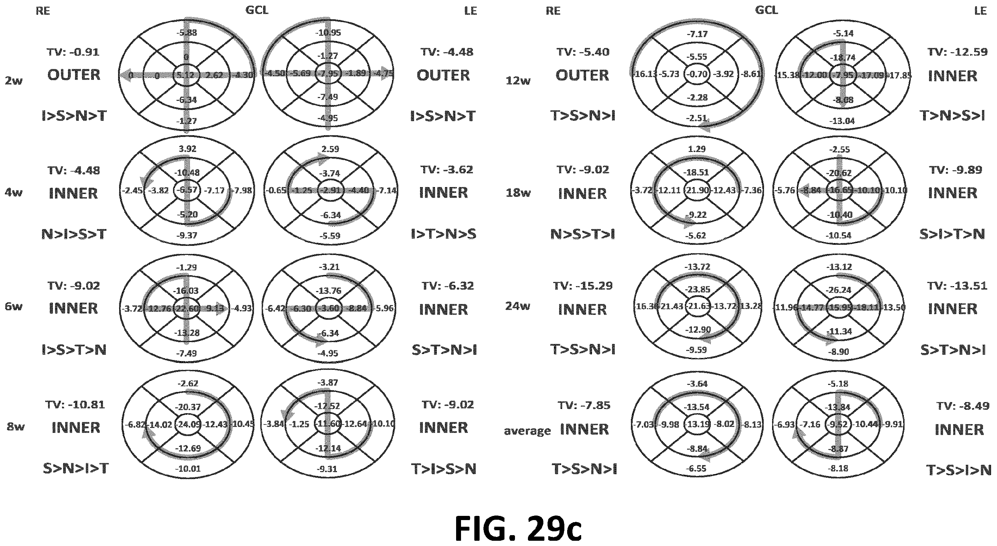

**Figure 29: Percentage thickness loss by optical coherence tomography (OCT) sectors and loss trend in MsDexafibro model over 6 month. a:** Retina. **b:** Retinal nerve fiber layer (RNFL). **c:** Ganglion cell layer (GCL). RE: right eye; LE: left eye; w: week; TV: total volume; S: superior; I: inferior; N: nasal; T: temporal; >: higher loss than.

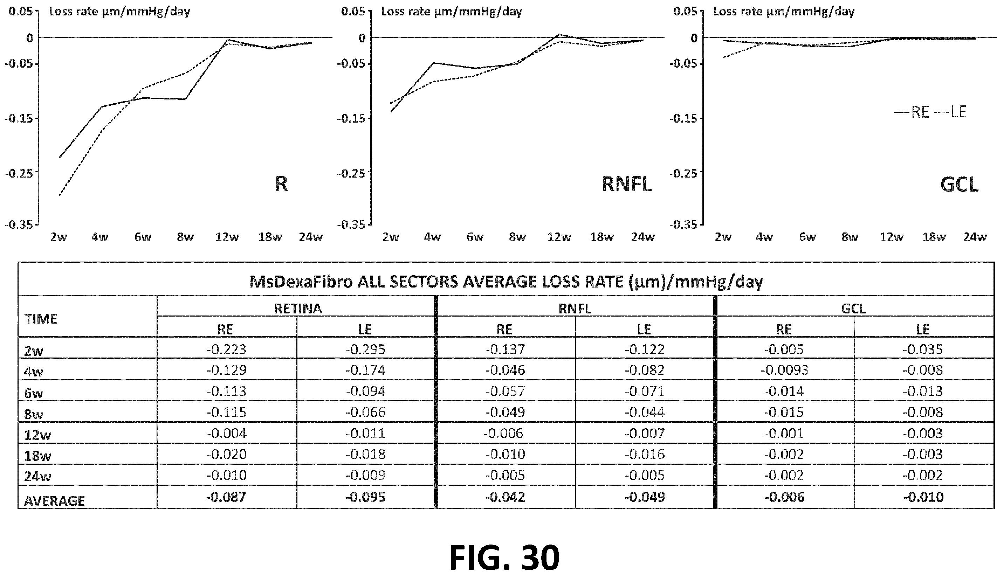

**Figure 30: Neuro-retinal thickness loss rate measured by optical coherence tomography (OCT) in microspheres co-loaded with dexamethasone and fibronectine (MsDexaFibro) model.** MsDexaFibro: microspheres co-loaded with dexamethasone and fibronectine; RE: right eye; LE: left eye; w: week; RNFL: Retinal Nerve Fiber Layer; GCL: Ganglion Cell Layer complex.

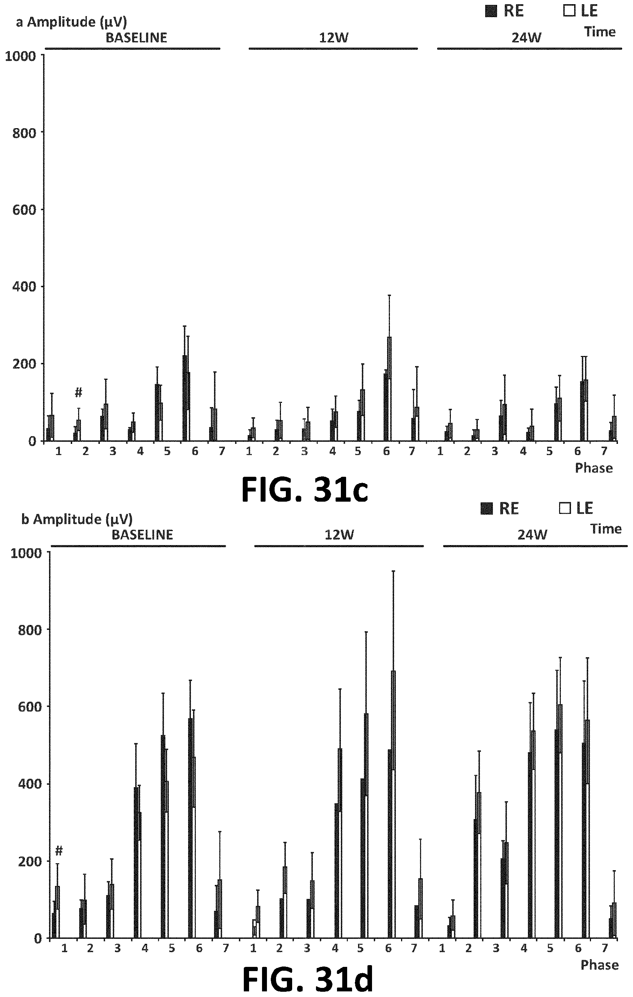

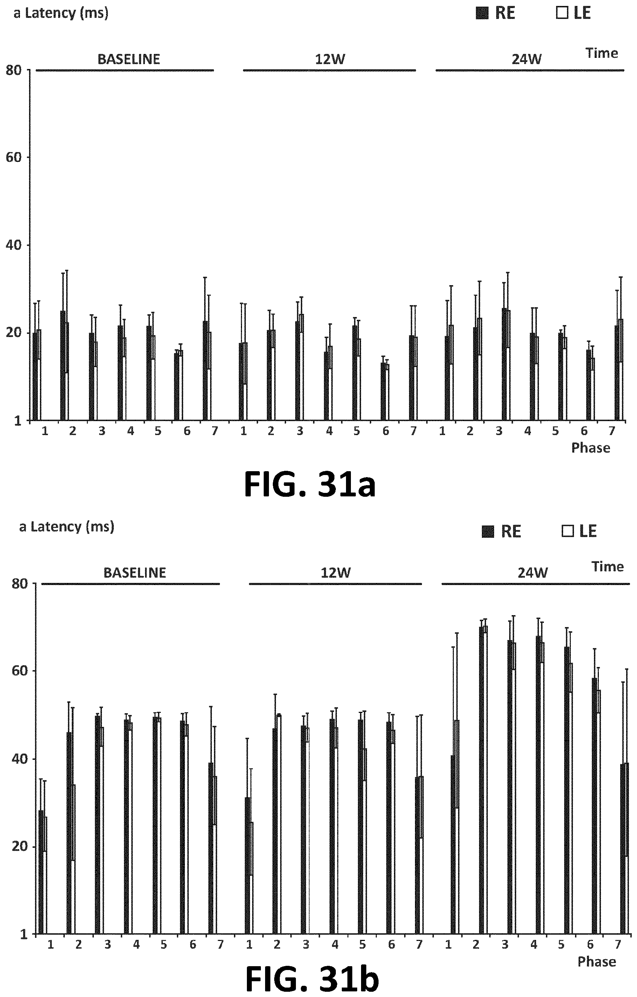

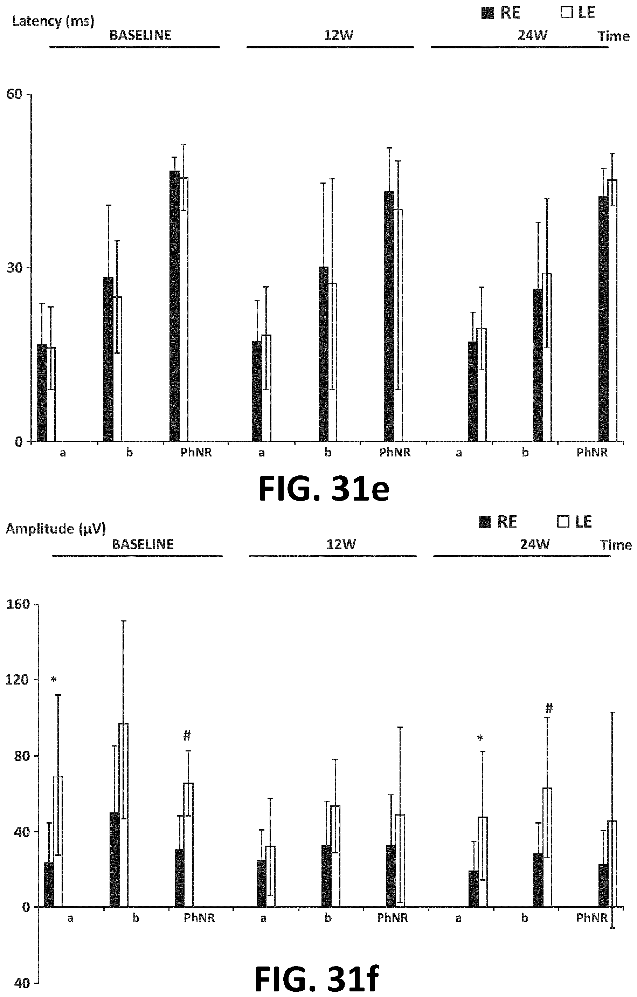

**Figure 31: Functionality of neuroretina by dark and light adapted electroretinography (ERG) in microspheres co-loaded with dexamethasone and fibronectine (MsDexaFibro) model over 6 month follow up.** MsDexaFibro: microspheres co-loaded with dexamethasone and fibronectine; RE: right eye; LE: left eye; w: week; DA: dark adapted; LA: Light adapted; $\mu$V: microvolts; ms: milliseconds. a-wave: photoreceptor functionality; b-wave: intermediate-bipolar cells functionality; PhNR-wave: retinal ganglion cell functionality.

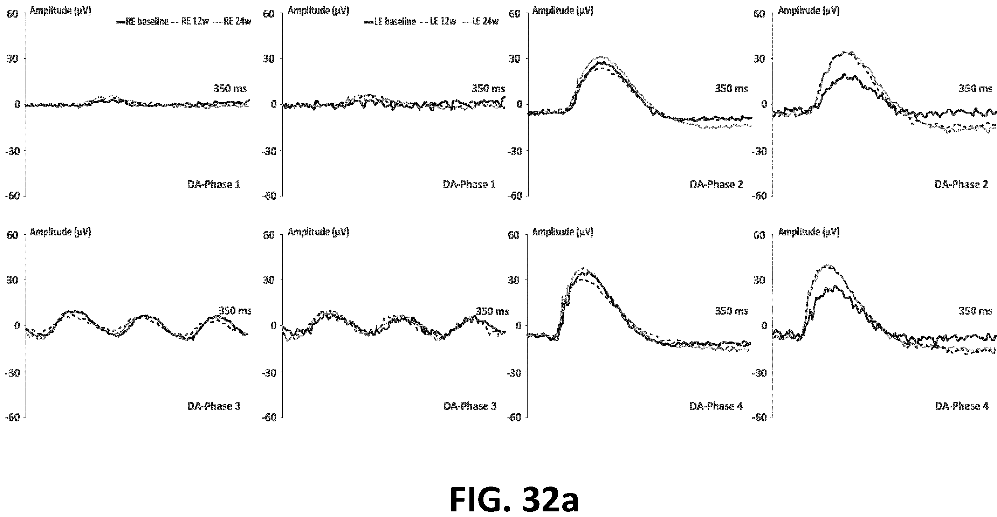

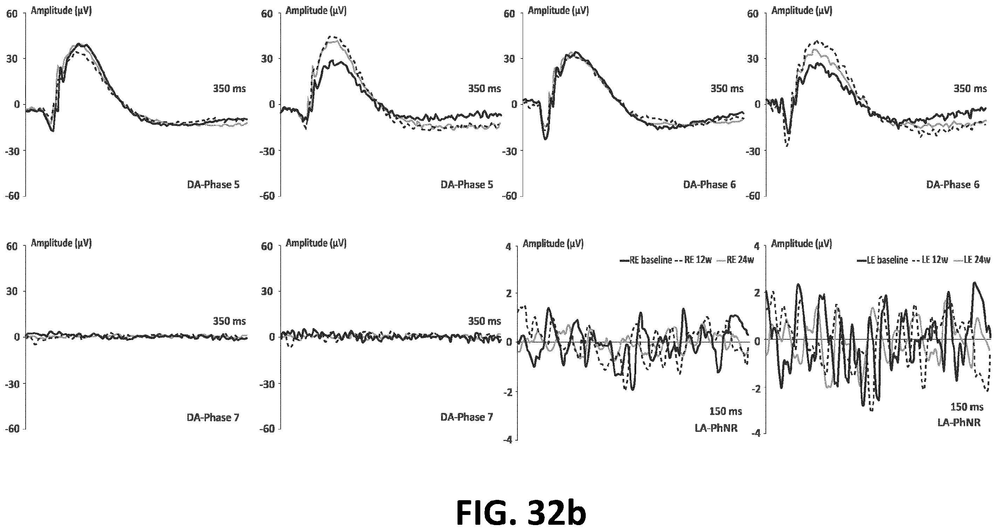

**Figure 32: Waves of neuroretinal functionality by dark and light adapted electroretinography (ERG) in microspheres co-loaded with dexamethasone and fibronectine (MsDexaFibro) model over 6 month follow up.** MsDexaFibro: microspheres co-loaded with dexamethasone and fibronectine; RE: right eye; LE: left eye; w: week; DA: dark adapted; LA: Light adapted; PhNR: photopic negative response; $\mu$V: microvolts; ms: milliseconds.

## OBJECT OF THE INVENTION

The main object of the present invention is represented by a non-human animal mammalian model of chronic glaucoma as defined in any one of claims 1-5..

It is also an object of the invention a method for preparing the non-human animal mammalian model of chronic glaucoma according to the invention as defined in any one of claims 6-11.

It is finally an object, the use of the non-human animal mammalian model of the invention for the study of physiopathology of glaucoma as well as a tool for pharmacological, biomaterial and/or surgical studies.

## DETAILED DESCRIPTION OF THE INVENTION

### Non-human animal mammalian model of chronic glaucoma

In a first aspect, the invention refers to a non-human animal mammalian model of chronic glaucoma wherein the animal has intraocular Polylactic-co-glycolic acid (PLGA), Polylactic acid (PLA) or Polyglicolic acid (PGA) microparticles, having a particle size between 5 $\mu$m and 40 $\mu$m in the anterior chamber of the eye of the animal in order to induce an increase in intraocular pressure resulting in a chronic gradual progressive neuroretinal degeneration from a functional-structural point of view and wherein the animal is a rodent.

The PLGA, PLA or PGA microparticles may be introduced in the eye in an unloaded form or loaded with any substance which may have an effect or an influence in the timing, intensity or any other parameter affecting the onset and development of the pathology. Although it is not primarily the aim in the present invention, the substance loaded in the microparticles can also be used for testing its therapeutic effect once the negative effects on retina and optic nerve degeneration of the animal model of the invention have started to become apparent.

In a particular embodiment of the invention the PLGA, PLA or PGA microparticles are loaded with dexamethasone or with a combination of dexamethasone and fibronectin. The use of PLGA, PLA or PGA microparticles loaded with dexamethasone or with a combination of dexamethasone and fibronectin have proven as effective in reducing the number of ocular injections needed in order to achieve the onset and development of the glaucomatous pathology.

The animal model of the invention is a chronic glaucoma model with ocular hypertension for at least 6 months' time (24 weeks). The model implies a gradual increase, without large fluctuations and with sustained IOP values, which produces a retinal, RGC and optic nerve neurodegeneration that mimics the one in chronic OAG.

The administration of biodegradable microparticles of PLGA, PLA or PGA, preferably in the anterior chamber of the eye has proven to cause a mechanical stop in the drainage of aqueous humor and a mild inflammatory reaction at a local level and, in the case of loaded microparticles, also the sustained release of substances such as dexamethasone (Dex) and fibronectin (Fibro) have proven to alter the trabecular meshwork.

The use of biodegradable microspheres or microparticles such as PLGA, PLA or PGA microparticles by means of corneal injections provide an animal model with a healthy ocular surface and anterior segment, no lens opacities, free visual axis, ocular biocompatibility, as well as, the need for less reinjections since microspheres' residues remain in the eye. Advantageously, the PLGA, PLA or PGA biomaterials used in the elaboration of microspheres are biocompatible, being $CO_2$ and $H_2O$ the products of their degradation (Herrero-Vanrell et al., 2001). According to molecular weight and composition, these polymers can have different degradation rates, ranging from months to years. Furthermore, these polymers have been used to create controlled drug delivery systems and that is why they are useful in the context of the invention to induce increased IOP and, at the same time, to release substances in a sustained and controlled manner in the case of loaded microparticles that can exert additional effects to be studied or tested.

In a particular and preferred embodiment of the invention, the non-human animal mammalian model which is a rodent is a rat, a mouse or a guinea pig. In the more preferred embodiment of the invention, the non-human animal mammalian model is a rat.

With regard to the size of the microparticles used in the context of the present invention, the microparticles of PLGA, PLA or PGA have a particle size between 5 $\mu$m and 40 $\mu$m, more preferably between 10 $\mu$m and 20 $\mu$m.

The microparticles must be injected in in the anterior chamber of the eye of the animal causing a gradual increase of IOP. The administration of the microparticles in the anterior chamber of the non-human mammal's eye produces a chronic intraocular pressure rise with ganglion cell death, which simulates the primary open-angle glaucoma of human physiopathology without negatively affecting other visual or motor functions in the eye. The process is not painful for the animal as it produces a gradual increase of IOP but not acute hypertension nor ischemia, nor retinal occlusions, nor corneal edema. Moreover, it is a simple surgical technique.

The resulting non-human mammal animal model thus mimics a chronic open-angle glaucoma of humans and has the following characteristics:

a) A chronic gradual progressive IOP increase.
b) Chronic gradual progressive neuroretinal degeneration from a functional-structural point of view.
c) Reduction in the number of animals used to assess long-term studies.
d) Animal refinement due to the need for injecting fewer times in the animal eye in order to obtain a progressive and slow rise in IOP with no pain.

e) An easier surgical technique for inducing increased IOP.

Since the non-human mammal animal model of the invention evolves in chronic gradual retinal, RGC and optic nerve degeneration, it has the advantage that it allows treating and/or preventing neuroretinal loss in a functional damage frame before cell death and the subsequent secondary degeneration occur, focusing on detection and early treatment of neurodegeneration. As such it represents an hypertensive and neurodegenerative ocular model which can be used as a potential effective tool for later pharmacological, biomaterial and/or surgical studies.

In addition, although primarily focused in the study of the physiopathology of glaucoma as well as in the development of innovative treatments of glaucoma, the animal model of the present invention can also be used as a research tool for the rest of the pathologies that cause optic nerve degeneration (ischemic optic neuropathology, hereditary optic neuropathology, tox-nutritional optic neuropathology, etc.)

The non-human mammal animal model of the present invention when compared with the well-established episcleral injection of hypertonic saline solution model, has the advantage of causing intraocular pressure in a slow, progressive and sustained manner over time, with an associated relevant ganglion cell and RNFL (Retinal Nerve Fiber layer) thickness loss, although in a slower and less aggressive manner, with fewer reinjections and also a better preserved ocular surface.

Method for generating the non-human animal mammalian model of chronic glaucoma

A further aspect of the present invention is a method for generating or preparing the non-human animal mammalian model of chronic glaucoma of the invention comprising the intraocular injection in the animal's anterior chamber of the eye of a suspension, preferably an aqueous suspension, of PLGA, PLA or PGA microparticles and having a particle size between 5 $\mu$m and 40 $\mu$m.

In particular and preferred embodiment of the method, the microparticles are loaded with dexamethasone or with a combination of dexamethasone and fibronectin. As explained before, the use of these compounds or substances have proven to effectively reduce the number of ocular injections needed in order to achieve the onset and development of the physiological and anatomical symptoms of glaucoma. For example, when non-loaded PLGA microparticles are used in the development of the animal model, microparticles were injected 7 times in 24 weeks. However, when the same microparticles are loaded with dexamethasone the frequency of injection can be reduced to two injections in the 24 weeks study, namely at the start of the study an in week 4. In the case the same microparticles are loaded with dexamethasone and fibronectin a single injection at the start of the 24 week study is enough for achieving the desired effects.

The preferred embodiment of the method for generating a rodent model of chronic glaucoma comprises using a rat, a mouse or a guinea pig. In a particularly preferred embodiment, the animal used in the method is a rat.

As already mentioned, the method of the invention comprises that the intraocular injection is performed in the anterior chamber of the eye of the animal.

The optionally loaded microparticles injected must have a particle size between 5 and 40 $\mu$m, more preferably between 10 $\mu$m and 20 $\mu$m. They are preferably injected in aqueous suspension with a concentration of microparticles of preferably 0.005% to 20% by weight of the total suspension. A volume of 1 $\mu$l to 5 $\mu$l of the aqueous suspension of microparticles is generally and preferably-injected in the animal's eye.

Prior to injection, animals are preferably anesthetized by any usual mean. For the correct injection the use of atraumatic forceps is highly recommended. The forceps allow holding the eyeball by using the eyelid skin as girth thus avoiding sudden an intense eyeball prolapse. Injection can be then performed by usual means such as a needle or microneedle, preferably a glass microneedle.

Use non-human animal mammalian model

The final aspect of the invention refers to the use of the non-human animal mammalian model of glaucoma of the present invention.

The more important application of the non-human animal mammalian model of the present invention is for its use in the study of physiopathology of glaucoma. More particularly, the present animal model is a research tool for the study of chronic OAG as the pathology evolves in the model with a progressive and chronic increase of IOP and consequently, a chronic neurodegenerative process which simulates the conditions which appear in chronic glaucoma patients. The mechanism works causing both a mechanical and pharmacological blockage in the trabecular meshwork and which can simulate OAG physiopathology and cause a slow, persistent and progressive rise of IOP with slow retinal degeneration, including among others: retinal ganglion cell layer, optic nerve, photoreceptors layer, activation and proliferation of glial cells, etc.

As a model of glaucoma, the non-human animal mammalian model of the present invention is useful as a tool for pharmacological, biomaterial and/or surgical studies.

## EXAMPLES

The development and validation of the non-human animal mammalian model of glaucoma of the present invention was achieved on the basis of the results of a study which is the object of the next examples. The conditions and methods used in the study will become apparent in the next examples.

## 1-ETHICAL IMPLICATIONS AND/OR SECURITY

This invention involves animal experimentation for its development, therefore it adjusted to comply with the current legislation, both the European directive (2010/63/EU) and the Spanish Royal Decree (RD 53/2013), as well as the Animals Protection regulation (Ley11/2003) in Aragon, which establishes basic rules applicable to the protection of animals used for experimental purposes.

Conversely, the staff in charge of working with animals is qualified to do so and followed the Order ECC/566/2015 which establishes the requirements to be met by the researchers who operate with animals used, bred or provided for the purpose of experimentation and other scientific purposes.

Studies were carried out in the research support center of the Centre for Biomedical Research in Aragon (CIBA) and Aragon Institute of Health Research (IIS Aragon), accredited center who has an animal experimentation department, classified as breeding, suppliers and users' facility (in accordance with article 13 del R.D. 1201/2005 for the protection of animals used for experimentation).

On the one hand, this invention allows reducing the number of animals used, since it generates long periods of ocular hypertension with progressive and chronic rise in the IOP in the same animal, and structural and functional follow-up performed through non-invasive tests such as OCT (optical coherence tomography) and ERG (Electroretinography).

Besides, we created a refined model of glaucoma in which the intervention to generate it is minimally invasive by means of corneal injection. It does not produce the common sudden hypertension that occurs in the known prior art models and which could be the cause of the pain, but instead hypertension is progressive and asymptomatic, as it happens in the human pathology.

The model was achieved by injecting biocompatible and biodegradable material, and as an 'add-on' reducing the need for multiple reinjections to maintain ocular hypertension. For example, a total of 7 injections were needed in 6 months for the hypertensive curve achievement; 2 in the model using Ms loaded with dexamethasone and just 1 injection in the model by Ms co-loaded with dexamethasone and fibronectin.

In addition to reducing the number of interventions in the animal, surgical time oscillated between 6 and 7 minutes from the beginning of the anesthetic induction to ocular injection and introduction in an oxygen enrichment box for kind recovery. A model of chronic glaucoma has been achieved inflicting as little discomfort on the animal as possible, being minimally invasive, without the need of genetic modification that could alter its biology and/or tumorigenesis, easy to conduct and reproducible, approaching to the real human glaucomatous pathology.

## 2- PREPARATION AND CHARACTERIZATION OF MICROSPHERES

Biodegradable microspheres (Ms) were elaborated using the solvent extraction-evaporation method from a previously formed emulsion composed by an inner oily phase and an external aqueous phase (O/W emulsion). The polymer used was PLGA 50:50 with a molecular weight of 16,000 g/mol GPC and an inherent viscosity of 0.24 dl/g [Resomer 502]. The method employed for microspheres preparation was the following: PLGA (400 mg) was dissolved in methylene chloride (2 mL). This organic phase was emulsified with 5 mL of PVA MiliQ water solution 1 % (w/v) using a homogenizer (Polytron® RECO, Kinematica, GmbHT PT3000, Lucerna, Swithzerland) at 7,000 rpm for 1 minute. The formed emulsion was poured onto 100 mL of PVA MiliQ water solution 0.1 % (w/v) and maintained under magnetic stirring for 3 hours to allow organic solvent extraction and evaporation, and subsequently Ms maturation. Afterwards, Ms were washed in MilliQ water in order to remove PVA and separated in two granulometric fractions (38-20 $\mu$m and 20-10 $\mu$m) employing three sieves (mesh size 38, 20 and 10 $\mu$m). Finally, Ms were freeze-dried (Freezing: -60°C /15 min, drying: -60 °C/12h/0.1 mBar) and storage at -30°C in dry conditions

Two granulometric fractions were selected for *in vivo* study with non-loaded Ms (10-20 and 20-38 micrometers). Injections were performed at basal, 2, 4 and 8, 12, 16 and 20 weeks. Using Ms sized 10-20, as comparable results were obtained comparing to the hypersaline episcleral model.

PLGA microspheres are homogeneous degradable matrices in which active compounds can be included and subsequently released for several months, depending on the polymer and active characteristics. In this case, we have prepared dexamethasone loaded PLGA microspheres in the 10-20 micrometer range using the same polymer mentioned before.

Dex-loaded PLGA Ms were obtained following the already mentioned Oil-in-/Water (O/W) emulsion solvent extraction-evaporation technique. First, PLGA (400 mg) was dissolved in methylene chloride (2 mL). Micronized Dexamethasone (40 mg) was added to the polymeric solution and dispersed by ultrasonication (Ultrasons; J.P. Selecta, Barcelona, Spain) for 5 minutes and further sonicated (Sonicator XL; Heat Systems, Inc., Farmingdale, NY, USA) for 1 minute at 4 °C to obtain an homogenous dispersion. The so-formed O-phase was emulsified adding 5 mL of PVA MiliQ water solution 1 % (w/v) through a homogenizer (Polytron®RECO, Kinematica, GmbHT PT3000, Lucerna, Swithzerland) at 7,000 rpm for 1 minute. The resulting O/W emulsion was incorporated to 100 mL of PVA MiliQ water solution 0,1 % (w/v) and stirred for 3 hours leading to Ms maturation.

Once maturation step was completed, MiliQ water was employed to wash Ms, removing the remaining surfactant PVA. Subsequently, the 20-10 μm size fraction was selected, freeze-dried (Freezing: -60°C /15 min, drying: -60 °C/12h/0.1 mBar) and storage at -30°C in dry conditions

In a third part of the study dexamethasone/fibronectine-loaded PLGA microspheres were prepared as follows: The water-in-oil-in-water (W/O/W) double emulsion solvent extraction-evaporation technique was employed for dexamethasone/fibronectine-loaded PLGA microspheres elaboration. Briefly, micronized Dex (40 mg) was added to a polymeric solution (400 mg of PLGA dissolved in 2 mL of methylene chloride). The suspension was homogenized by ultrasonication with ice-water bath (Ultrasons; J.P. Selecta, Barcelona, Spain) for 5 minutes and sonication (Sonicator XL; Heat Systems, Inc., Farmingdale, NY, USA) for 1 additional minute in ice-water bath. The inner aqueous phase composed by 20 μL of fibronectine water solution (containing 42.8 μg of fibronectin) was added to this organic phase and emulsified by sonication (Sonicator XL; Heat Systems, Inc., Farmingdale, NY, USA) for 30 seconds at 4 °C to create the initial W1/O emulsion. Afterwards, 5 mL of PVA MiliQ water solution 1 % (w/v) were added to the mentioned W1/O emulsion and the mixture was emulsified (Polytron®RECO, Kinematica, GmbHT PT3000, Lucerna, Swithzerland) at 7,000 rpm for 1 minute to form the final W/O/W emulsion that was finally added to 100 mL of PVA MiliQ water solution 0,1% (w/v) to get hardening by organic solvent extraction and evaporation under magnetic stirring at room temperature for 3 hours. After that, MSs were washed with MiliQ water to remove surface PVA. The desired granulometric size fraction (20-10 μm) was collected by sieving, freeze-dried (Freezing: -60°C /15 min, drying: -60°C/12h/0.1 mBar) and storage at -30°C in dry conditions.

Loaded and non-loaded PLGA Ms characterization was performed in terms of morphological evaluation, mean particle size and particle size distribution, encapsulation efficiency and *in vitro* release studies (for loaded Ms). Production yield percentage of the selected granulometric fraction was also determined.

### 2.1. Production yield percentage (PY %)

Production yield percentage was calculated according to the following equation (1).

$$PY\% = \frac{amount\ of\ microspheres}{Total\ amount\ of\ polymer + Total\ amount\ of\ active\ compound(s)} x100$$

Equation 1.

### 2.2. External morphological evaluation

Gold sputter-coated freeze-dried loaded and non-loaded PLGA Ms were observed by scanning electron microscopy (SEM, Jeol, JSM-6335F, Tokyo, Japan).

### 2.3. Mean particle size and particle size distribution

Particle size and particle size distribution were measured by dual light scattering (Microtrac® S3500 Series Particle Size Analyzer, Montgomeryville, PA, USA). Volume mean diameters (± standard deviation) obtained from 3 measurements were used to express the mean particle size.

### 2.4. Dexamethasone quantification by HPLC/MS

The HPLC/MS system was composed by a liquid chromatography instrument (Waters 1525 binary HPLC pump and Waters 2707 autosampler) employing a Nova-Pak C18 column (4 μm, ID 2.1 mm×150 mm) coupled to a guard column (4 μm, 3.9 mm ×20 mm), both maintained at 45 °C. MS detector (Waters 3100 single quadrupole mass spectrometer) was connected to this system via Empower 2 (Waters, Milford, USA). The ESI source was adjusted in the positive ion mode (ESI(+)) for Dex detection. Selected ion recordings (SIR) DX mass (m/z) 147.10 was measured under mass spectrometer source conditions of 3.5 kV electrospray voltage, 130 °C heated capillary temperature. Nebulization (100 L/h flow rate, 130 °C source temperature, 5 V extractor voltage) and desolvation (400 L/h flow rate, 300 °C desolvation temperature) were performed employing nitrogen gas (> 99.999 %). An isocratic HPLC method was developed to quantify Dex encapsulation efficiency and release from Ms. This method was composed by 50 % ammonium acetate 15 mM/1 mL formic acid in MiliQ water and 50 % acetonitrile at a flow rate 0.3 mL/min.

### 2.5. Dexamethasone Encapsulation efficiency

A known amount of Dex-loaded PLGA Ms (1 mg) was dissolved in 2.5 mL of methylene chloride. Then, 6 mL of methanol were incorporated in order to precipitate the dissolved polymer. After vortex mixing and centrifugation (5,000 rpm for 5 minutes at 20°C), the etanol:methanol supernatant was collected, filtered (0.22 $\mu$m) and analyzed using the previously described HPLC/MS method for dexamethasone quantification.

### 2.6 *In vitro* Dexamethasone release studies from Dex-loaded and Dex/Fibro-loaded PLGA Ms

A Dex-loaded PLGA Ms suspensions (2.5 mg/mL) was prepared in quadruplicate using phosphate buffered saline (PBS, pH 7.4) with sodium azide (0.02 % (w/v) as release media using 2 mL Eppendorf tubes. The so-prepared samples were located in a water shaker bath (100 rpm, 37 °C, Memmert Shaking Bath, Memmert, Schwabach, Germany). Supernatants were periodically collected after gently centrifugation (5,000 rpm for 5 min, 20°C), filtered (0.22 $\mu$m) for dexamethasone quantification by HPLC/MS employing the method previously mentioned). At each time point, the tubes containing the remaining Ms samples were refilled with fresh release media to continue with the release study. The same protocol was performed at each time-point.

In order to mimic the animal study, a second "dose" of 2.5 mg of Dex-loaded PLGA Ms were included at 28-days post-study to each sample, also increasing the amount of release media to maintain the initial suspension concentration. The release study was performed as explained before for 140 additional days (total time of in vitro release study: 168 days, that is 24 weeks).

### 2.7 *In vitro* Fibronectin release studies from Dex/Fibro-loaded PLGA Ms

2.5 mg of dexamethasone/fibronectin-loaded PLGA Ms were suspended in phosphate buffered saline (1 ml, PBS, pH 7.4) including sodium azide (0.02% (w/v) and bovine serum albumin (BSA) (1%) in a 2 mL low-binding Eppendorf tubes. The suspension was placed in a water shaker bath (100 rpm, 37°C, Memmert Shaking Bath, Memmert Schwabach, Germany) (n=2). At pre-set times, the so-prepared samples were centrifuged (5,000 rpm, 5 minutes, 20°C), the supernatants were removed for Fibronectine quantification by Enzyme-Linked ImmunoSorbent Assay (ELISA). At each time point, the tubes containing the remaining microspheres were refilled with fresh PBS/azide/BSA media to continue with the release study.

### 3. ANIMAL MODEL CHARACTERIZATION

Thus far, there are no studies which approach the development of a chronic glaucoma animal model using this methodology with PLGA biodegradable microspheres.

During the development of this invention, we have proved that the injection of 2 microliters of PLGA microsphere suspensions (10%) (w:y), when injected in the anterior chamber of the rats' eyes, is mainly stored in the trabecular meshwork provoking a stop at the transition of the aqueous humor, inducing a slow and progressive increase in the IOP. This IOP increase results in chronic glaucoma, slow degeneration of the ganglion cell layer and loss of progressive visual capacity in the animal.

In order to correctly develop the new animal model, a well stablished model (which is an episcleral veins sclerosis model by injecting a hypertonic solution -NaCl 1.8M-) was compared with the new proposed strategy. In a first step two particle size ranges were used: (38/20 $\mu$m) and (20/10 $\mu$m). The comparative study was performed for 8-week study. The three models were characterized through weekly clinical analysis and measurements of IOP; biweekly structural study with optical coherent tomography (OCT) (0, 2, 4, 6, 8 weeks), and baseline and final functional study with electroretinogram (ERG). In the group of microspheres, their position was visualized at a trabecular level through direct visualization proving the preferential localization of microspheres at the inferior iridiocorneal angle (due to higher density of Ms compared to aqueous humor).

The episcleral sclerosis model and the long-term 20/10 $\mu$m microspheres model were subsequently compared in a long-term study (24-week study). Both models were characterized through clinical analysis and weekly IOP measuring, structural study with *in vivo* OCT at baseline 8, 12, 18 and 24 weeks (because earlier times were deeply analysed in the preliminary) and functional study with ERG at 0, 12 and 24 weeks.

For both 24-weeks studies performed with loaded Ms (dexamethasone and co-loaded dexamethasone-fibronectin) clinical signs and IOP were recorded weekly, OCT at 0, 2, 4, 6, 8, 12, 18, 24 weeks and ERG at 0, 12, 24 weeks in order to compare with non-loaded Ms.

In all experiences the first injection of microspheres were performed in 4-week-old Long Evans rats weighed between 50 and 100 grams at the beginning of the study.

In the 8-week study 25 animals were used in the episcleral model, 16 animals in the 38/20 microspheres and 23 in the 20/10 microspheres. In the long-term (24-weeks) study 25 animals were employed in the episcleral model and 25 in the non-loaded 20/10 microspheres. In the 24-week study performed with dexamethasone loaded microspheres 43 animals were used. Finally, in the 24-week study with dexamethasone-fibronectin loaded microspheres 45 animals were used. All cohorts were composed by 40% males and 60% females.

The experiment was approved by the Committee on Animal Research and Ethics (PI34/17) according to ARVO requests. Likewise, the premise of using the least number of rats was accomplished so as to obtain a reliable average value in each of the programmed post-injection times.

### 3.1. Anterior chamber injection (Injection procedure):

Pre-surgical preparation:

An hour before starting the surgical procedure the animal received a subcutaneous injection of buprenorphine (0.05 mg/kg) to avoid any intra- or postoperative discomfort, buprenorphine produced an intraoperative miosis, which opens the iridocorneal angle and protects the lens from a iatrogenic trauma with the glass micropipette.

The anesthetic induction was carried out in an induction box with a mixture of 3% sevoflurane and 1.5% oxygen. Once the animal was asleep, 1 drop of topical corneal anesthetic (doble anesthetic Colircusi®) and a povidone-iodine solution (10% Betadine®) was instilled for ocular cleaning. The same action was repeated in the surgical table. The animal remains under gas anesthesia with a nose-mouth mask and temperature control in the course of the surgical procedure.

Reconstitution of PLGA microspheres:

Freeze-dried PLGA microspheres were reconstituted in a saline solution NaCl 0.9%, at a concentration of 10% (w:v), vortexed and pipetted until achieving a yellowish-white homogeneous suspension.

Surgical injection:

By means of atraumatic forceps the eyeball is held using the eyelid skin as girth, avoiding sudden and intense eyeball prolapse. 2 microlitres of particles suspension were injected with a glass microneedle by a 10 microlitres Hamilton® syringe. The injection was performed in the superotemporal quadrant in clear cornea, between the limbo-corneal and apex, eluding visual axis and valved to avoid reflux.

The animal was allowed to recovering in a box with temperature control and 2% oxygen enrichment. This leads to a progressive awakening without hyperpressure, vasalva or ocular discomfort. The complete surgical procedure was performed in 7 minutes from induction to complete recuperation.

### 3.2. Clinical analysis:

The corneal surface appearance, the anterior chamber (to confirm the presence of microspheres inside as well as to discard any acute inflammatory reactions or bleeding), the iris and the crystalline were assessed, 24 hours post-injection and weekly until the end of the study.

**3.3. IOP measuring:** IOP measuring were carried out weekly with the rebound tonometer Tonolab® (Tonolab; Tiolat Oy Helsinki, Finland). The measurements were always performed in the morning under gas anesthesia (sevofluorane 3%) not exceeding 3 minutes to avoid changes in the IOP due to the anesthetic effect. The final measurement was the average of 3 consecutive measuring, being this the average of 6 consecutive rebounds, which makes a total of 18 measurements.

### 3.4. Retina and optic nerve structural study:

OCT is a technique of digital image analysis widely used in ophthalmology. It registers light from a source which rebounds in the retina and is able to take high resolution tomography slides, which allow identifying retinal layers similar to an *in vivo* histological analysis. The advantages of this powerful test are that it is not invasive, it is comfortable for the patient and examiner, fast, accurate, reliable and easy to carry out even in animals which do not focus sight. It allows visualization, analysis, and a quantitative follow-up of parameters such as thickness of the nerve fiber layers in the retina around the optic nerve, and the thickness of each layer of the retina (by segmentation) to the level of the posterior pole of the eye. This test was carried out in every eye using the latest technology of a fourier-domain device (high-resolution Spectralis, Heidelberg Engeneering, Germany). The version for rodents of this system acquires cross sectional images by means of 61 b-scans around of 3 mm of length centered in the optic nerve-and a contact lens adapted on rat's cornea to get higher quality images. In order to carry out this test, the animal received intraperitoneal mixture of ketamine (60mg/kg) - dexmedetomidine (0.25 mg/kg) anaesthesia as well as a mixture of tetracaine (1mg/ml) - oxibuprocaine (4mg/ml) topical ocular anaesthesia and kept under thermal control during the procedure.

### 3.5. Functional study with electroretinogram (ERG):

ERG (Roland consult® RETIanimal ERG, Germany) allows efficient characterization of animal models, since it assesses the functioning of the retina and the transmission of visual impulse to the brain. This test was performed in a dark room after darkness acclimation for at least 12 hours and pupillary dilation with tropicamide and phenylephrine eyedrops. It was carried out under intraperitoneal ketamine-dexmedetomidine anesthesia and thermal control.

Dark-adapted flash scotopic ERG (specific to assess middle and outer retina layers such as bipolar and photoreceptor) and light-adapted photopic negative response (PhNR) protocol (specific for RGC evaluation) were used.

With these two tests (OCT and ERG) structural and functional damage of retina and specifically the ganglion cell layer in alive animals were determined without causing harm nor sacrifice in the different established examination times. These techniques allow the monitorization of the illness using the less number of animals and trying to cause the less damage as possible (both tests are non-invasive and therefore do not cause damage to animals, only risks due to cumulative anesthesia).

### 4. RESULTS

### 4.1. PRELIMINARY STUDY: Ms (20/10) and (38/20) for 8 weeks of follow-up and comparison with the episcleral model.

#### 4.1.1. Microparticles characterization

**Non-loaded PLGA MS:**

**Production yield, mean particle size and particle size distribution**

PLGA Ms prepared showed a production yield (PY) between 43 and 46 % for the 38-20 $\mu$m size range, while the 20-10 $\mu$m fraction resulted in a PY% between 37 and 40 %. In both cases a monodisperse distribution of particle size was observed. The mean particle size for each size range is compiled in table 1.

**Table 1:** Production yield, and mean particle size obtained for both size ranges used in the study (n=3)

| Ms Fraction | PY (%) | Mean Particle Size |
|---|---|---|
| 38-20 $\mu$m | 44.94 $\pm$ 1.60 | 21.84 $\pm$ 1.26 $\mu$m |
| 20-10 $\mu$m | 39.37 $\pm$ 1.75 | 14.07 $\pm$ 1.07 $\mu$m |

**Morphological evaluation**

According to SEM pictures, both size fractions showed spherical particles in the micro-range with non-porous smooth surfaces (figure 1).

#### 4.1.2. Ophthalmological clinical signs and intraocular pressure

No infection, intraocular inflammation (synechae), cataract formation, neither retinal detachment was found in any ocular hypertensive (OHT) model but corneal surface was better preserved in the Ms20/10 and Ms38/20 models. Microparticles showed a tendency to localize at the inferior iridocorneal angle. In some cases they agglomerate forming a solid depot. This disposition allowed a clear visual axis and subsequently a correct OCT and ERG acquisition (figure 2).

Injection of PLGA Ms in the anterior chamber was able to promote a continuous elevation of IOP. OHT was detected three weeks after first injection in both microspheres models, but since first week in EPIm. The Ms38/20 model showed more fluctuations in IOP values. High percentages of OHT (>20mmHg) eyes were found over time in all the three models but EPI model showed the biggest percentages at nearly all times explored (figure 3).

### 4.1.3. Structural neuroretinal analysis by optical coherence tomography

Both Ms models and epiescleral model experienced a progressive decrease in retina, Retinal Nerve Fiber Layer (RNFL) and Ganglion Cell Layer (GCL) and although fluctuations in thickness were observed in all the three models, the Ms38/20 model showed the biggest one (table 2). The percentual loss of OCT thickness (change of thickness respect to baseline measurement of each variable) was also analyzed and the Ms 38/20 model showed at the end of the study, the highest percentage thickness loss in all OCT parameters. The GCL parameter experienced the biggest loss in each Ms model (figure 4)

| RIGHT EYE (Ms 38/20) | | | | | | |
|---|---|---|---|---|---|---|
| OCT PARAMETERS (µm) | BASELINE Mean±SD | 2w Mean±SD | 4w Mean±SD | 6w Mean±SD | 8w Mean±SD | P* |
| **RETINAL THICKNESS** | | | | | | |
| CENTRAL | 274.63±13.50 | 273.41±16.08 | 274.90±15.07 | 270.44±24.12 | 271.00±16.40 | 0.441 |
| INNER INFERIOR | 268.73±12.29 | 263.92±9.48 | 269.70±13.37 | 263.33±13.06 | 259.44±8.07 | 0.017[#] |
| OUTER INFERIOR | 265.55±11.08 | 261.33±7.34 | 264.00±11.46 | 256.66±8.47 | 253.55±8.42 | 0.012[#] |
| INNER SUPERIOR | 261.64±10.23 | 255.83±8.41 | 261.30±16.82 | 253.44±12.84 | 247.77±9.53 | 0.050 |
| OUTER SUPERIOR | 270.82±10.94 | 264.08±7.87 | 267.90±12.10 | 261.77±13.03 | 258.55±7.98 | 0.012[#] |
| INNER NASAL | 265.23±11.74 | 262.33±6.02 | 262.90±7.95 | 259.77±9.97 | 255.33±7.12 | 0.068 |
| OUTER NASAL | 266.23±10.04 | 262.50±5.36 | 262.80±7.36 | 260.66±10.07 | 254.77±6.64 | 0.018[#] |
| INNER TEMPORAL | 264.95±11.12 | 258.92±7.65 | 262.20±11.96 | 258.22±9.32 | 252.33±3.60 | 0.017[#] |
| OUTER TEMPORAL | 266.95±10.34 | 260.58±6.33 | 263.80±12.70 | 258.25±7.24 | 255.11±3.44 | 0.018[#] |

| TOTAL VOLUME | 1.97±0.05 | 1.97±0.04 | 1.97±0.05 | 1.83±0.11 | 1.81±0.03 | 0.012[#] |
|---|---|---|---|---|---|---|
| **RNFL THICKNESS** | | | | | | |
| GLOBAL | 46.04±5.65 | 42.90±3.41 | 45.90±5.19 | 43.22±3.30 | 41.00±3.95 | 0.058 |
| INFERIOR TEMPORAL | 45.61±5.08 | 39.60±6.04 | 47.10±11.29 | 41.33±6.00 | 39.28±5.93 | 0.075 |
| INFERIOR NASAL | 45.70±9.10 | 42.50±5.56 | 45.80±8.89 | 43.22±6.75 | 41.85±7.24 | 0.237 |
| SUPERIOR TEMPORAL | 48.48±11.54 | 46.90±11.58 | 47.10±9.25 | 46.11±10.70 | 41.42±11.90 | 0.674 |
| SUPERIOR NASAL | 45.42±10.00 | 44.60±5.54 | 42.50±5.16 | 46.11±8.78 | 42.85±7.10 | 0.892 |
| NASAL | 44.09±8.53 | 39.60±6.60 | 44.70±5.33 | 39.66±7.41 | 37.14±9.40 | 0.271 |
| TEMPORAL | 43.35±13.15 | 45.40±4.45 | 47.40±10.76 | 45.11±4.37 | 43.85±5.36 | 0.225 |
| **GCL THICKNESS** | | | | | | |
| CENTRAL | 20.27±2.54 | 21.00±2.86 | 19.80±2.52 | 18.66±3.24 | 16.11±2.14 | 0.020[#] |
| INNER INFERIOR | 28.06±2.23 | 26.50±3.06 | 25.80±1.75 | 22.22±6.88 | 24.44±2.40 | 0.027 |
| OUTER INFERIOR | 27.89±1.37 | 27.17±1.89 | 26.70±1.33 | 22.77±7.99 | 26.00±1.50 | 0.017[#] |
| INNER SUPERIOR | 26.50±1.89 | 24.17±4.56 | 24.20±3.99 | 22.22±3.56 | 20.44±3.43 | 0.028 |
| OUTER SUPERIOR | 26.27±2.86 | 25.67±3.28 | 26.10±3.57 | 25.66±1.93 | 24.33±2.50 | 0.180 |
| INNER NASAL | 24.68±4.44 | 23.00±4.30 | 24.60±2.83 | 22.33±3.77 | 20.89±3.33 | 0.141 |
| OUTER NASAL | 25.45±3.55 | 24.42±3.23 | 26.40±1.42 | 24.33±3.77 | 24.11±3.48 | 0.799 |
| INNER TEMPORAL | 24.23±4.93 | 22.50±3.45 | 22.20±3.19 | 20.55±5.41 | 19.78±4.65 | 0.205 |
| OUTER TEMPORAL | 26.50±3.36 | 24.33±2.70 | 25.20±2.52 | 22.75±5.59 | 22.33±3.57 | 0.049 |
| TOTAL VOLUME | 0.18±.01 | 0.17±0.01 | 0.15±0.01 | 0.13±0.05 | 0.15±0.01 | 0.026 |

**Table 2: Structural neuro-retinal analysis by optical coherence tomography (OCT) with the Ms 38/20 model**. Ms 38/20: microspheres sized 38/20 model; RNFL: Retina Nerve Fiber Layer; GCL: Ganglion cell layer complex; thickness in microns

(µm); mean±SD (SD: standard deviation); p<0.05 statistical significance; p<0.020$^{\#}$ statistical significance with Bonferroni correction for multiple comparisons. Grey color cells showed the two thinnest sectors at every exploration.

EPI and Ms 38/20 models showed higher loss in the outer retinal sectors and all models showed higher percentage loss in the superior-inferior axis sectors in RNFL. Moreover all OHT models showed higher loss in the inner sectors of GCL and RE from both EPI and Ms 20/10 models experienced the same percentage loss trend by OCT sectors (S>I>N>T) (figure 5) at 8 week.

The RE loss rate per day and mmHg that IOP had increased in each week was calculated and expressed in µm/mmHg/day from all OCT sectors average to standardized the neuroretinal loss. The Ms 38/20 model experienced the more important loss rate (in average) in the Retina, RNFL and GCL over the study and it occurred at most examinations. EPI and Ms 20/10 models experienced similarly loss rate in RNFL (0.0033 vs 0.0030 µm/mmHg/day) at the end of the study (week 8) (table 3).

| | RE LOSS RATE (µm)/mmHg/day (ALL SECTORS AVERAGE) | | | | | | | | |
|---|---|---|---|---|---|---|---|---|---|
| TIME | RNFL | | | GCL | | | RETINA | | |
| | EPI | Ms 38/20 | Ms 20/10 | EPI | Ms 38/20 | Ms 20/10 | EPI | Ms 38/20 | Ms 20/10 |
| 2w | 0.0743 | -0.1595 | -0.0454 | 0.0040 | -0.0721 | -0.0372 | -0.0492 | -0.2716 | -0.0110 |
| 4w | 0.0438 | 0.0029 | -0.0116 | 0 | -0.0100 | -0.0030 | -0.0601 | -0.0170 | -0.0268 |
| 6w | -0.0024 | -0.0094 | -0.0080 | -0.0043 | -0.0134 | -0.0105 | -0.0063 | -0.0295 | -0.0350 |
| 8w | -0.0033 | -0.0102 | -0.0030 | -0.009 | -0.0072 | -0.0059 | -0.0095 | -0.0221 | -0.0145 |
| AVERAGE | 0.0281 | -0.0440 | -0.017 | -0.0018 | -0.0257 | -0.0142 | -0.0313 | -0.0851 | -0.021 |

**Table 3: Right eye neuro-retinal loss rate measured by optical coherence tomography (OCT) in the ocular hypertensive models**. EPI: episcleral sclerosis model; Ms 38/20: microsphere sized 38/20 model, Ms 20/10: microsphere sized 20/10 model; RNFL: Retinal Nerve Fiber Layer; GCL: Ganglion cell layer complex; RE: right eye; thickness in microns (µm); w: week. Grey color cells showed the lowest measurements.

As comparable results were found between EPI and Ms 20/10 models, deeper analysis was performed and statistical differences were only found in 12 from 135 OCT parameters (table 4), so models resulted highly comparable . The figure 6 showed the RE neuro-retinal changes by the average of thickness percentage loss up to week 8. The EPI model had the more intense and prompt loss in RNFL thickness from week 4 to 6, however the Ms 20/10 model showed a progressive decrease but reached the biggest percentage loss in GCL at later time (8 week).

| OCT PARAMETERS (µm) | BASELINE | | 2w | | | 4w | | | 6w | | | 8w | | |
|---|---|---|---|---|---|---|---|---|---|---|---|---|---|---|
| | Mean±SD | p | Mean±SD | %Ch | p | Mean±SD | %Ch | p | Mean±SD | %Ch | p | Mean±SD | %Ch | p |
| **RETINAL THICKNESS** | | | | | | | | | | | | | | |
| CENTRAL | 273.80±14.57 | 0.182 | 271.66±12.22 | -0.78 | 0.727 | 271.33±21.38 | -0.90 | 0.827 | 265.12±19.04 | -3.17 | 0.315 | 265.91±14.79 | -2.88 | 0.123 |
| | 266.69±20.06 | | 268.61±21.20 | 0.72 | | 265.95±20.00 | -0.27 | | 259.76±20.88 | -2.59 | | 258.10±14.88 | -3.22 | |
| INNER INFERIOR | 259.90±17.17 | 0.975 | 261.66±12.74 | 0.68 | 0.896 | 260.66±4.50 | 0.29 | 0.726 | 261.68±10.22 | 0.68 | 0.256 | 256.16±9.18 | -1.44 | 0.256 |
| | 261.46±12.86 | | 262.19±9.99 | 0.27 | | 260.19±13.66 | -0.48 | | 257.66±13.18 | -1.45 | | 251.78±11.91 | -3.70 | |
| OUTER INFERIOR | 258.30±13.59 | 0.780 | 243.33±17.61 | -5.80 | 0.073 | 244.33±6.11 | -5.41 | 0.080 | 259.08±13.82 | 0.30 | 0.164 | 246.58±10.57 | -4.54 | 0.612 |
| | 258.62±10.18 | | 258.90±8.70 | 0.10 | | 255.29±12.19 | -1.28 | | 252.14±11.11 | -2.50 | | 248.68±11.75 | -3.84 | |
| INNER SUPERIOR | 256.70±12.38 | 0.291 | 261.00±7.21 | 1.68 | 0.186 | 244.00±4.35 | -4.95 | 0.485 | 254.96±14.92 | -0.68 | **0.049** | 255.00±24.93 | -0.66 | 0.319 |
| | 250.77±12.89 | | 252.60±14.21 | 0.72 | | 250.95±15.77 | 0.07 | | 246.38±14.81 | -1.75 | | 245.72±16.93 | -2.01 | |
| OUTER SUPERIOR | 267.40±15.02 | 0.263 | 262.33±6.50 | -1.90 | 0.250 | 245.00±9.53 | -8.38 | 0.105 | 262.80±15.22 | -1.72 | **0.049** | 257.66±18.63 | -3.64 | 0.610 |
| | 260.23±9.71 | | 258.32±11.24 | -0.73 | | 257.62±13.51 | -1.00 | | 254.00±13.93 | -2.39 | | 254.52±19.25 | -2.19 | |
| INNER NASAL | 258.10±12.99 | 0.901 | 261.00±8.18 | 1.12 | 0.512 | 250.00±4.58 | -3.14 | 0.274 | 255.40±14.03 | -1.05 | 0.310 | 256.00±14.07 | -0.81 | 0.529 |
| | 257.00±10.73 | | 256.71±9.52 | -0.11 | | 257.48±13.46 | 0.18 | | 251.47±12.83 | -2.15 | | 251.84±12.02 | -2.00 | |
| OUTER NASAL | 261.22±12.42 | 0.462 | 253.66±2.51 | -2.89 | 0.384 | 244.33±6.65 | -6.47 | 0.088 | 257.62±10.87 | -1.38 | 0.103 | 253.50±15.90 | -2.96 | 0.626 |
| | 258.15±8.90 | | 258.10±9.78 | -0.01 | | 256.62±13.72 | -0.59 | | 251.23±14.09 | -2.68 | | 252.41±11.75 | -2.22 | |
| INNER TEMPORAL | 261.00±15.84 | 0.841 | 259.00±3.60 | -0.77 | 0.999 | 247.66±4.61 | -5.11 | 0.359 | 256.00±16.79 | -1.92 | 0.508 | 256.58±29.11 | -1.69 | 0.792 |
| | 259.15±14.12 | | 257.29±11.25 | -0.71 | | 254.71±14.14 | -1.71 | | 252.09±14.89 | -2.72 | | 247.63±13.63 | -4.44 | |
| OUTER TEMPORAL | 261.80±13.27 | 0.804 | 253.00±2.64 | -3.36 | 0.238 | 243.33±7.50 | -7.06 | 0.088 | 263.56±19.89 | 0.67 | 0.087 | 258.58±26.94 | -1.23 | 0.570 |
| | 291.77±11.56 | | 257.90±10.31 | -1.47 | | 255.81±12.46 | -2.27 | | 253.95±13.09 | -2.98 | | 250.89±14.55 | -4.15 | |

**RIGHT EYE STRUCTURAL NEURORETINAL MEASUREMENTS ACCORDING TO OHT MODELS (EPI vs Ms 20/10)**

| | Value | p | Value | Δ% | p | Value | Δ% | p | Value | Δ% | p | Value | Δ% | p |
|---|---|---|---|---|---|---|---|---|---|---|---|---|---|---|
| **TOTAL VOLUME** | 1.93±0.09 | 0.418 | 1.82±0.00 | -5.70 | 0.003# | 1.76±0.05 | -8.81 | 0.001# | 1.83±0.10 | -5.18 | 0.204 | 1.81±0.11 | -6.22 | 0.597 |
| | 1.88±0.10 | | 1.92±0.23 | 2.07 | | 1.96±0.07 | 3.78 | | 1.80±0.09 | -4.68 | | 1.74±0.15 | -7.86 | |
| **RNFL THICKNESS** | | | | | | | | | | | | | | |
| **GLOBAL** | 43.44±2.69 | 0.267 | 47.66±6.35 | 9.71 | 0.179 | 50.66±7.76 | 16.64 | 0.148 | 42.96±4.42 | -1.10 | 0.311 | 41.45±3.53 | -4.58 | 0.367 |
| | 44.84±3.64 | | 43.70±4.81 | -2.55 | | 44.05±6.61 | -1.77 | | 43.50±6.47 | -3.00 | | 43.44±9.80 | -3.13 | |
| **INFERIOR TEMPORAL** | 46.20±5.13 | 0.732 | 46.00±16.82 | -0.43 | 0.615 | 42.66±3.21 | -7.64 | 0.631 | 45.40±10.31 | -1.73 | 0.721 | 40.36±7.24 | -12.64 | 0.072 |
| | 50.54±13.31 | | 51.50±15.47 | 1.89 | | 47.47±14.95 | -6.07 | | 48.11±15.02 | -4.80 | | 48.94±16.42 | -3.16 | |
| **INFERIOR NASAL** | 45.20±7.99 | 0.400 | 52.66±5.77 | 16.50 | 0.437 | 48.66±1.52 | 7.68 | 0.886 | 46.96±8.76 | 3.89 | 0.786 | 45.45±5.57 | 0.55 | 0.290 |
| | 49.15±11.43 | | 48.00±17.56 | -2.33 | | 49.00±14.78 | -0.30 | | 47.33±11.14 | -3.70 | | 49.05 | -0.20 | |
| **SUPERIOR TEMPORAL** | 44.89±10.56 | 0.999 | 54.66±9.23 | 21.76 | 0.027 | 62.66±14.74 | 39.61 | 0.044 | 38.75±15.03 | -13.68 | 0.469 | 43.72±11.84 | -2.61 | 0.499 |
| | 42.46±8.05 | | 39.37±10.58 | -7.27 | | 42.47±10.99 | 0.02 | | 42.61±12.53 | 0.35 | | 40.33±13.88 | -5.01 | |
| **SUPERIOR NASAL** | 41.44±4.06 | 0.947 | 44.66±7.23 | 7.77 | 0.201 | 56.00±20.07 | 35.14 | 0.150 | 33.36±13.67 | -19.50 | 0.107 | 36.00±14.58 | -13.13 | 0.719 |
| | 39.23±13.08 | | 36.75±11.84 | -6.32 | | 39.47±15.67 | 0.60 | | 39.88±15.05 | 1.65 | | 35.88±17.52 | -8.54 | |
| **NASAL** | 39.30±6.68 | 0.320 | 46.66±5.03 | 18.73 | 0.119 | 43.66±2.30 | 11.12 | 0.363 | 43.76±7.82 | 11.35 | 0.004# | 41.81±3.40 | 6.39 | 0.215 |
| | 43.31±9.18 | | 39.35±10.40 | -9.14 | | 40.42±9.92 | -6.67 | | 38.33±8.87 | -11.49 | | 39.94±16.86 | -7.78 | |
| **TEMPORAL** | 42±10.89 | 0.709 | 43.66±8.02 | 3.98 | 0.464 | 53.00±15.71 | 26.19 | 0.631 | 45.32±10.50 | 7.90 | 0.482 | 41.18±7.11 | -1.95 | 0.170 |
| | 45.54±6.72 | | 47.20±7.45 | 3.64 | | 46.42±7.64 | 1.93 | | 46.44±8.43 | 1.97 | | 46.94±13.43 | 3.07 | |
| **GCL THICKNESS** | | | | | | | | | | | | | | |
| **CENTRAL** | 20.50±2.66 | 0.321 | 22.00±3.00 | 7.32 | 0.040 | 20.66±1.52 | 0.78 | 0.455 | 17.76±3.19 | -13.37 | 0.885 | 18.92±3.47 | -7.71 | 0.009# |
| | 19.16±3.48 | | 18.52±2.46 | -3.35 | | 19.61±3.00 | 2.31 | | 17.71±3.13 | -7.60 | | 15.68±2.23 | -18.19 | |

| Sector | Value | p | Value | %Ch | p | Value | %Ch | p | Value | %Ch | p | Value | %Ch | p |
|---|---|---|---|---|---|---|---|---|---|---|---|---|---|---|
| INNER INFERIOR | 26.50±3.78 | 0.877 | 24.00±3.00 | -9.43 | 0.309 | 25.66±0.57 | -3.17 | 0.560 | 23.48±4.23 | -11.40 | 0.548 | 26.25±2.05 | -0.94 | 0.001# |
| | 26.44±3.04 | | 25.71±2.17 | -2.76 | | 26.33±1.65 | -0.41 | | 24.42±2.87 | -7.63 | | 22.21±4.32 | -15.99 | |
| OUTER INFERIOR | 27.40±1.67 | 0.596 | 26.00±2.64 | -5.11 | 0.627 | 24.66±2.08 | -10.00 | 0.170 | 24.48±4.31 | -10.66 | 0.697 | 26.25±3.59 | -4.20 | 0.276 |
| | 26.25±2.25 | | 25.43±2.73 | -3.12 | | 26.57±2.18 | 1.21 | | 25.66±2.30 | -2.24 | | 22.74±5.68 | -13.37 | |
| INNER SUPERIOR | 26.17±2.63 | 0.570 | 26.33±2.30 | 0.61 | 0.119 | 23.00±1.73 | -12.11 | 0.508 | 22.84±3.28 | -12.72 | 0.380 | 22.42±4.18 | -14.33 | 0.079 |
| | 25.25±2.17 | | 22.80±3.59 | -9.70 | | 23.80±3.40 | -5.74 | | 21.71±3.73 | -14.01 | | 20.06±3.88 | -20.55 | |
| OUTER SUPERIOR | 25.50±3.93 | 0.669 | 26.33±2.30 | 3.25 | 0.524 | 23.00±3.46 | -9.80 | 0.186 | 25.04±2.74 | -1.80 | 0.417 | 23.50±3.89 | -7.84 | 0.754 |
| | 26.33±2.27 | | 25.37±1.89 | -3.64 | | 25.57±2.27 | -2.88 | | 24.80±2.69 | -5.81 | | 22.71±5.12 | -13.74 | |
| INNER NASAL | 23.83±4.35 | 0.706 | 24.00±1.00 | 0.71 | 0.235 | 20.66±4.04 | -13.30 | 0.233 | 21.88±4.98 | -8.18 | 0.723 | 23.50±1.78 | -1.38 | 0.034 |
| | 23.25±2.83 | | 21.95±3.41 | -5.59 | | 22.61±3.35 | -2.75 | | 21.71±4.06 | -6.62 | | 20.21±4.45 | -13.07 | |
| OUTER NASAL | 23.67±4.13 | 0.182 | 26.33±0.57 | 11.24 | 0.129 | 24.00±3.00 | 1.39 | 0.626 | 23.79±5.25 | 0.51 | 0.299 | 25.50±1.62 | 7.73 | 0.140 |
| | 25.75±2.05 | | 23.60±3.88 | -8.34 | | 24.71±2.55 | -4.03 | | 23.19±4.30 | -9.94 | | 22.76±5.19 | -11.61 | |
| INNER TEMPORAL | 21.67±5.50 | 0.963 | 23.33±2.51 | 7.66 | 0.271 | 23.33±2.88 | 7.66 | 0.566 | 21.40±4.76 | -1.25 | 0.207 | 23.50±4.70 | 8.44 | 0.018# |
| | 21.83±5.00 | | 21.10±3.41 | -3.34 | | 22.71±3.75 | 4.03 | | 20.00±3.80 | -8.38 | | 19.68±3.74 | -9.84 | |
| OUTER TEMPORAL | 25.17±3.97 | 0.925 | 24.66±2.51 | -1.99 | 0.826 | 24.66±2.88 | -2.03 | 0.628 | 24.64±4.56 | -2.11 | 0.083 | 25.42±3.47 | 0.99 | 0.147 |
| | 24.75±3.74 | | 23.76±3.72 | -4.00 | | 24.95±3.33 | 0.80 | | 23.19±3.85 | -6.30 | | 23.26±4.70 | -6.02 | |
| TOTAL VOLUME | 0.17±0.02 | 0.942 | 0.17±0.00 | 0.00 | 0.363 | 0.16±0.01 | -5.88 | 0.670 | 0.13±0.07 | -23.53 | 0.787 | 0.15±0.04 | -11.76 | 0.076 |
| | 0.17±0.02 | | 0.16±0.01 | -5.55 | | 0.17±0.01 | -1.67 | | 0.15±0.03 | -13.24 | | 0.14±0.02 | -19.02 | |

Table 4: **Right eye neuro-retinal analysis follow-up by optical coherence tomography (OCT) in both ocular hypertensive models**. EPI: epiescleral sclerosis model; Ms 20/10: microsphere sized 20/10 model; RNFL: Retina Nerve Fiber Layer; GCL: Ganglion cell layer complex; thickness in microns (μm); mean ± SD (SD: standard deviation); %Ch: percentage change of thickness loss; p<0.050 statistical significance; p<0.020# statistical significance with Bonferroni correction for multiple comparisons; w: week. Grey cells colored when EPI model showed thinner sectors or/and higher percentage loss compared to Ms 20/10.

## 4.1.4. Functional neuroretinal analysis by electroretinography

Lower values according to the b-wave amplitude were recorded with scotopic flash ERG for both Ms 38/20 and Ms 20/10 models at week 8. Although slightly lower signals were found for Ms 38/20 model compared to the Ms 20/10 model (figure 7), no statistical differences were found between them.

According to the presented results, and due to the similarities found between the episcleral model and the Ms20/10 model. A long-term study was planned comparing both methods.

## 4.2. STUDY OF REPRODUCIBILITY OF MS (20/10) FOR 24 WEEKS of follow-up and comparison with the episcleral model.

### 4.2.1. Microspheres characterization

The PLGA non-loaded Ms (size range 20-10 $\mu$m) used were the same used in the preliminary study (see Table 1 and figure 1).

### 4.2.2. Ophthalmological clinical signs and intraocular pressure

None case of infection, severe intraocular inflammation or retinal detachment or cataract formation and better preserved corneal surface was found in Ms20/10 model, compared with the EPI model. In the Ms20/10 model microspheres were seen in the anterior chamber of the eye during the 24 weeks (figure 8). IOP values observed in the Ms20/10 model showed a progressive increase over the study, with levels higher than 20 mmHg since week 12. On the contrary, in the case of EPI model, OHT levels were reached since week 1, they were maintained until week 10 but, after that, the IOP sustainably decreased. The comparative analysis between both models revealed that IOP from EPIm was statistically higher than the Ms20/10 model for the first half of the study (figure 9).

### 4.2.3. Structural neuroretinal analysis by optical coherence tomography

Ms20/10 model showed statistical tendency to **decrease in thickness** of Retina, RNFL and GCL over time (table 5) as EPIm also did (figure 10).

| RIGHT EYE MICROSPHERES 20/10 MODEL | | | | | | | | | | | | | | | | |
|---|---|---|---|---|---|---|---|---|---|---|---|---|---|---|---|---|
| | BASELINE | 8W | | | | 12W | | | 18W | | | 24W | | | | |
| OCT PARAMETERS (µm) | | | | | | | | | | | | | | | | |
| | Mean±SD | Mean±SD | %Ch | P | | Mean±SD | %Ch | P | Mean±SD | %Ch | P | Mean±SD | %Ch | P | | |
| **RETINAL THICKNESS** | | | | | | | | | | | | | | | | |
| CENTRAL | 271.04±13.48 | 267.50±10.68 | -1.31 | 0.347 | | 268.80±17.72 | -0.83 | 0.500 | 261.60±9.91 | -3.48 | 0.893 | 265.00±22.45 | -2.23 | 0.600 | | |
| INNER INFERIOR | 256.54±7.39 | 253.42±8.59 | -1.22 | 0.237 | | 250.60±8.50 | -2.32 | 0.221 | 245.40±10.13 | -4.34 | 0.043 | 247.50±14.48 | -3.52 | 0.116 | | |
| OUTER INFERIOR | 246.33±5.94 | 246.42±10.71 | 0.04 | 0.802 | | 239.20±6.09 | -2.89 | 0.345 | 234.20±2.58 | -4.92 | **0.042** | 234.33±10.25 | -4.87 | **0.028** | | |
| INNER SUPERIOR | 251.08±7.70 | 253.75±25.36 | 1.06 | 0.846 | | 242.60±15.50 | -3.38 | 0.345 | 238.20±6.68 | -5.13 | **0.041** | 246.67±16.23 | -1.76 | 0.752 | | |
| OUTER SUPERIOR | 250.21±6.37 | 255.33±20.02 | 2.05 | 0.573 | | 244.40±7.43 | -2.32 | 0.581 | 238.20±6.18 | -4.80 | **0.043** | 249.00±10.67 | -0.48 | 0.753 | | |
| INNER NASAL | 253.21±6.93 | 253.83±15.81 | 0.24 | 0.931 | | 245.40±9.39 | -3.08 | 0.144 | 244.60±7.63 | -3.40 | **0.043** | 244.83±15.80 | -3.31 | **0.027** | | |
| OUTER NASAL | 248.00±6.03 | 252.67±16.90 | 1.88 | 0.608 | | 242.20±9.09 | -2.34 | 0.683 | 238.00±5.61 | -4.03 | **0.043** | 242.00±10.90 | -2.42 | 0.116 | | |

| | | | | | | | | | | | | |
|---|---|---|---|---|---|---|---|---|---|---|---|---|
| INNER TEMPORAL | 253.04±10.20 | 257.33±28.91 | 1.70 | 0.928 | 244.20±8.10 | -3.49 | 0.345 | 239.40±5.45 | -5.39 | **0.043** | 247.33±14.41 | -2.26 | 0.078 |
| OUTER TEMPORAL | 248.38±7.42 | 259.42±26.63 | 4.44 | 0.365 | 246.20±10.08 | -0.88 | 0.225 | 242.20±2.86 | -2.49 | 0.078 | 250.67±12.43 | 0.92 | 0.500 |
| TOTAL VOLUME | 1.71±0.36 | 1.73±0.05 | 1.17 | **0.038** | 1.74±0.06 | 1.75 | 0.126 | 1.71±0.36 | -0.58 | 0.500 | 1.75±0.08 | 2.24 | **0.027** |
| **RNFL THICKNESS** | | | | | | | | | | | | |
| GLOBAL | 46.00±4.40 | 42.36±6.26 | -7.91 | **0.040** | 44.20±3.27 | -3.91 | 0.345 | 39.80±2.77 | -13.48 | **0.042** | 40.17±8.65 | -12.67 | **0.043** |
| INFERIOR TEMPORAL | 46.75±6.52 | 42.73±7.86 | -8.60 | **0.008#** | 45.00±6.96 | -3.74 | 0.990 | 36.80±4.65 | -21.28 | **0.042** | 44.67±12.12 | -4.45 | 0.207 |
| INFERIOR NASAL | 46.96±6.19 | 44.82±5.87 | -4.56 | 0.814 | 51.40±6.80 | 9.45 | 0.990 | 40.20±5.58 | -14.40 | 0.068 | 46.17±10.77 | -1.68 | 0.458 |
| SUPERIOR TEMPORAL | 49.38±8.15 | 40.64±11.73 | -17.70 | **0.012#** | 44.00±9.43 | -10.90 | 0.080 | 37.40±4.09 | -24.26 | **0.043** | 41.00±12.61 | -16.97 | **0.046** |
| SUPERIOR NASAL | 39.00±8.20 | 32.64±16.72 | -16.31 | 0.548 | 35.40±4.03 | -9.23 | **0.042** | 36.80±7.53 | -5.64 | **0.043** | 29.50±9.99 | -24.36 | **0.046** |
| NASAL | 43.54±6.15 | 42.82±6.33 | -1.65 | 0.878 | 41.80±6.90 | -4.00 | 0.144 | 40.60±5.94 | -6.75 | **0.042** | 38.67±12.06 | -11.19 | 0.075 |
| TEMPORAL | 49.21±8.21 | 46.18±9.71 | -6.16 | 0.095 | 47.20±6.09 | -4.08 | 0.683 | 42.60±5.89 | -13.43 | **0.042** | 41.83±8.32 | -15.00 | **0.027** |
| **GCL THICKNESS** | | | | | | | | | | | | |
| CENTRAL | 22.46±2.10 | 19.42±3.23 | -13.54 | **0.033** | 19.40±2.70 | -13.62 | 0.066 | 18.20±2.49 | -18.97 | **0.043** | 17.67±3.55 | -21.33 | **0.027** |
| INNER INFERIOR | 28.29±1.57 | 26.33±2.10 | -6.93 | **0.002#** | 25.20±1.64 | -10.92 | 0.131 | 25.40±1.51 | -10.22 | **0.042** | 24.17±3.25 | -14.56 | **0.042** |
| OUTER INFERIOR | 27.25±1.35 | 26.58±3.50 | -2.46 | 0.441 | 24.40±1.67 | -10.46 | 0.066 | 24.80±0.83 | -8.99 | **0.042** | 22.17±4.79 | -18.64 | **0.027** |
| INNER SUPERIOR | 26.96±1.92 | 22.08±3.82 | -18.10 | **0.006#** | 23.60±3.64 | -12.46 | **0.042** | 21.20±1.09 | -21.36 | **0.043** | 21.17±3.97 | -21.48 | **0.042** |
| OUTER SUPERIOR | 25.71±2.57 | 22.83±3.71 | -11.20 | **0.037** | 24.20±2.77 | -5.87 | 0.285 | 24.80±0.83 | -3.54 | 0.492 | 22.00±5.21 | -14.43 | 0.168 |
| INNER NASAL | 26.38±2.33 | 22.83±2.03 | -13.42 | **0.004#** | 23.80±2.38 | -9.75 | 0.257 | 22.60±1.81 | -14.30 | **0.042** | 21.67±5.78 | -17.82 | **0.027** |
| OUTER NASAL | 26.96±1.75 | 24.92±2.02 | -7.57 | **0.013#** | 24.40±2.07 | -9.50 | 0.059 | 24.80±0.83 | -8.01 | 0.066 | 21.50±3.93 | -20.25 | **0.027** |
| INNER TEMPORAL | 26.33±2.18 | 23.08±4.83 | -12.34 | **0.044** | 23.80±1.92 | -9.61 | 0.063 | 21.60±4.61 | -17.96 | **0.039** | 21.50±4.18 | -18.34 | 0.058 |
| OUTER TEMPORAL | 27.46±1.56 | 25.17±3.38 | -8.34 | 0.061 | 24.80±2.86 | -9.69 | 0.066 | 24.00±2.82 | -12.60 | **0.042** | 22.50±3.14 | -18.06 | **0.026** |
| TOTAL VOLUME | 0.19±0.01 | 0.17±0.01 | -9.96 | **0.047** | 0.17±0.01 | -9.58 | 0.059 | 0.17±0.01 | -10.66 | **0.042** | 0.15±0.03 | -18.35 | **0.042** |

**Table 5: Structural analysis of neuro-retina by optical coherence tomography in right eyes (Ms20/10 model)**. OCT: optical coherence tomography; RNFL: Retinal Nerve Fiber Layer; GCL: Ganglion Cell Layer complex; thickness in microns (µm); mean±SD (SD: standard deviation); %Ch: percentage change of thickness loss (with respect to baseline); p<0.05 statistical significance; p<0.02# statistical significance with Bonferroni correction for multiple comparisons. Black upward arrow: tendency to increase in thickness between consecutive explorations.

Both models were compared and no statistical differences were found in thickness in most parameters analyzed. In fact, only 6 from the 135 OCT parameters studied showed statistical differences between models. In general, EPIm showed a tendency to retinal thicker (in fair grey color) and GCL thinner (in dark grey color) over time (table 6).

| RE STRUCTURAL NEURORETINAL MEASUREMENTS ACCORDING TO THE OHT MODEL (EPIm vs Ms20/10) | | | | | | | | | | |
|---|---|---|---|---|---|---|---|---|---|---|
| **OCT PARAMETERS (µm)** | **BASELINE** Mean±SD | p | **8w** Mean±SD | p | **12w** Mean±SD | p | **18w** Mean±SD | p | **24w** Mean±SD | p |
| **RETINAL THICKNESS** | | | | | | | | | | |
| **CENTRAL** | 271.08±14.04 | 0.904 | 265.92±14.79 | 0.794 | 265.22±18.72 | 0.655 | 253.50±11.67 | 0.268 | 255.00±18.17 | 0.253 |
| | 271.04±13.48 | | 267.50±10.68 | | 268.80±17.72 | | 261.60±9.91 | | 265.00±22.45 | |
| **INNER INFERIOR** | 256.24±0.14 | 0.904 | 256.17±9.18 | 0.385 | 254.83±9.19 | 0.478 | 248.50±10.66 | 0.902 | 245.00±12.91 | 0.668 |
| | 256.54±7.39 | | 253.42±8.59 | | 250.60±8.50 | | 245.40±10.13 | | 247.50±14.48 | |
| **OUTER INFERIOR** | 247.48±6.75 | 0.520 | 246.58±10.57 | 0.977 | 251.06±11.36 | **0.036** | 242.25±10.50 | 0.221 | 237.43±11.74 | 0.568 |
| | 246.33±5.94 | | 246.42±10.71 | | 239.20±6.09 | | 234.20±2.58 | | 234.33±10.25 | |
| **INNER SUPERIOR** | 250.12±8.09 | 0.787 | 255.00±24.93 | 0.750 | 253.11±23.62 | 0.179 | 240.25±3.50 | 0.539 | 240.86±10.18 | 0.317 |
| | 251.08±7.70 | | 253.75±25.36 | | 242.60±15.50 | | 238.20±6.68 | | 246.67±16.23 | |
| **OUTER SUPERIOR** | 250.88±6.39 | 0.367 | 257.67±18.63 | 0.543 | 260.44±22.89 | **0.048** | 246.25±8.99 | 0.268 | 245.57±15.61 | 0.775 |
| | 250.21±6.37 | | 255.33±20.02 | | 244.40±7.43 | | 238.20±6.18 | | 249.00±10.67 | |
| **INNER NASAL** | 253.32±6.44 | 0.880 | 256.00±14.07 | 0.728 | 254.39±17.03 | 0.191 | 251.75±7.67 | 0.176 | 247.71±17.28 | 0.431 |
| | 253.21±6.93 | | 253.83±15.81 | | 245.40±9.39 | | 244.60±7.63 | | 244.83±15.80 | |
| **OUTER NASAL** | 249.00±6.29 | 0.521 | 253.50±15.90 | 0.907 | 255.33±18.95 | 0.080 | 246.75±6.13 | 0.059 | 250.00±8.22 | 0.520 |

| | | | | | | | | | | |
|---|---|---|---|---|---|---|---|---|---|---|
| | 248.00±6.03 | | 252.67±16.90 | | 242.20±9.09 | | 238.00±5.61 | | 242.00±10.90 | |
| INNER TEMPORAL | 253.20±10.02 | 0.976 | 256.58±29.11 | 0.750 | 253.61±22.14 | 0.232 | 243.50±6.13 | 0.327 | 248.29±13.62 | 0.250 |
| | 253.04±10.20 | | 252.67±16.90 | | 244.20±8.10 | | 239.40±5.45 | | 247.33±14.41 | |
| OUTER TEMPORAL | 249.96±8.57 | 0.560 | 258.58±26.94 | 0.729 | 259.78±24.89 | 0.117 | 249.25±5.37 | **0.036** | 252.00±16.41 | 0.943 |
| | 248.38±7.42 | | 259.42±26.63 | | 246.20±10.08 | | 242.20±2.86 | | 250.67±12.43 | |
| TOTAL VOLUME | 1.97±0.07 | <0.001[#] | 1.81±0.11 | 0.064 | 1.81±0.11 | 0.156 | 1.74±0.03 | 0.138 | 1.71±0.16 | 0.720 |
| | 1.71±0.36 | | 1.73±0.05 | | 1.74±0.06 | | 1.71±0.36 | | 1.75±0.08 | |
| **RNFL THICKNESS** | | | | | | | | | | |

| | | | | | | | | | | |
|---|---|---|---|---|---|---|---|---|---|---|
| GLOBAL | 46.16±4.36 | 0.880 | 41.45±3.53 | 0.974 | 43.15±9.53 | 0.414 | 41.75±5.12 | 0.532 | 40.86±4.22 | 0.474 |
| | 46.00±4.40 | | 42.36±6.26 | | 44.20±3.27 | | 39.80±2.77 | | 40.17±8.65 | |
| INFERIOR TEMPORAL | 48.72±11.03 | 0.764 | 40.36±7.24 | 0.666 | 41.80±12.46 | **0.563** | 35.75±11.26 | 0.219 | 38.29±4.46 | 0.283 |
| | 46.75±6.52 | | 42.73±7.86 | | 45.00±6.96 | | 36.80±4.65 | | 44.67±12.12 | |
| INFERIOR NASAL | 48.40±8.23 | 0.666 | 45.45±5.57 | 0.691 | 45.10±9.33 | 0.134 | 41.00±13.54 | 0.537 | 42.43±8.77 | 0.774 |
| | 46.96±6.19 | | 44.82±5.87 | | 51.40±6.80 | | 40.20±5.58 | | 46.17±10.77 | |
| SUPERIOR TEMPORAL | 48.32±8.82 | 0.645 | 43.73±11.85 | 0.598 | 43.60±22.02 | 0.454 | 40.50±7.32 | 0.461 | 40.00±12.66 | 0.721 |
| | 49.38±8.15 | | 40.64±11.73 | | 44.00±9.43 | | 37.40±4.09 | | 41.00±12.61 | |
| SUPERIOR NASAL | 38.56±9.40 | 0.952 | 36.00±14.58 | 0.307 | 33.55±22.62 | 0.324 | 34.00±2.16 | 0.624 | 35.57±8.65 | 0.317 |

| | | | | | | | | | |
|---|---|---|---|---|---|---|---|---|---|
| | 39.00±8.20 | | 32.64±16.72 | | 35.40±4.03 | | 36.80±7.53 | | 29.50±9.99 |
| **NASAL** | 43.68±7.12 | 0.968 | 41.82±3.40 | 0.894 | 43.15±8.52 | 0.973 | 45.25±6.29 | 0.221 | 43.29±3.54 |
| | 43.54±6.15 | | 42.82±6.33 | | 41.80±6.90 | | 40.60±5.94 | | 38.67±12.06 |
| | | | | | | | | | 0.719 |
| **TEMPORAL** | 48.80±8.41 | 0.833 | 41.18±7.11 | 0.156 | 47.40±12.04 | 0.539 | 45.75±10.24 | 0.806 | 42.43±8.03 |
| | 49.21±8.21 | | 46.18±9.71 | | 47.20±6.09 | | 42.60±5.89 | | 41.83±8.32 |
| | | | | | | | | | 0.943 |

**GCL THICKNESS**

| | | | | | | | | | |
|---|---|---|---|---|---|---|---|---|---|
| **CENTRAL** | 22.32±2.30 | 0.951 | 18.92±3.47 | 0.663 | 16.78±3.59 | 0.107 | 15.25±1.50 | 0.080 | 15.57±3.78 |
| | 22.46±2.10 | | 19.42±3.23 | | 19.40±2.70 | | 18.20±2.49 | | 17.67±3.55 |
| | | | | | | | | | 0.195 |

| | | | | | | | | | |
|---|---|---|---|---|---|---|---|---|---|
| **INNER INFERIOR** | 27.88±2.10 | 0.610 | 26.25±2.05 | 0.741 | 23.50±4.79 | 0.650 | 24.00±1.41 | 0.167 | 21.57±5.59 |
| | 28.29±1.57 | | 26.33±2.10 | | 25.20±1.64 | | 25.40±1.51 | | 24.17±3.25 |
| | | | | | | | | | 0.109 |
| **OUTER INFERIOR** | 26.84±1.77 | 0.575 | 26.25±3.59 | 0.497 | 24.44±4.09 | 0.218 | 23.25±2.98 | 0.366 | 22.14±6.28 |
| | 27.25±1.35 | | 26.58±3.50 | | 24.40±1.67 | | 24.80±0.83 | | 22.17±4.79 |
| | | | | | | | | | 0.560 |
| **INNER SUPERIOR** | 26.40±1.58 | 0.324 | 22.42±4.18 | 0.680 | 21.50±4.50 | 0.142 | 18.00±2.44 | **0.044** | 17.57±4.92 |
| | 26.96±1.92 | | 22.08±3.82 | | 23.60±3.64 | | 21.20±1.09 | | 21.17±3.97 |
| | | | | | | | | | 0.150 |
| **OUTER SUPERIOR** | 25.40±2.50 | 0.647 | 23.50±3.89 | 0.520 | 22.44±3.60 | 0.228 | 24.75±2.21 | 0.900 | 20.14±5.64 |
| | 25.71±2.57 | | 22.83±3.71 | | 24.20±2.77 | | 24.80±0.83 | | 22.00±5.21 |
| | | | | | | | | | 0.387 |
| **INNER NASAL** | 26.16±2.35 | 0.723 | 23.50±1.78 | 0.379 | 21.56±5.05 | 0.410 | 19.00±4.08 | 0.082 | 19.57±5.31 |
| | | | | | | | | | 0.221 |

| | | | | | | | | | | |
|---|---|---|---|---|---|---|---|---|---|---|
| | 26.38±2.33 | | 22.83±2.03 | | 23.80±2.38 | | 22.60±1.81 | | 21.67±5.78 | |
| OUTER NASAL | 26.68±1.72 | 0.569 | 25.50±1.62 | 0.479 | 22.00±6.29 | 0.499 | 22.25±4.99 | 0.530 | 22.00±1.78 | 0.685 |
| | 26.96±1.75 | | 24.92±2.02 | | 24.40±2.07 | | 24.80±0.83 | | 21.50±3.93 | |
| INNER TEMPORAL | 25.56±3.34 | 0.520 | 23.50±4.70 | 0.748 | 20.89±5.31 | 0.032 | 20.25±1.70 | 0.268 | 18.71±4.92 | 0.425 |
| | 26.33±2.18 | | 23.08±4.83 | | 23.80±1.92 | | 21.60±4.61 | | 21.50±4.18 | |
| OUTER TEMPORAL | 26.72±2.57 | 0.349 | 25.42±3.47 | 0.605 | 25.00±5.16 | 0.733 | 25.00±1.41 | 0.898 | 20.43±5.28 | 0.278 |
| | 27.46±1.56 | | 25.17±3.38 | | 24.80±2.86 | | 24.00±2.82 | | 22.50±3.14 | |
| TOTAL VOLUME | 0.18±0.01 | 0.668 | 0.16±0.00 | 0.914 | 0.16±0.02 | 0.259 | 0.15±0.01 | 0.093 | 0.13±0.03 | 0.343 |

| | | | | | | | | | |
|---|---|---|---|---|---|---|---|---|---|
| | 0.19 ±0.01 | | 0.17±0.01 | | 0.17±0.01 | | 0.17±0.01 | | 0.15±0.03 |

Table 6: Structural analysis of neuro-retina by optical coherence tomography in right eyes (Ms20/10 model).

OCT: optical coherence tomography; RNFL: Retinal Nerve Fiber Layer; GCL: Ganglion Cell Layer complex; thickness in microns (µm); mean±SD (SD: standard deviation); %Ch: percentage change of thickness loss (with respect to baseline); $p < 0.05$ statistical significance; $p < 0.02$# statistical significance with Bonferroni correction for multiple comparisons. Black upward arrow: tendency to increase in thickness between consecutive explorations.

The percentage loss in thickness was also quantified. In both models, the inner sectors of the superior-inferior axis in Retina and RNFL experienced the highest percentage thickness loss at every late time examined (figure 11).

The figure 12 showed fluctuations but with tendency to higher percentage loss over time. The highest average percentage loss in both models was in GCL, followed by RNFL and finally Retina; as it also occurred in the preliminary study. In average, EPIm suffered bigger loss than Ms20/10.

In this longer 24-week study the Ms20/10 at 6 week was not injected, as done in the preliminary and as consequence the retinal and GCL degeneration delayed obtaining similar values of percentage thickness loss found at week 8 (-14.14%) in the preliminary study until week 18-24 (-12.66% and -18.33% respectively) in this ulterior 24-week study. It suggests that we could modulate the timing of degeneration according to the number of injections performed.

The loss rate expressed in microns per mmHg and day extracted from all sectors average was also quantified in both eyes and models compared as standardization. The highest loss rate in the OHT inducted eye (RE) was found in RNFL followed by GCL and finally Retina; so based on IOP (mmHg) the RNFL was the structure earlier and more severe affected. In average of the follow-up the EPI and Ms20/10 models showed exactly the same rate loss in RNFL (-0.005 µm/mmHg/d) but at 24w the loss rate in EPIm was in all the three OCT parameters higher than Ms20/10 (table 7).

**Table 7: Neuro-retinal loss rate measured by optical coherence tomography (OCT) in both ocular hypertensive models (episcleral and microspheres).** EPIm: episcleral sclerosis model; Ms 20/10: microsphere sized 20/10 model; RNFL: Retinal Nerve Fiber Layer; GCL: Ganglion cell layer complex; thickness in microns ($\mu$m); w: week.

| RIGT EYE ALL SECTORS AVERAGE LOSS RATE ($\mu$m)/mmHg/day | | | | | | |
|---|---|---|---|---|---|---|
| TIME | RETINA | | RNFL | | GCL | |
| | EPIm | Ms20/10 | EPIm | Ms20/10 | EPIm | Ms20/10 |
| 8w | 0.002 | 0.004 | -0.005 | -0.008 | -0.002 | -0.005 |
| 12w | 0.004 | -0.006 | -0.005 | -0.002 | -0.005 | -0.003 |
| 18w | -0.008 | -0.011 | -0.007 | -0.008 | -0.006 | -0.004 |
| 24w | -0.003 | -0.002 | -0.003 | -0.002 | -0.003 | -0.002 |
| AVERAGE | **-0.001** | **-0.004** | **-0.005** | **-0.005** | **-0.004** | **-0.003** |

### 4.2.4. Functional neuroretinal analysis by electroretinography

Both OHT models showed progressive decrease in neuro-retinal functionality at week 12 and week 24 in dark and light adapted tests. In general, EPIm showed statistical slower (in latency) or lower (in amplitude) recordings than Ms20/10.

In dark adapted cells from both models, a-wave (from photoreceptors) showed smaller amplitude but faster response than b-wave (from intermediate cells), but both also experienced the lowest recordings within the first phases stimuli by the lightest flash intensities. EPIm showed statistical slower records in a-wave up to week 12 but also in b-wave over the study (figure 13 a,b), as well as lower amplitude in a-wave (figure 13.c). However lower amplitude in b-wave was found with the Ms20/10 model (figure13.d).

Light adapted test by PhNR protocol was performed to specifically study the RGC functionality. In this case the EPIm showed a statistical slower response at week 12 that inverted later (figure 13.e) but also lower amplitude of GCL at any time than Ms20/10 (figure 13.f).

### 4.3 Resembling OHT curve and neuroretinal degeneration using fewer ocular injections with loaded PLGA microspheres. (DEXAMETHASONE).

### 4.3.1 DEXAMETHASONE-Loaded PLGA MS:

#### 4.3.1.1 Dexamethasone-loaded PLGA microspheres characterization

Dexamethasone-loaded PLGA Ms showed a production yield of 77.34 % for the 20-10 $\mu$m fraction. The particle size distribution resulted unimodal, with a mean particle size of 13.13 $\pm$ 0.60 $\mu$m. The drug loading was 60.70 $\pm$ 1.03 $\mu$g Dex/mg Ms, meaning 66.77 $\pm$ 1.14 % of encapsulation efficiency.

SEM images (figure 14) evidenced the presence of spherical and no porous Ms surfaces.

#### 4.3.1.2. *In vitro* release studies

The *in vitro* release profile of dexamethasone from PLGA microspheres showed the typical multiphasic shape release form PLGA microspheres, combining rapid and slow release periods.

From day 0 to day 7 a rapid release occurred leading to a total release of 62 $\mu$g, followed by a slow release period to day 28 with an average release rate of 0.191 $\mu$g/day. After the inclusion of additional amount of microspheres in the release media a new rapid release happened, delivering 94.5 $\mu$g in the following 3 days. Subsequently a second slow release rate of 0.30 $\mu$g/day was observed from day 31 to day 91 of *in vitro* release study. No dexamethasone release was observed from day 91 to end of the study (day 168) (figure 15).

### 4.3.2. Ophthalmological clinical signs and intraocular pressure

Ocular injections were generally well tolerated; the visual axis maintained clear allowing suitable tests. The iridocorneal angle was open, appearing normal by light microscopy. As cons, four animals developed peripheral mild corneal leucomas that did not preclude proper testing and follow-up. One rat developed cataract with pupillary seclusion and ocular hypotension, so this animal was discarded for results.

The IOP increased in both eyes over follow-up. The injected right eye (RE) experienced increase (4 mmHg) since the first week, reached ocular hypertension (OHT) (>20mmHg) at week 5 and maintained significantly higher than the contralateral left eye (LE) up to week 9 (23.22±3.63 vs 19.68±4.03 mmHg, p=0.013). LE reached OHT at week 8 and even out number at later times (16.46±2.11 vs 21.88±4.21 mmHg, p=0.029) (figure 16 a). This model showed a sustained and increasing percentage of OHT in both eyes, although 3 weeks retarded in LE. RE reached the percentage peak of OHT (87.5%) at week 9 and even higher (90%) was quantified in LE at 15-16 weeks (figure 16 b). Most of rats experienced an IOP increase between 6 and 15 mmHg (medium corticoresponse) and only the 5% in average, showed an increase higher than 15 mmHg (high corticoresponse) (figure16 c).

### 4.3.3. Structural neuroretinal analysis by optical coherence tomography

Both eyes experienced a progressive decreased thickness in retina, RNFL and GCL over 6 months follow-up. RE showed lower thickness in all sectors explored except nasal sectors, with statistical differences up to week 8 than LE. At week 24 RE showed smaller thickness in RNFL and GCL but higher values in retina (table 8).

| OCT PARAMETERS | BASELINE Mean±SD | p | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|---|
| CENTRAL | 288.90±16.32 | 0.373 | 267.00±26.90 | -7.58 | 0.431 | 275.33±18.49 | -4.69 | 0.580 | 255.33±19.18 | -11.61 | 0.058 | 260.40±19.61 | -9.87 | 0.190 | 292.50±17.74 | 1.25 | 0.461 | 262.00±14.56 | -9.31 | 0.780 | 260.60±24.05 | -9.80 | 0.774 |
| | 295.20±14.45 | | 276.50±9.02 | -6.33 | | 282.50±24.45 | -4.30 | | 276.00±13.82 | -6.50 | | 276.50±18.06 | -6.76 | | 285.60±9.99 | -3.25 | | 259.80±8.75 | -11.99 | | 263.83±11.08 | -10.63 | |
| INNER INFERIOR | 267.90±8.71 | 0.668 | 257.33±17.31 | -3.94 | 0.254 | 262.33±6.59 | -2.07 | 0.443 | 250.33±6.25 | -6.55 | 0.316 | 255.60±4.61 | -4.59 | 0.575 | 259.83±7.62 | -3.01 | 0.709 | 255.20±13.18 | -4.74 | 0.804 | 249.80±17.65 | -6.76 | 0.980 |
| | 266.30±7.64 | | 267.00±9.12 | 0.26 | | 259.67±4.84 | -2.48 | | 254.17±6.33 | -4.55 | | 253.83±5.30 | -4.91 | | 262.20±12.59 | -1.54 | | 258.60±26.50 | -2.89 | | 250.00±6.63 | -6.12 | |
| OUTER INFERIOR | 254.80±6.59 | 0.955 | 244.50±6.71 | -4.04 | 0.269 | 244.50±2.07 | -4.04 | 0.561 | 240.17±2.63 | -5.74 | 0.254 | 240.20±6.30 | -5.73 | 0.856 | 244.00±5.96 | -4.24 | 0.543 | 239.20±6.26 | -6.12 | 0.875 | 241.40±20.37 | -5.26 | 0.596 |
| | 254.60±8.82 | | 248.50±5.01 | -2.39 | | 245.67±4.27 | -3.50 | | 242.33±3.50 | -4.81 | | 241.00±7.64 | -5.64 | | 247.40±11.52 | -2.83 | | 238.00±15.28 | -6.52 | | 236.50±7.63 | -7.11 | |
| RETINAL THICKNESS | 261.20±7.81 | 0.897 | 245.50±10.60 | -6.01 | 0.209 | 242.00±6.00 | -7.35 | **0.039** | 238.83±4.53 | -8.56 | **0.005** | 240.80±8.46 | -7.81 | 0.278 | 260.50±14.30 | -0.27 | 0.588 | 243.40±7.19 | -6.81 | 0.930 | 241.20±12.55 | -7.66 | 0.812 |
| | 261.60±5.56 | | 255.50±14.78 | -2.33 | | 254.00±10.84 | -2.90 | | 248.33±4.63 | -5.07 | | 246.67±8.33 | -6.05 | | 256.00±11.76 | -2.14 | | 243.80±7.19 | -6.80 | | 243.17±13.84 | -7.05 | |
| OUTER SUPERIOR | 260.00±7.24 | 0.941 | 247.83±8.03 | -4.68 | 0.311 | 242.50±2.73 | -6.73 | 0.072 | 243.17±4.66 | -6.47 | 0.098 | 246.60±5.94 | -5.15 | 0.696 | 258.17±7.78 | -0.70 | 0.634 | 242.20±7.53 | -6.85 | 0.940 | 252.20±12.35 | -3.00 | 0.249 |
| | 259.70±10.39 | | 252.83±8.18 | -2.64 | | 248.83±7.22 | -4.18 | | 247.33±3.07 | -4.76 | | 248.33±7.89 | -4.58 | | 256.00±6.55 | -1.42 | | 242.60±8.79 | -6.58 | | 243.67±10.63 | -6.17 | |
| INNER NASAL | 263.10±7.63 | 0.810 | 250.00±9.57 | -4.97 | 0.139 | 250.33±6.37 | -4.85 | **0.010** | 244.17±6.52 | -7.19 | **0.035** | 247.40±7.47 | -5.97 | 0.751 | 257.67±10.13 | -2.06 | 0.646 | 247.00±7.96 | -6.12 | 0.192 | 251.80±7.49 | -4.29 | 0.748 |
| | 264.00±8.81 | | 258.67±9.11 | -2.01 | | 261.50±5.82 | -0.94 | | 253.50±6.74 | -39.727 | | 249.17±9.90 | -5.95 | | 261.80±18.29 | -0.83 | | 254.80±9.28 | -3.48 | | 250.67±3.55 | -5.05 | |

| Parameter | Group 1 | Group 2 | Group 3 | Group 4 | Group 5 | Group 6 | Group 7 | Group 8 |
|---|---|---|---|---|---|---|---|---|
| OUTER NASAL | 258.70±7.11 / 257.20±7.81; p=0.659 | 246.83±4.62 (-4.58) / 248.00±6.03 (-3.57); p=0.715 | 244.33±2.87 (-5.55) / 245.83±4.95 (-4.42); p=0.536 | 239.33±7.17 (-7.48) / 242.83±2.92 (-5.58); p=0.294 | 244.00±5.24 (-5.68) / 244.50±5.54 (-5.19); p=0.882 | 250.17±6.43 (-3.30) / 250.60±11.45 (-2.57); p=0.938 | 241.20±8.22 (-6.76) / 241.40±9.94 (-6.14); p=0.973 | 248.75±2.63 (-3.85) / 241.00±7.40 (-6.30); p=0.083 |
| INNER TEMPORAL | 261.60±7.80 / 258.80±7.77; p=0.432 | 252.83±9.90 (-3.35) / 257.17±8.88 (-.62); p=0.444 | 248.50±8.01 (-5.00) / 249.67±8.06 (-3.52); p=0.807 | 244.17±5.19 (-6.66) / 245.00±2.09 (-5.33); p=0.723 | 240.60±6.84 (-8.03) / 243.83±7.13 (-6.14); p=0.466 | 255.50±11.45 (-2.33) / 250.40±7.02 (-3.25); p=0.409 | 252.20±11.32 (-3.59) / 247.00±9.56 (-4.56); p=0.455 | 246.40±11.03 (-5.81) / 243.83±9.04 (-5.78); p=0.681 |
| OUTER TEMPORAL | 255.70±7.36 / 256.70±7.10; p=0.761 | 243.83±6.55 (-4.64) / 252.00±5.86 (-1.83); **p=0.046** | 243.50±3.27 (-4.77) / 248.33±2.80 (-3.26); **p=0.021** | 241.50±5.82 (-5.55) / 247.33±2.87 (-3.65); p=0.052 | 245.40±8.41 (-4.03) / 246.17±7.19 (-4.28); p=0.874 | 250.67±8.71 (-1.97) / 251.60±3.84 (-1.99); p=0.830 | 242.60±9.18 (-5.12) / 243.80±8.52 (-5.03); p=0.836 | 248.60±6.50 (-2.78) / 241.83±7.02 (-5.79); p=0.135 |
| TOTAL VOLUME | 1.85±0.04 / 1.86±0.04; p=0.852 | 1.76±0.06 (-4.90) / 1.81±0.03 (-2.41); p=0.127 | 1.76±0.35 (-4.90) / 1.79±0.43 (-3.40); p=0.194 | 1.72±0.02 (-7.23) / 1.76±0.02 (-5.10); **p=0.017#** | 1.74±0.05 (-6.03) / 1.76±0.05 (-5.38); p=0.549 | 1.82±0.05 (-1.67) / 1.82±0.06 (-2.15); p=0.892 | 1.74±0.05 (-6.14) / 1.74±0.06 (-6.34); p=0.999 | 1.71±0.13 (-7.76) / 1.73±0.04 (-6.72); p=0.696 |
| GLOBAL | 49.10±5.34 / 49.00±3.94; p=0.963 | 43.00±3.52 (-12.42) / 49.67±6.80 (1.36); p=0.059 | 40.17±3.06 (-18.18) / 44.33±5.20 (-9.53); p=0.122 | 39.50±3.20 (-19.55) / 41.00±2.82 (-16.32); p=0.411 | 40.40±1.14 (-17.72) / 41.17±1.47 (-19.02); p=0.368 | 40.33±3.67 (-17.86) / 41.60±5.03 (-15.10); p=0.640 | 39.60±4.33 (-19.35) / 38.40±1.51 (-21.63); p=0.579 | 33.40±8.47 (-31.98) / 41.83±6.64 (-14.63); p=0.097 |
| INFERIOR TEMPORAL | 51.30±6.53 / 52.30±7.68; p=0.758 | 44.50±8.26 (-13.25) / 46.50±7.91 (-11.08); p=0.678 | 35.17±6.82 (-31.44) / 42.83±4.26 (-18.10); **p=0.042** | 32.67±9.35 (-36.31) / 38.17±2.99 (-27.01); p=0.200 | 44.40±6.42 (-13.45) / 39.83±4.91 (-31.31); p=0.214 | 42.67±10.15 (-16.82) / 43.40±11.14 (-17.02); p=0.912 | 45.60±27.68 (-11.11) / 38.00±3.39 (-27.34); p=0.559 | 37.80±9.75 (-26.32) / 51.33±13.42 (-1.85); p=0.094 |
| RNFL THICKNESS | 51.70±4.83 / 49.30±10.29; p=0.513 | 43.67±6.86 (-15.53) / 55.33±12.53 (12.23); p=0.073 | 41.67±2.58 (-19.40) / 47.67±5.00 (-3.30); **p=0.026** | 42.67±8.26 (-17.46) / 40.67±7.20 (-17.50); p=0.664 | 45.80±5.35 (-11.41) / 43.17±8.61 (-14.20); p=0.568 | 50.50±8.96 (-2.32) / 47.40±11.88 (-3.85); p=0.633 | 36.40±6.02 (-29.59) / 39.80±9.09 (-19.27); p=0.506 | 43.00±11.42 (-16.83) / 49.33±15.87 (0.06); p=0.476 |

| GCL THICKNESS | Group 1 | Group 2 | Group 3 | Group 4 | Group 5 | Group 6 | Group 7 | Group 8 |
|---|---|---|---|---|---|---|---|---|
| SUPERIOR TEMPORAL | 51.90±6.72 / 52.60±9.30; p=0.849 | 43.83±12.28 (-15.54) / 55.17±13.93 (4.88); p=0.166 | 51.33±11.74 (-1.09) / 48.33±8.54 (-8.11); p=0.624 | 41.50±11.52 (-20.03) / 49.17±7.78 (-6.52); p=0.207 | 41.20±3.76 (-20.62) / 46.00±11.24 (-14.35); p=0.388 | 40.00±13.19 (-22.93) / 41.40±12.03 (-21.29); p=0.859 | 42.60±6.46 (-17.92) / 45.20±4.81 (-14.07); p=0.491 | 30.40±18.91 (-41.43) / 38.83±19.38 (-26.18); p=0.486 |
| SUPERIOR NASAL | 40.30±12.07 / 42.20±11.67; p=0.725 | 41.67±8.95 (3.39) / 43.17±5.34 (2.29); p=0.732 | 34.00±3.74 (-15.63) / 40.17±6.61 (-4.81); p=0.075 | 37.67±5.20 (-6.52) / 37.83±3.43 (-10.35); p=0.949 | 32.80±6.14 (-18.61) / 36.67±8.01 (-15.08); p=0.401 | 33.60±9.65 (-16.63) / 35.20±6.38 (-16.59); p=0.765 | 36.20±3.11 (-10.17) / 34.00±2.73 (-19.43); p=0.270 | 25.40±7.47 (-36.97) / 33.83±4.99 (-19.83); p=0.052 |
| NASAL | 48.20±10.62 / 42.50±5.01; p=0.142 | 40.83±4.11 (-15.29) / 43.67±5.92 (27.576); p=0.359 | 40.50±2.88 (-15.97) / 40.33±5.35 (-5.10); p=0.359 | 39.50±3.56 (-18.04) / 36.50±3.20 (-14.11); p=0.948 | 41.40±6.65 (-14.11) / 39.50±5.32 (-7.50); p=0.156 | 40.67±3.77 (-15.62) / 36.80±9.93 (-13.41); p=0.611 | 37.00±6.28 (-23.24) / 34.60±3.43 (-18.59); p=0.475 | 32.20±10.28 (-33.20) / 39.67±7.36 (-6.66); p=0.194 |
| TEMPORAL | 51.40±11.66 / 55.30±8.97; p=0.413 | 44.00±3.74 (-14.39) / 54.33±9.85 (-1.75); **p=0.037** | 38.67±5.61 (-24.76) / 47.83±7.05 (-13.50); **p=0.032** | 41.17±10.96 (-19.90) / 44.67±5.31 (-19.22); p=0.498 | 39.00±2.55 (-24.12) / 42.83±2.78 (-29.12); **p=0.043** | 40.33±4.32 (-21.54) / 46.60±8.96 (-15.73); p=0.162 | 40.40±4.77 (-21.40) / 40.40±3.13 (-26.94); p=0.999 | 32.80±11.03 (-36.13) / 41.17±4.75 (-25.55); p=0.125 |
| CENTRAL | 22.00±2.70 / 21.70±3.30; p=0.827 | 20.67±4.27 (-6.04) / 20.67±1.86 (4.74); p=0.999 | 18.67±3.14 (-15.13) / 21.00±1.67 (-3.22); p=0.139 | 18.17±2.78 (-17.40) / 20.33±3.01 (-6.31); p=0.225 | 18.00±2.73 (-18.18) / 19.50±3.01 (-11.28); p=0.415 | 21.00±2.82 (-4.55) / 17.60±2.51 (-18.89); p=0.067 | 18.60±2.51 (-15.45) / 10.00±2.82 (-12.44); p=0.819 | 17.40±2.40 (-20.91) / 18.83±1.83 (-13.23); p=0.291 |
| INNER INFERIOR | 27.10±2.13 / 27.50±2.01; p=0.671 | 26.67±3.67 (-1.58) / 27.83±1.72 (1.2); p=0.497 | 26.83±2.04 (-0.99) / 27.17±.98 (-1.2); p=0.726 | 24.83±1.32 (-8.37) / 26.00±1.41 (-5.45); p=0.172 | 25.40±1.14 (-6.27) / 25.67±1.36 (-7.13); p=0.737 | 25.50±1.64 (-5.90) / 25.80±1.30 (-6.18); p=0.749 | 25.60±3.36 (-5.54) / 26.20±1.92 (-4.73); p=0.738 | 22.80±2.38 (-15.87) / 25.83±1.16 (-6.07); **p=0.022** |
| OUTER INFERIOR | 26.10±1.79; p=0.819 | 25.33±1.03 (-2.95); p=0.260 | 25.00±1.54 (-4.21); p=0.172 | 23.50±2.34 (-9.96); p=0.091 | 23.40±2.60 (-10.34); p=0.967 | 24.50±1.87 (-6.13); p=0.531 | 23.00±3.53 (-11.88); p=0.324 | 21.40±3.43 (-18.01); p=0.135 |

| Sector | Eye | 1 | 2 | 3 | 4 | 5 | 6 | 7 | 8 |
|---|---|---|---|---|---|---|---|---|---|
| INNER SUPERIOR | Right eye | 26.30±2.05 | 26.00±0.8 (-1.14) | 26.17±1.16 (-0.49) | 25.33±0.51 (-3.68) | 23.33±2.58 (-12.73) | 25.20±1.64 (-4.18) | 24.80±1.48 (-5.70) | 23.83±1.16 (-9.39) |
| | p | 0.682 | 0.165 | 0.169 | 0.012# | 0.035 | 0.999 | 0.945 | 0.653 |
| | Left eye | 25.70±2.83 | 23.00±2.75 (-10.50) | 22.67±2.80 (-11.78) | 19.50±2.16 (-24.12) | 21.20±2.38 (-17.51) | 23.00±1.78 (-10.51) | 21.20±4.65 (-17.51) | 18.20±0.83 (-29.18) |
| OUTER SUPERIOR | Right eye | 24.70±2.79 | 24.50±2.074 (-0.80) | 23.83±1.47 (-3.52) | 24.17±2.71 (-2.14) | 24.60±2.19 (-0.40) | 24.00±3.79 (-2.83) | 25.00±2.55 (1.21) | 19.40±2.60 (-21.46) |
| | p | 0.710 | 0.892 | 0.064 | 0.528 | 0.464 | 0.615 | 0.636 | 0.104 |
| | Left eye | 25.10±1.85 | 24.67±2.06 (-1.711) | 25.83±1.83 (2.90) | 25.00±1.54 (-0.39) | 25.33±0.81 (0.91) | 22.60±5.12 (-9.96) | 24.20±2.58 (-3.59) | 22.33±2.73 (-11.04) |
| INNER NASAL | Right eye | 25.60±2.31 | 23.00±4.19 (-10.15) | 23.50±2.34 (-8.20) | 21.50±3.93 (-16.01) | 22.80±1.30 (-10.94) | 21.67±3.26 (-15.35) | 22.80±2.28 (-10.94) | 19.00±1.41 (-25.78) |
| | p | 0.916 | 0.215 | 0.260 | 0.401 | 0.728 | 0.498 | 0.551 | 0.293 |
| | Left eye | 25.70±1.82 | 25.33±1.03 (-1.43) | 25.33±2.94 (-1.43) | 23.33±3.26 (-9.22) | 23.17±1.94 (-10.92) | 23.00±2.91 (-10.51) | 21.80±2.77 (-15.18) | 20.17±1.94 (-21.52) |
| OUTER NASAL | Right eye | 26.10±1.66 | 25.17±1.47 (-3.56) | 25.50±1.37 (-2.29) | 23.17±2.48 (-11.22) | 24.40±1.34 (-6.51) | 22.50±3.01 (-13.79) | 23.00±2.44 (-11.88) | 19.75±1.25 (-24.33) |
| | p | 0.558 | 0.169 | 0.679 | 0.178 | 0.596 | 0.559 | 0.550 | 0.337 |
| | Left eye | 25.60±2.06 | 24.17±0.7 (-5.58) | 25.17±1.32 (-1.67) | 24.83±1.32 (-3.00) | 23.83±1.94 (-7.43) | 23.60±2.96 (-7.81) | 23.80±1.48 (-7.03) | 21.00±2.19 (-17.97) |
| INNER TEMPORAL | Right eye | 26.30±2.26 | 24.00±2.96 (-8.74) | 23.33±1.86 (-11.29) | 22.00±2.28 (-16.34) | 22.40±2.60 (-14.83) | 22.33±0.81 (-15.10) | 23.20±2.77 (-11.79) | 21.40±2.30 (-18.63) |
| | p | 0.777 | 0.432 | 0.056 | 0.401 | 0.527 | 0.409 | 0.497 | 0.599 |
| | Left eye | 26.00±2.40 | 25.17±1.83 (-3.19) | 25.83±2.13 (-0.65) | 23.33±2.94 (-10.26) | 23.50±2.88 (-10.64) | 21.20±3.11 (-18.46) | 24.20±1.48 (-6.92) | 20.67±2.16 (-20.50) |
| OUTER TEMPORAL | Right eye | 26.30±2.21 | 25.33±2.42 (-3.68) | 24.67±1.63 (-6.19) | 24.50±2.07 (-6.84) | 24.60±1.14 (-6.46) | 24.00±2.28 (-8.75) | 25.00±2.73 (-4.94) | 21.00±3.53 (-20.15) |
| | p | 0.610 | 0.144 | 0.112 | 0.746 | 0.956 | 0.808 | 0.897 | 0.455 |
| | Left eye | 26.80±2.09 | 27.17±1.47 (1.38) | 26.17±1.32 (-2.35) | 24.00±3.03 (-10.44) | 24.67±2.42 (-8.63) | 23.40±5.36 (-12.69) | 24.80±1.92 (-7.46) | 22.17±0.98 (-17.28) |
| TOTAL VOLUME | Right eye | 0.18±0.01 | 0.17±0.01 (-4.61) | 0.17±0.00 (-5.55) | 0.16±0.01 (-11.11) | 0.16±0.01 (-10.00) | 0.16±0.01 (-8.33) | 0.16±0.01 (-8.89) | 0.13±0.01 (-23.33) |
| | p | 0.999 | 0.347 | 0.207 | 0.201 | 0.259 | 0.736 | 0.820 | 0.057 |
| | Left eye | 0.18±0.00 | 0.17±0.00 (-0.94) | 0.17±0.00 (-1.83) | 0.17±0.00 (-5.55) | 0.17±0.01 (-5.88) | 0.16±0.01 (-10.00) | 0.16±0.01 (-7.78) | 0.15±0.01 (-14.83) |

**Table 8: Structural neuro-retinal analysis by optical coherence tomography (OCT) with microspheres loaded with dexamethasone (MsDex) over 6 months**. MsDex: microspheres loaded with dexamethasone; RNFL: Retinal Nerve Fiber Layer; GCL: Ganglion cell layer complex; thickness in microns (µm); mean±SD (SD: standard deviation); p<0.05 statistical significance; p<0.020# statistical significance with Bonferroni correction for multiple comparisons. Grey cells colored when right eye showed thinner sectors or/and higher percentage loss compared to left eye. Up cell: right eye. Down cell: left eye.

Fluctuations were observed in both eyes and they were more evident in retina and at week 12. RE experienced the same fluctuation tendency in RNFL and GCL, and similarly occurred in LE but 2 weeks postponed (figure 17).

The figure 18 showed the thickness percentage loss over time. RNFL was the parameter that showed the biggest percentage loss in both eyes at every time explored and average; followed by GCL and finally retina. The injected RE showed bigger loss than LE.

The neuroretinal percentage loss by OCT sectors from retina, RFNL and GCL was quantified and loss tendency analyzed (see figures 19, 20, 21, respectively). Variability in retina alteration was observed in RE over time alternating from outer to inner sectors, however, in LE the outer sectors experienced tendency to bigger percentage loss in thickness. The superior-inferior sectors from vertical axis in RNFL were the most often altered. In GCL, the inner sectors showed bigger percentage loss at any time explored in both eyes; and the nasal-temporal sectors from the horizontal axis were the most affected and the inferior sector the least.

The loss rate expressed in microns per mmHg and day extracted from all sectors average was also quantified in both eyes and times. The highest levels of thickness loss were found in Retina at early times (up to 8 weeks) and in RNFL at intermediate and later times. In average, the highest loss rate was found in retina, followed by RNFL and then GCL. RE showed a higher loss rate in RNFL but lower in retina than its no- injected contralateral LE (figure 22).

### 4.3.4. Functional neuroretinal analysis by electroretinography

The RE showed longer latency and smaller amplitude in all the dark adapted (DA) phases explored over time and the biggest decrease in signal was found from baseline to week 12. The light adapted PhNR protocol also showed diminished signal over time and RE smaller compared to LE (see figure 23). RE showed statistical decreased amplitudes compared to LE at week 12 in DA phase 4 (a wave 14.23±9.49 vs 47.38±28.07 µV; p=0.021), phase 7 (b wave: 66.88±26.20 vs 135.13±39.38 µV; p=0.005) and LA-PhNR (b wave: 23.05±21.17 vs 75.70±48.78 µV; p=0.036) and at week 24 in phase 1 (b wave: 34.09±22.14 vs 97.88±45.69 μV; p=0.012) phase 2 (a wave: 25.77±16.84 vs 60.87±32.51 μV, p=0.041) and PhNR (b wave: 22.35±12.81 vs 54.40±30.21 μV; p=0.038).

## 4.4 Resembling OHT curve and neuroretinal degeneration using single ocular injection with loaded PLGA microspheres. (DEXAMETHASONE-FIBRONECTINE).

### 4.4.1 DEXAMETHASONE/FIBRONECTINE-loaded PLGA MS

#### 4.4.1.1. Dexamethasone-Fibronectine loaded PLGA microspheres characterization

Ms showed a production yield of 55.14 % for the 20-10 μm fraction. The particle size distribution resulted unimodal with a mean particle size value of 14.81 ± 0.30 μm. The dexamethasone encapsulation efficiency measurements lead to a 79.13 ± 2.64 % of the initial drug included during the preparation procedure (71.94 ± 2.40 μg Dex/mg Ms). Unfortunately, the fibronectine lability made impossible the real quantification of the protein loaded.

SEM images evidenced the presence of spherical and regular sized Ms with porous and slightly rough surface (figure 24).

#### 4.4.1.2. In vitro release studies

Both dexamethasone and fibronectin *in vitro* release profile showed a multiphasic shape combining rapid and slow release periods typically observed for PLGA microspheres.

Dexamethasone showed an initial release in the first 10 days of the *in vitro* study of 53 μg Dex/mg Ms, followed by a slow release step of 0.0125 μg Dex/mg Ms/day from day 10 to day 38 and another one of 0.0015 μg Dex/mg Ms/day from day 38 to day 77. After that, no Dex release was observed until the end of the study, although the drug remained in the microspheres resulted in almost 20% of the initial charge (Figure 25a). Fibronectin underwent an initial rapid *in vitro* release of 34.6 ng/mg Ms in 7 days, followed by a more sustained release with a total amount of 9.8 ng/mg Ms from day 7 to day 168 (end- point of the release study) (figure 25b).

### 4.4.2. Ophthalmological clinical signs and intraocular pressure

Animals did not show infection, intraocular inflammation, cataract formation or retinal detachment and the surface was well preserved which let correct OCT and ERG acquisitions. The MsDexaFibro floated on the aqueous humor, showing a tendency to localize at the superior iridocorneal angle. This disposition allowed a clear visual axis. One animal developed corneal leucoma and other an iridocorneal synechia that did not preclude proper testing and follow-up. Another third rat developed a focus of vitreoretinitis so this animal was discarded from the study.

It was found a mild and sustained IOP increase over the study. The injected right eye (RE) showed statistical significant higher measurements than the non-injected left eye (LE) up to 6 week but then this difference vanished though both eyes experienced a progressive IOP increase up to 24 week. Both eyes reached ocular hypertension (OHT) (>20mmHg) at week 11. IOP fluctuations were observed over the study (figure 26.a). This model caused a progressive increase of OHT eyes over the study. The highest percentage (88.9%) was reached in both eyes at 20 weeks (figure 26.b). Most rats experienced an IOP increase (in average) between 0 and 6 mmHg (low corticosteroid response). A constant lineal tendency on corticosteroid response was observed in the injected eyes (figure 26.c). Very few animals were high corticosteroid responder (>15 mmHg increase) but fluctuated over the follow-up entering and going out this group with the highest IOP levels (figure 26).

### 4.4.3. Structural neuroretinal analysis by optical coherence tomography

All the three R, RNFL and GCL protocols showed a progressive decrease in neuroretinal thickness over the study. Although very few OCT sectors showed statistical significance (p<0.05) between RE and LE, a tendency to lower thickness measurements was detected in the injected eyes over the study except at week 12 (see table 9). The averaged thickness over the study was also calculated; R experienced the biggest decrease in thickness followed by RNFL and GCL and it occurred in both eyes. However, an increasing fluctuation was detected at week 12 especially in injected RE (figure 27).

**The RNFL parameter showed the highest percentage loss in thickness** at every time explored, then GCL and finally R. The injected RE showed lower thickness percentual loss in RNFL and GCL than the non-injected LE (figure 28). R experienced bigger loss in outer sectors with the STIN averaged loss trend. The inferior sector in RNFL showed the most intense and frequent loss. In GCL the inner sectors showed bigger percentage loss at any time explored in both eyes; from week 8 to the end of the study was observed a loss pattern by sectors in touch and the least affected was the inferior sector (see figure 29 a, b, c). Retina showed the highest loss rate followed by RNFL and finally GCL and the biggest loss rate occurred at early times. Moreover, both eyes lost similar quantity of microns in GCL per every mmHg increased (figure 30).

| OCT PARAMETERS | | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|---|
| RETINA THICKNESS | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | | 263.67±24.66 | 255.17±10.42 | -3.22 | | 256.00±7.29 | -2.90 | | 261.50±9.18 | -0.82 | | 252.00±4.69 | -4.43 | | 259.33±10.39 | -1.6 | | 254.00±11.80 | -3.67 | | 254.78±12.23 | -3.37 | |
| | OUTER INFERIOR | 263.17±7.68 | 248.50±7.89 | -5.57 | 0.295 | 240.83±4.26 | -8.48 | 0.298 | 241.83±3.76 | -8.10 | 0.379 | 237.67±4.32 | -9.69 | 0.192 | 246.83±11.46 | -6.20 | 0.487 | 239.33±5.20 | -9.06 | 0.166 | 236.78±6.85 | -10.03 | 0.776 |
| | | 259.17±5.91 | 244.00±6.10 | -5.85 | | 237.50±6.09 | -8.36 | | 244.50±6.03 | -5.66 | | 241.17±4.36 | -6.95 | | 243.00±6.13 | -6.23 | | 235.67±3.01 | -9.07 | | 237.67±6.19 | -8.30 | |

| OCT PARAMETERS | | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|---|
| RETINA THICKNESS | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | INNER SUPERIOR | 266.67±10.75 | 254.33±8.02 | -4.63 | 0.948 | 242.17±3.97 | -9.18 | 0.160 | 244.00±11.63 | -8.50 | 0.654 | 239.17±6.71 | -10.31 | 0.267 | 263.67±26.64 | -1.12 | 0.155 | 243.33±6.19 | -8.75 | 0.339 | 249.33±9.89 | -6.50 | 0.723 |
| | | 264.17±7.25 | 254.67±9.16 | -3.60 | | 248.67±9.71 | -5.86 | | 247.00±10.83 | -6.49 | | 244.17±7.96 | -7.57 | | 246.00±8.97 | -6.87 | | 248.33±10.52 | -6.00 | | 247.78±8.38 | -6.20 | |

| OCT PARAMETERS | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|
| RETINA THICKNES CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| OUTER SUPERIOR | 265.83±8.42 | 257.33±4.37 | -3.20 | 0.305 | 242.33±3.93 | -8.84 | 0.965 | 244.17±5.20 | -8.14 | 0.165 | 244.17±6.18 | -8.15 | 0.821 | 248.00±7.93 | -6.70 | 0.741 | 249.00±10.00 | -6.33 | 0.854 | 247.33±6.41 | -6.96 | 0.972 |
| | 261.50±6.69 | 253.50±7.50 | -3.06 | | 242.17±8.04 | -7.39 | | 248.67±5.20 | -4.90 | | 245.00±6.23 | -6.31 | | 246.67±5.43 | -5.67 | | 250.00±8.32 | -4.40 | | 247.44±6.67 | -5.38 | |
| INNER NASAL | 268.50±9.57 | 259.67±12.13 | -3.29 | 0.246 | 250.17±4.07 | -6.82 | 0.847 | 249.83±7.96 | -6.95 | 0.221 | 245.50±5.68 | -8.57 | 0.011# | 269.83±35.58 | 0.49 | 0.693 | 253.67±5.92 | -5.52 | 0.277 | 253.00±6.78 | -5.77 | 0.407 |

| OCT PARAMETERS | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|
| RETINA THICKNES CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | 266.17±8.28 | 251.50±10.78 | -5.51 | | 249.50±7.15 | -6.26 | | 256.33±9.25 | -3.69 | | 255.50±5.47 | -4.01 | | 263.67±10.78 | -0.93 | | 258.83±9.28 | -2.76 | | 233.78±67.36 | -12.17 | |
| OUTER NASAL | 263.00±6.36 | 252.67±6.41 | -3.93 | 0.057 | 240.67±3.27 | -8.49 | 0.423 | 244.33±6.50 | -7.09 | 0.559 | 242.33±6.62 | -7.86 | 0.226 | 247.67±9.29 | -5.82 | 0.972 | 243.83±4.07 | -7.79 | 0.949 | 241.44±5.55 | -8.20 | 0.415 |
| | 256.67±2.34 | 244.67±6.50 | -4.68 | | 238.83±4.26 | -6.95 | | 246.33±4.84 | -4.02 | | 246.60±3.36 | -3.92 | | 247.83±6.62 | -3.44 | | 243.67±4.63 | -5.06 | | 244.22±8.27 | -4.85 | |

| OCT PARAMETERS | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|
| RETINA THICKNES CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | 11.64 | 0.404 | 321.17±69.73 | 11.06 | 0.249 | 271.83±19.47 | -6.00 | 0.820 | 274.33±16.03 | -5.13 | 0.242 |
|  | 294.00±7.90 | 274.67±16.65 | -6.57 |  | 275.33±8.26 | -6.35 |  | 282.83±18.28 | -3.79 |  | 265.67±21.30 | -9.64 |  | 285.17±18.39 | -3.00 |  | 268.50±28.97 | -8.67 |  | 283.67±16.55 | -3.51 |  |
| INNER INFERIOR | 273.67± 8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| INNER TEMPORAL | 268.83±7.41 | 251.33±8.34 | -6.51 | 0.208 | 249.17±7.63 | -7.31 | 0.344 | 246.17±6.56 | -8.42 | 0.640 | 246.50±7.87 | -8.31 | 0.511 | 266.17±30.86 | -0.98 | 0.135 | 247.67±7.01 | -7.87 | 0.316 | 247.67±13.70 | -7.87 | 0.984 |
|  | 265.50±5.65 | 256.83±5.53 | -3.27 |  | 245.33±5.57 | -7.59 |  | 247.83±5.35 | -6.65 |  | 244.00±4.34 | -8.10 |  | 245.50±4.04 | -7.53 |  | 251.00±3.29 | -5.46 |  | 247.56±9.67 | -6.76 |  |
| OUTER TEMPORAL | 262.67±7.40 | 250.17±5.91 | -4.76 | 0.889 | 241.33±3.56 | -8.12 | 0.754 | 244.83±3.66 | -6.79 | 0.892 | 243.17±5.81 | -7.42 | 0.615 | 247.00±5.06 | -5.96 | 0.601 | 244.33±6.62 | -6.98 | 0.614 | 245.56±10.33 | -6.51 | 0.840 |

| OCT PARAMETERS | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|
| RETINA THICKNES CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | 11.64 | 0.404 | 321.17±69.73 | 11.06 | 0.249 | 271.83±19.47 | -6.00 | 0.820 | 274.33±16.03 | -5.13 | 0.242 |
|  | 294.00±7.90 | 274.67±16.65 | -6.57 |  | 275.33±8.26 | -6.35 |  | 282.83±18.28 | -3.79 |  | 265.67±21.30 | -9.64 |  | 285.17±18.39 | -3.00 |  | 268.50±28.97 | -8.67 |  | 283.67±16.55 | -3.51 |  |
| INNER INFERIOR | 273.67± 8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
|  | 262.17±5.88 | 250.67±6.15 | -4.39 |  | 240.50±5.24 | -8.26 |  | 245.17±4.58 | -6.48 |  | 244.83±5.31 | -6.61 |  | 245.00±7.54 | -6.54 |  | 246.00±4.20 | -6.17 |  | 246.44±7.89 | -6.00 |  |
| TOTAL VOLUME | 1.89±0.05 | 1.81±0.05 | -4.65 | 0.509 | 1.75±0.03 | -7.63 | 0.814 | 1.75±0.04 | -7.72 | 0.185 | 1.72±0.04 | -8.96 | 0.810 | 1.85±0.14 | -2.10 | 0.258 | 1.76±0.05 | -7.11 | 0.945 | 1.76±0.06 | -7.29 | 0.649 |
|  | 1.88±0.03 | 1.78±0.06 | -4.88 |  | 1.74±0.04 | -7.00 |  | 1.78±0.04 | -5.05 |  | 1.71±0.13 | -8.78 |  | 1.78±0.31 | -4.96 |  | 1.76±0.04 | -6.12 |  | 1.77±0.05 | -5.73 |  |

| OCT PARAMETERS | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|
| RETINA THICKNESS CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
|  | 294.00±7.90 | 274.67±16.65 | -6.57 |  | 275.33±8.26 | -6.35 |  | 282.83±18.28 | -3.79 |  | 265.67±21.30 | -9.64 |  | 285.17±18.39 | -3.00 |  | 268.50±28.97 | -8.67 |  | 283.67±16.55 | -3.51 |  |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| RNFL THICKNESS GLOBAL | 49.83±5.27 | 43.50±5.32 | -12.70 | 0.380 | 44.17±2.71 | -11.35 | 0.542 | 40.83±2.79 | -18.06 | 0.282 | 41.00±2.19 | -17.72 | 0.656 | 55.00±24.00 | 10.37 | 0.326 | 41.67±1.86 | -16.38 | 0.058 | 40.67±3.94 | -18.38 | 0.429 |
|  | 51.00±6.69 | 46.83±7.11 | -8.18 |  | 43.33±1.75 | -15.03 |  | 42.50±2.26 | -16.66 |  | 41.50±1.52 | -18.63 |  | 44.50±6.60 | -12.74 |  | 39.50±1.64 | -22.55 |  | 42.33±4.74 | -17.00 |  |

| OCT PARAMETERS | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|
| RETINA THICKNESS CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
|  | 294.00±7.90 | 274.67±16.65 | -6.57 |  | 275.33±8.26 | -6.35 |  | 282.83±18.28 | -3.79 |  | 265.67±21.30 | -9.64 |  | 285.17±18.39 | -3.00 |  | 268.50±28.97 | -8.67 |  | 283.67±16.55 | -3.51 |  |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| INFERIOR TEMPORAL | 52.33±10.84 | 44.00±5.25 | -15.92 | 0.530 | 38.67±5.75 | -26.10 | 0.755 | 32.00±13.54 | -38.84 | 0.175 | 37.33±3.50 | -28.66 | 0.460 | 49.83±18.63 | -4.77 | 0.325 | 39.33±4.41 | -24.84 | 0.080 | 38.67±5.17 | -26.10 | 0.779 |
|  | 55.00±11.45 | 48.00±14.10 | -12.73 |  | 40.00±8.41 | -27.27 |  | 42.33±10.82 | -23.03 |  | 39.67±6.56 | -27.87 |  | 40.50±11.85 | -26.36 |  | 35.33±2.42 | -35.76 |  | 37.89±6.35 | -31.11 |  |

| OCT PARAMETERS | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|
| | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNESS CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| INFERIOR NASAL | 55.83±8.91 | 50.33±7.58 | -9.85 | 0.798 | 43.50±5.61 | -22.08 | 0.187 | 41.17±5.95 | -26.25 | 0.953 | 41.17±3.92 | -26.26 | 0.386 | 59.33±38.77 | 6.26 | 0.665 | 46.67±14.81 | -16.41 | 0.268 | 42.00±8.60 | -24.77 | 0.999 |
| | 58.17±14.57 | 52.00±13.57 | -10.61 | | 40.00±2.28 | -31.23 | | 41.33±3.33 | -28.94 | | 43.00±3.03 | -26.08 | | 51.83±13.76 | 10.89 | | 39.50±2.17 | -32.10 | | 42.00±8.50 | -27.80 | |

| OCT PARAMETERS | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|
| | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNESS CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| SUPERIOR TEMPORAL | 50.67±14.07 | 41.83±13.24 | -17.45 | 0.207 | 52.50±9.07 | 3.61 | 0.688 | 50.00±8.10 | 1.32 | 0.061 | 48.00±8.51 | -5.27 | 0.103 | 61.17±30.06 | 20.72 | 0.223 | 40.50±5.93 | -20.07 | 0.367 | 39.89±17.21 | -21.27 | 0.334 |
| | 55.33±8.71 | 49.50±4.32 | -10.54 | | 54.50±7.61 | -1.50 | | 42.33±3.72 | -23.49 | | 39.00±8.88 | -29.51 | | 44.67±7.92 | -19.26 | | 44.33±7.97 | -19.88 | | 46.22±8.21 | -16.46 | |

**Table 1**

| OCT PARAMETERS | BASELINE | 2w Mean±SD | 2w %Ch | 2w p | 4w Mean±SD | 4w %Ch | 4w p | 6w Mean±SD | 6w %Ch | 6w p | 8w Mean±SD | 8w %Ch | 8w p | 12w Mean±SD | 12w %Ch | 12w p | 18w Mean±SD | 18w %Ch | 18w p | 24w Mean±SD | 24w %Ch | 24w p |
|---|---|---|---|---|---|---|---|---|---|---|---|---|---|---|---|---|---|---|---|---|---|---|
| RETINA THICKNES CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
|  | 294.00±7.90 | 274.67±16.65 | -6.57 |  | 275.33±8.26 | -6.35 |  | 282.83±18.28 | -3.79 |  | 265.67±21.30 | -9.64 |  | 285.17±18.39 | -3.00 |  | 268.50±28.97 | -8.67 |  | 283.67±16.55 | -3.51 |  |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| SUPERIOR NASAL | 44.67±4.97 | 35.33±10.35 | -20.91 | 0.937 | 37.33±7.53 | -16.43 | 0.413 | 36.50±4.81 | -18.28 | **0.043** | 37.83±12.01 | -15.31 | 0.491 | 53.00±24.12 | 18.64 | 0.079 | 37.83±7.08 | -15.31 | 0.479 | 36.44±9.81 | -18.42 | 0.295 |
|  | 36.33±9.95 | 34.83±10.91 | -4.13 |  | 41.33±8.64 | 13.76 |  | 44.83±7.36 | 23.39 |  | 33.17±10.57 | -8.70 |  | 32.67±8.34 | 10.07 |  | 35.50±3.21 | -2.28 |  | 43.00±15.27 | 18.36 |  |

**Table 2**

| OCT PARAMETERS | BASELINE | 2w Mean±SD | 2w %Ch | 2w p | 4w Mean±SD | 4w %Ch | 4w p | 6w Mean±SD | 6w %Ch | 6w p | 8w Mean±SD | 8w %Ch | 8w p | 12w Mean±SD | 12w %Ch | 12w p | 18w Mean±SD | 18w %Ch | 18w p | 24w Mean±SD | 24w %Ch | 24w p |
|---|---|---|---|---|---|---|---|---|---|---|---|---|---|---|---|---|---|---|---|---|---|---|
| RETINA THICKNES CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
|  | 294.00±7.90 | 274.67±16.65 | -6.57 |  | 275.33±8.26 | -6.35 |  | 282.83±18.28 | -3.79 |  | 265.67±21.30 | -9.64 |  | 285.17±18.39 | -3.00 |  | 268.50±28.97 | -8.67 |  | 283.67±16.55 | -3.51 |  |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| NASAL | 55.00±7.85 | 47.00±8.22 | -14.55 | 0.110 | 42.67±7.94 | -22.41 | 0.335 | 41.83±2.48 | -23.94 | 0.614 | 39.67±3.67 | -27.87 | 0.517 | 53.00±26.71 | -3.63 | 0.485 | 43.83±4.49 | -20.31 | **0.045** | 43.00±5.43 | -21.82 | 0.478 |
|  | 42.50±6.25 | 40.00±5.29 | -5.88 |  | 39.17±2.93 | -7.83 |  | 41.00±3.03 | -3.52 |  | 41.33±4.84 | -2.75 |  | 44.83±6.88 | 5.48 |  | 38.67±3.20 | -9.01 |  | 40.89±6.81 | -3.79 |  |
| TEMPORAL | 43.50±8.22 | 40.67±6.74 | -6.51 | **0.023** | 47.83±5.78 | 9.95 | 0.633 | 42.50±4.64 | -2.29 | 0.770 | 42.00±3.29 | -3.45 | 0.173 | 55.50±19.44 | 27.58 | 0.339 | 41.00±5.87 | -5.75 | 0.908 | 41.00±7.79 | -5.75 | 0.426 |

| OCT PARAMETERS | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|
| RETINA THICKNES CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | 59.00±11.78 | 55.00±11.24 | -6.78 | | 46.50±3.27 | -21.18 | | 43.17±2.86 | -26.83 | | 47.17±7.99 | -20.05 | | 46.67±9.27 | -20.89 | | 41.33±3.56 | -29.95 | | 43.56±5.25 | -26.17 | |
| GCL THI CENTRAL | 22.83±2.71 | 24.00±3.16 | 5.12 | 0.142 | 21.33±3.20 | -6.57 | 0.535 | 17.67±3.01 | -22.60 | 0.017# | 17.33±3.50 | -24.09 | 0.115 | 22.67±5.68 | -0.70 | 0.605 | 17.83±2.14 | -21.90 | 0.477 | 17.89±1.69 | -21.64 | 0.099 |

| OCT PARAMETERS | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|
| RETINA THICKNES CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| CKNESS | 23.00±2.10 | 21.17±2.99 | -7.96 | | 22.33±2.07 | -2.913 | | 22.17±2.40 | -3.60 | | 20.33±2.42 | -11.61 | | 21.17±3.87 | -7.95 | | 19.17±3.87 | -16.65 | | 19.33±1.80 | -15.96 | |
| INNER INFERIOR | 28.83±2.40 | 27.00±1.55 | -6.35 | 0.687 | 27.33±1.51 | -5.20 | 0.743 | 25.00±2.10 | -13.28 | 0.122 | 25.17±1.47 | -12.70 | 0.813 | 28.17±2.99 | -2.28 | 0.227 | 26.17±1.72 | -9.23 | 0.778 | 25.11±2.52 | -12.90 | 0.742 |

| OCT PARAMETERS | | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|---|
| | | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNESS | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | | 28.83±1.72 | 26.67±1.21 | -7.49 | | 27.00±1.90 | -6.34 | | 27.00±2.00 | -6.34 | | 25.33±0.82 | -12.14 | | 26.50±1.05 | -8.08 | | 25.83±2.23 | -10.41 | | 25.56±3.09 | -11.34 | |
| | OUTER INFERIOR | 26.67±1.51 | 26.33±1.21 | -1.27 | 0.196 | 24.17±1.72 | -9.37 | 0.272 | 24.67±1.97 | -7.49 | 0.381 | 24.00±1.27 | -10.01 | 0.670 | 26.00±2.28 | -2.51 | 0.055 | 25.17±2.48 | -5.62 | 0.408 | 24.11±1.69 | -9.60 | 0.657 |

| OCT PARAMETERS | | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|---|
| | | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNESS | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | | 26.83±0.75 | 25.50±0.84 | -4.96 | | 25.33±1.75 | -5.59 | | 25.50±1.05 | -4.95 | | 24.33±1.37 | -9.32 | | 23.33±1.97 | -13.04 | | 24.00±2.19 | -10.55 | | 24.44±1.42 | -8.91 | |
| | INNER SUPERIOR | 27.00±1.26 | 27.00±1.79 | 0.00 | 0.485 | 24.17±0.98 | -10.48 | 0.068 | 22.67±2.42 | -16.03 | 0.811 | 21.50±3.62 | -20.37 | 0.307 | 25.50±4.97 | -5.55 | 0.127 | 22.00±3.35 | -18.52 | 0.665 | 20.56±4.25 | -23.85 | 0.603 |

**Table 1**

| OCT PARAMETERS | | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|---|
| | | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNESS | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | | 26.67±1.75 | 26.33±1.37 | -1.27 | | 25.67±1.51 | -3.74 | | 23.00±2.28 | -13.76 | | 23.33±2.07 | -12.52 | | 21.67±2.66 | -18.74 | | 21.17±3.13 | -20.62 | | 19.67±2.69 | -26.25 | |
| | OUTER SUPERIOR | 25.50±2.43 | 24.00±3.69 | -5.88 | 0.616 | 26.50±0.55 | 3.92 | 0.999 | 25.17±1.33 | -1.29 | 0.852 | 24.83±1.17 | -2.63 | 0.999 | 23.67±5.65 | -7.17 | 0.740 | 25.83±1.60 | 1.29 | 0.518 | 22.00±4.50 | 13.73 | 0.789 |

**Table 2**

| OCT PARAMETERS | | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|---|
| | | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNESS | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | | 25.83±2.14 | 23.00±2.97 | -10.96 | | 26.50±1.98 | 2.59 | | 25.00±1.67 | -3.21 | | 24.83±1.94 | -3.87 | | 24.50±1.98 | -5.14 | | 25.17±1.84 | -2.56 | | 22.44±1.94 | -13.12 | |
| | INNER NASAL | 25.50±1.98 | 26.17±2.23 | 2.63 | 0.315 | 23.67±1.86 | -7.17 | 0.069 | 23.17±3.43 | -9.13 | 0.336 | 22.33±3.08 | -12.43 | **0.045** | 24.50±4.09 | -3.92 | 0.487 | 22.33±1.75 | -12.43 | 0.186 | 22.00±2.24 | -13.73 | 0.663 |

| OCT PARAMETERS | | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|---|
| | | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNESS | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | | 26.33±3.33 | 24.83±2.14 | -5.70 | | 26.00±2.10 | -1.25 | | 24.67±1.21 | -6.30 | | 26.00±2.45 | -1.25 | | 23.17±1.94 | -12.00 | | 24.00±2.28 | -8.85 | | 22.44±2.01 | -14.77 | |
| | OUTER NASAL | 27.17±2.14 | 26.00±1.67 | -4.31 | 0.246 | 25.00±1.55 | -7.98 | 0.263 | 25.83±2.22 | -4.93 | 0.166 | 24.33±1.63 | -10.45 | 0.421 | 24.83±1.72 | -8.61 | **0.047** | 25.17±1.17 | -7.36 | 0.458 | 23.56±1.33 | -13.29 | 0.391 |

| OCT PARAMETERS | | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|---|
| | | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNESS | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | | 26.00±3.16 | 24.83±1.60 | -4.50 | | 25.83±0.75 | -0.65 | | 24.33±1.03 | -6.42 | | 25.00±0.71 | -3.85 | | 22.00±2.53 | -15.38 | | 24.50±1.76 | -5.77 | | 22.89±1.83 | -11.96 | |
| | INNER TEMPORAL | 26.17±1.94 | 26.17±0.75 | 0.00 | 0.570 | 25.17±1.72 | -3.82 | 0.999 | 22.83±3.66 | -12.76 | 0.513 | 22.50±2.59 | -14.02 | 0.716 | 24.67±3.39 | -5.73 | 0.129 | 23.00±0.89 | -12.11 | 0.541 | 20.56±4.75 | -21.44 | 0.555 |

| OCT PARAMETERS | | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|---|
| | | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNES | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | | 26.33±1.37 | 25.83±1.17 | -1.90 | | 25.17±1.33 | -4.40 | | 24.00±2.10 | -8.84 | | 23.00±2.00 | -12.65 | | 21.83±2.48 | -17.09 | | 23.67±2.42 | -10.10 | | 21.56±1.51 | -18.12 | |
| | OUTER TEMPORAL | 26.83±2.64 | 26.83±0.98 | 0.00 | 0.780 | 26.17±0.75 | -2.45 | 0.765 | 25.83±1.84 | -3.72 | 0.590 | 25.00±0.89 | -6.82 | 0.787 | 22.50±3.21 | -16.13 | 0.738 | 25.83±1.33 | -3.73 | 0.518 | 22.44±5.53 | -16.36 | 0.356 |

| OCT PARAMETERS | | BASELINE | 2w | | | 4w | | | 6w | | | 8w | | | 12w | | | 18w | | | 24w | | |
|---|---|---|---|---|---|---|---|---|---|---|---|---|---|---|---|---|---|---|---|---|---|---|---|
| | | | Mean±SD | %Ch | p | Mean±SD | %Ch | p | Mean±SD | %Ch | p | Mean±SD | %Ch | p | Mean±SD | %Ch | p | Mean±SD | %Ch | p | Mean±SD | %Ch | p |
| RETINA THICKNES | CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
| | | 294.00±7.90 | 274.67±16.65 | -6.57 | | 275.33±8.26 | -6.35 | | 282.83±18.28 | -3.79 | | 265.67±21.30 | -9.64 | | 285.17±18.39 | -3.00 | | 268.50±28.97 | -8.67 | | 283.67±16.55 | -3.51 | |
| | INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
| | | 28.00±1.79 | 26.67±1.03 | -4.75 | | 26.00±1.10 | -7.14 | | 26.33±1.21 | -5.96 | | 25.17±1.17 | -10.11 | | 23.00±1.55 | -17.85 | | 25.17±2.04 | -10.11 | | 24.22±0.97 | -13.50 | |
| | TOTAL VOLUME | 0.18±0.01 | 0.18±0.01 | -0.92 | 0.290 | 0.17±0.01 | -4.48 | 0.664 | 0.16±0.01 | -9.02 | 0.360 | 0.16±0.01 | -10.81 | 0.719 | 0.17±0.01 | -5.40 | 0.030 | 0.16±0.01 | -9.03 | 0.813 | 0.15±0.02 | -15.30 | 0.634 |

| OCT PARAMETERS | BASELINE | 2w Mean±SD | %Ch | p | 4w Mean±SD | %Ch | p | 6w Mean±SD | %Ch | p | 8w Mean±SD | %Ch | p | 12w Mean±SD | %Ch | p | 18w Mean±SD | %Ch | p | 24w Mean±SD | %Ch | p |
|---|---|---|---|---|---|---|---|---|---|---|---|---|---|---|---|---|---|---|---|---|---|---|
| RETINA THICKNESS CENTRAL | 289.17±8.42 | 272.67±15.08 | -5.71 | 0.832 | 272.33±21.96 | -5.82 | 0.761 | 261.17±16.33 | -9.68 | 0.056 | 255.50±19.01 | -11.64 | 0.404 | 321.17±69.73 | 11.06 | 0.249 | 271.83±19.47 | -6.00 | 0.820 | 274.33±16.03 | -5.13 | 0.242 |
|  | 294.00±7.90 | 274.67±16.65 | -6.57 |  | 275.33±8.26 | -6.35 |  | 282.83±18.28 | -3.79 |  | 265.67±21.30 | -9.64 |  | 285.17±18.39 | -3.00 |  | 268.50±28.97 | -8.67 |  | 283.67±16.55 | -3.51 |  |
| INNER INFERIOR | 273.67±8.17 | 261.00±10.97 | -4.63 | 0.367 | 263.83±7.68 | -3.59 | 0.100 | 257.00±10.20 | -6.09 | 0.440 | 250.67±6.47 | -8.40 | 0.691 | 274.67±43.52 | 0.36 | 0.421 | 260.17±14.78 | -4.93 | 0.443 | 254.78±13.08 | -6.90 | 0.999 |
|  | 0.18±0.01 | 0.17±0.01 | -4.49 |  | 0.17±0.01 | -3.62 |  | 0.17±0.01 | -6.32 |  | 0.16±0.02 | -9.03 |  | 0.16±0.01 | -12.59 |  | 0.16±0.01 | -9.89 |  | 0.16±0.01 | -13.51 |  |

**Table 9: Structural neuro-retinal analysis by optical coherence tomography (OCT) in microspheres co-loaded with dexamethasone and fibronectine (MsDexaFibro) model.** RNFL: Retina Nerve Fiber Layer; GCL: Ganglion cell layer complex; thickness in microns (µm); mean ± SD (SD: standard deviation); %Ch: percentage change of thickness loss; p<0.050 statistical significance; p<0.020# statistical significance with Bonferroni correction for multiple comparisons; w: week. Grey cells colored when right eye showed thinner sectors or/and higher percentage loss compared to left eye. Up cell: right eye. Down cell: left eye.

### 4.4.4. Functional neuroretinal analysis by electroretinography

The scotopic ERG did not show statistical differences in latency or amplitude but RE experienced a tendency to longer signals in b-wave as well as smaller a-wave and b-wave amplitude compared to LE and over the study. The RE showed maintained scotopic functionality comparing to an increasing functionality of LE at 12w. However, the light adapted PhNR protocol detected smaller amplitudes statistically significant in the injected RE (figures 31 and 32).

## Claims

1. A non-human animal mammalian model of chronic glaucoma wherein the animal has intraocular Polylactic-co-glycolic acid (PLGA), Polylactic acid (PLA) or Polyglicolic acid (PGA) microparticles, having a particle size between 5 µm and 40 µm in the anterior chamber of the eye of the animal in order to induce an increase in intraocular pressure resulting in a chronic gradual progressive neuroretinal degeneration from a functional-structural point of view and wherein the animal is a rodent.

2. A non-human animal mammalian model according to claim 1 wherein de microparticles are loaded with dexamethasone or with a combination of dexamethasone and fibronectin.

3. A non-human animal mammalian model according to any of the previous claims wherein the rodent is a rat, a mouse or a guinea pig.

4. A non-human animal mammalian model according to any of the previous claims wherein chronic glaucoma is open-angle chronic glaucoma.

5. A non-human animal mammalian model according to any of the previous claims wherein the intraocular microparticles have a particle size between 10 μm and 20 μm.

6. A method for preparing a non-human animal mammalian model of chronic glaucoma according to any of the previous claims comprising the intraocular injection in the animal's anterior chamber of the eye of a suspension of PLGA, PLA or PGA microparticles, and having a particle size between 5 μm and 40 μm wherein the animal is a rodent.

7. A method according to claim 6 wherein the microparticles are loaded with dexamethasone or with a combination of dexamethasone and fibronectin.

8. A method according to claim 6 or 7 wherein the rodent is a rat, a mouse or a guinea pig.

9. A method according to any of previous claims 6 to 8 wherein the aqueous suspension has a concentration of microparticles of 0.005% to 20% by weight of the total suspension.

10. A method according to any of previous claims 6 to 9 wherein 1 to 5 microlitres of the aqueous suspension of microparticles are injected in the animal's eye.

11. A method according to any of previous claims 6 to 10 wherein the microparticles have a particle size between 10 μm and 20 μm.

12. Use non-human animal mammalian model according to claim 1 to 5 for the study of physiopathology of glaucoma.

13. Use non-human animal mammalian model according to claim 1 to 5 as a tool for pharmacological, biomaterial and/or surgical studies.


**Patentansprüche**

1. Nicht-menschliches Säugetiermodell eines chronischen Glaukoms, wobei das Tier intraokulare Mikropartikel aus Polymilchsäure-co-glykolsäure (PLGA), Polymilchsäure (PLA) oder Polyglykolsäure (PGA) mit einer Partikelgröße zwischen 5 μm und 40 μm in der vorderen Augenkammer des Tieres aufweist, um eine Erhöhung des Augeninnendrucks zu induzieren, die in einer chronischen graduellen progressiven neuroretinalen Degeneration aus funktionell-struktureller Sicht resultiert, und wobei das Tier ein Nagetier ist.

2. Nicht-menschliches Säugetiermodell nach Anspruch 1, wobei die Mikropartikel mit Dexamethason oder mit einer Kombination aus Dexamethason und Fibronectin beladen sind.

3. Nicht-menschliches Säugetiermodell nach einem der vorhergehenden Ansprüche, wobei das Nagetier eine Ratte, eine Maus oder ein Meerschweinchen ist.

4. Nicht-menschliches Säugetiermodell nach einem der vorhergehenden Ansprüche, wobei das chronische Glaukom ein offenwinkliges chronisches Glaukom ist.

5. Nicht-menschliches Säugetiermodell nach einem der vorhergehenden Ansprüche, wobei die intraokularen Mikropartikel eine Partikelgröße zwischen 10 μm und 20 μm aufweisen.

6. Verfahren zur Vorbereitung eines nicht-menschlichen Säugetiermodells eines chronischen Glaukoms nach einem der vorhergehenden Ansprüche, umfassend die intraokulare Injektion einer Suspension von PLGA-, PLA- oder PGA-Mikropartikeln mit einer Partikelgröße zwischen 5 μm und 40 μm in die vordere Augenkammer des Tieres, wobei das Tier ein Nagetier ist.

7. Verfahren nach Anspruch 6, wobei die Mikropartikel mit Dexamethason oder mit einer Kombination aus Dexamethason und Fibronectin beladen sind.

8. Verfahren nach Anspruch 6 oder 7, wobei das Nagetier eine Ratte, eine Maus oder ein Meerschweinchen ist.

9. Verfahren nach einem der vorhergehenden Ansprüche 6 bis 8, wobei die wässrige Suspension eine Konzentration an Mikropartikeln von 0,005 bis 20 Gew.-% der gesamten Suspension aufweist.

10. Verfahren nach einem der vorhergehenden Ansprüche 6 bis 9, wobei 1 bis 5 Mikroliter der wässrigen Suspension von Mikropartikeln in das Auge des Tieres injiziert werden.

11. Verfahren nach einem der vorhergehenden Ansprüche 6 bis 10, wobei die Mikropartikel eine Partikelgröße zwischen 10 μm und 20 μm aufweisen.

12. Verwendung eines nicht-menschlichen Säugetiermodells nach Anspruch 1 bis 5 für die Untersuchung der Physiopathologie eines Glaukoms.

13. Verwendung eines nicht-menschlichen Säugetiermodells nach einem der Ansprüche 1 bis 5 als ein Werkzeug für pharmakologische, biomaterielle und/oder chirurgische Studien.

**Revendications**

1. Modèle animal mammifère non humain de glaucome chronique dans lequel l'animal a des microparticules intraoculaires d'acide polylactique-co-glycolique (PLGA), d'acide polylactique (PLA) ou d'acide polyglycolique (PGA), ayant une taille de particule comprise entre 5 μm et 40 μm dans la chambre antérieure de l'œil de l'animal afin d'induire une augmentation de la pression intraoculaire entraînant une dégénérescence neurorétinienne progressive chronique d'un point de vue fonctionnel-structurel et dans lequel l'animal est un rongeur.

2. Modèle animal mammifère non humain selon la revendication 1, dans lequel les microparticules sont chargées de dexaméthasone ou d'une combinaison de dexaméthasone et de fibronectine.

3. Modèle animal mammifère non humain selon l'une quelconque des revendications précédentes, dans lequel le rongeur est un rat, une souris ou un cobaye.

4. Modèle animal mammifère non humain selon l'une quelconque des revendications précédentes, dans lequel le glaucome chronique est un glaucome chronique à angle ouvert.

5. Modèle animal mammifère non humain selon l'une quelconque des revendications précédentes, dans lequel les microparticules intraoculaires ont une taille de particule comprise entre 10 μm et 20 μm.

6. Procédé de préparation d'un modèle animal mammifère non humain de glaucome chronique selon l'une quelconque des revendications précédentes, comprenant l'injection intraoculaire dans la chambre antérieure de l'œil de l'animal d'une suspension de microparticules de PLGA, PLA ou PGA, et ayant une taille de particule comprise entre 5 μm et 40 μm, dans lequel l'animal est un rongeur.

7. Procédé selon la revendication 6, dans lequel les microparticules sont chargées de dexaméthasone ou d'une combinaison de dexaméthasone et de fibronectine.

8. Procédé selon la revendication 6 ou 7, dans lequel le rongeur est un rat, une souris ou un cobaye.

9. Procédé selon l'une quelconque des revendications précédentes 6 à 8, dans lequel la suspension aqueuse a une concentration de microparticules de 0,005% à 20% en poids de la suspension totale.

10. Procédé selon l'une quelconque des revendications précédentes 6 à 9, dans lequel 1 à 5 microlitres de la suspension aqueuse de microparticules sont injectés dans l'œil de l'animal.

11. Un procédé selon l'une quelconque des revendications précédentes 6 à 10, dans lequel les microparticules ont une taille de particule comprise entre 10 μm et 20 μm.

12. Utilisation d'un modèle mammifère animal non humain selon les revendications 1 à 5 pour l'étude de la physiopathologie du glaucome.

13. Utilisation d'un modèle mammifère animal non humain selon les revendications 1 à 5 comme outil pour des études pharmacologiques, de biomatériaux et/ou chirurgicales.

**FIG. 1**

**FIG. 2**

FIG. 3

**FIG. 4**

FIG. 5

Thickness loss (%)

TIME

EPI RETINA · Ms 20/10 RETINA
EPI RNFL · Ms 20/10 RNFL
EPI GCL · Ms 20/10 GCL

| OCT PARAMETERS | Average of thickness percentage loss | | | | | | | |
|---|---|---|---|---|---|---|---|---|
| | EPI | | | | Ms 20/10 | | | |
| | 2w | 4w | 6w | 8w | 2w | 4w | 6w | 8w |
| RETINA | -1.77 | -4.99 | -1.34 | -2.61 | 0.08 | -0.35 | -2.59 | -3.56 |
| RNFL | 11.15 | 18.39 | -1.84 | -4.00 | -3.15 | -1.75 | -2.71 | -3.53 |
| GCL | 1.43 | -4.65 | -8.45 | -3.10 | -4.94 | -0.91 | -8.18 | -14.14 |

## FIG. 6

FIG. 7

EPISCLERAL SCLEROSIS MODEL

MICROSPHERES 20/10 MODEL

FIG. 8

**FIG. 9**

FIG. 10

| EPIm (OCTμm) | RETINA | | RNFL | | GCL | |
|---|---|---|---|---|---|---|
| TIME (W) | RE | LE | RE | LE | RE | LE |
| 0 | 253.47 | 253.37 | 46.09 | 45.36 | 25.99 | 26 |
| 8 | 256.22 | 246.86 | 41.42 | 42.08 | 23.91 | 24.22 |
| 12 | 256.41 | 250.80 | 42.53 | 40.78 | 22.01 | 21.53 |
| 18 | 246.88 | 246.50 | 40.57 | 40.45 | 21.30 | 21.11 |
| 24 | 246.87 | 246.46 | 40.41 | 37.20 | 19.74 | 19.85 |

EP 4 084 609 B1

FIG. 11

| OCT PARAMETERS | Average of thickness percentage loss (right eye) | | | | | | | | | |
| | EPIm | | | | | Ms20/10 | | | | |
| | 8w | 12w | 18w | 24w | average | 8w | 12w | 18w | 24w | average |
| RETINA | 0.19 | 0.27 | -3.47 | -3.63 | **-1.66** | 1.01 | -1.98 | -3.86 | -1.77 | **-1.65** |
| RNFL | -9.92 | -7.77 | -11.73 | -12.02 | **-10.36** | -8.98 | -3.77 | -14.18 | -12.33 | **-9.81** |
| GCL | -7.99 | -15.06 | -18.12 | -24.54 | **-16.42** | -10.39 | -10.15 | -12.66 | -18.33 | **-12.88** |

## FIG. 12

**FIG. 13a**

**FIG. 13b**

FIG. 13c

FIG. 13d

FIG. 13e

FIG. 13f

**FIG. 14**

FIG. 15

**FIG. 16a**

**FIG. 16b**

FIG. 16C

**FIG. 17**

| MsDex (OCT µm) TIME (W) | RETINA | | RNFL | | GCL | |
|---|---|---|---|---|---|---|
| | RE | LE | RE | LE | RE | LE |
| 0 | 263.54 | 263.78 | 49.12 | 49.02 | 25.544 | 25.64 |
| 2 | 250.62 | 257.35 | 43.07 | 46.69 | 24.18 | 25.13 |
| 4 | 250.36 | 255.11 | 40.21 | 44.49 | 23.77 | 25.26 |
| 6 | 244.11 | 250.75 | 39.24 | 41.14 | 22.371 | 23.94 |
| 8 | 246.77 | 250 | 40.71 | 41.30 | 22.97 | 23.63 |
| 12 | 258.77 | 257.95 | 41.15 | 41.77 | 23.16 | 22.82 |
| 18 | 247.22 | 247.75 | 39.68 | 38.62 | 23.044 | 23.31 |
| 24 | 248.97 | 246.05 | 33.57 | 42.28 | 20.03 | 21.51 |

RETINA, RNFL, GCL bar chart — Average thickness loss (%). RE -5.30, LE -4.42 (RETINA); RE -19.11, LE -13.02 (RNFL); RE -10.76, LE -7.78 (GCL). Legend: ■ RE ☐ LE

| OCT PARAMETERS | Average of thickness percentage loss MmDex | | | | | | | | | | | | | |
| --- | --- | --- | --- | --- | --- | --- | --- | --- | --- | --- | --- | --- | --- | --- |
| | RE | | | | | | | LE | | | | | | |
| | 2w | 4w | 6w | 8w | 12w | 18w | 24w | 2w | 4w | 6w | 8w | 12w | 18w | 24w |
| RETINA | -4.87 | -4.99 | -7.30 | -6.29 | -1.83 | -6.16 | -5.70 | -2.39 | -3.29 | -4.93 | -5.49 | -2.20 | -6.03 | -6.67 |
| RNFL | -11.86 | -18.07 | -19.69 | -17.15 | -16.25 | -18.97 | -31.84 | -1.52 | -8.92 | -15.86 | -18.67 | -14.71 | -21.04 | -13.52 |
| GCL | -5.26 | -6.92 | -12.35 | -10.15 | -9.12 | -9.76 | -21.77 | -1.97 | -1.54 | -6.49 | -8.40 | -11.06 | -9.04 | -15.97 |

**FIG. 18**

FIG. 19

FIG. 20

FIG. 21

| MsDexa ALL SECTORS AVERAGE LOSS RATE (µm)/mmHg/day | | | | | | |
|---|---|---|---|---|---|---|
| TIME | RETINA | | RNFL | | GCL | |
| | RE | LE | RE | LE | RE | LE |
| 2w | -0.110 | -0.304 | -0.057 | -0.034 | -0.011 | -0.024 |
| 4w | -0.054 | -0.052 | -0.040 | -0.030 | -0.007 | -0.002 |
| 6w | -0.040 | -0.041 | -0.023 | -0.027 | -0.006 | -0.005 |
| 8w | -0.024 | -0.026 | -0.013 | -0.016 | -0.004 | -0.004 |
| 12w | -0.005 | -0.006 | -0.009 | -0.008 | -0.002 | -0.003 |
| 18w | -0.011 | -0.009 | -0.007 | -0.006 | -0.002 | -0.001 |
| 24w | -0.018 | -0.015 | -0.022 | -0.006 | -0.007 | -0.003 |
| AVERAGE | -0.037 | -0.064 | -0.024 | -0.008 | -0.005 | -0.006 |

**FIG. 22**

**FIG. 23a**

FIG. 23b

FIG. 24

FIG. 25a

**FIG. 25b**

**FIG. 26a**

■MsDexaFibro RE  ☐MsDexaFibro LE

**FIG. 26b**

FIG. 26c

FIG. 26d

RIGHT EYE

FIG. 26e

AVERAGE

LEFT EYE

FIG. 26f

FIG. 27

| Ms Dexa Fibro (OCT μm) | RETINA | | RNFL | | GCL | |
|---|---|---|---|---|---|---|
| TIME (W) | RE | LE | RE | LE | RE | LE |
| 0 | 269.05 | 265.89 | 50.26 | 51.04 | 26.27 | 26.42 |
| 2 | 256.40 | 253.96 | 43.23 | 46.59 | 25.94 | 24.98 |
| 4 | 249.20 | 248.20 | 43.81 | 43.54 | 24.83 | 25.53 |
| 6 | 248.14 | 253.35 | 40.69 | 42.49 | 23.64 | 24.66 |
| 8 | 244.96 | 248.77 | 41.00 | 40.69 | 22.99 | 24.14 |
| 12 | 265.00 | 253.57 | 55.26 | 43.66 | 24.72 | 23.01 |
| 18 | 250.35 | 250.66 | 41.54 | 39.16 | 23.70 | 23.63 |
| 24 | 250.02 | 249.26 | 40.23 | 42.27 | 22.02 | 22.50 |

EP 4 084 609 B1

**FIG. 28**

| OCT PARAMETERS | Average of thickness percentage loss MmDexaFibro | | | | | | | | | | | | | |
|---|---|---|---|---|---|---|---|---|---|---|---|---|---|
| | RE | | | | | | | LE | | | | | | |
| | 2w | 4w | 6w | 8w | 12w | 18w | 24w | 2w | 4w | 6w | 8w | 12w | 18w | 24w |
| RETINA | -4.69 | -7.43 | -7.75 | -8.93 | -1.70 | -6.98 | -7.12 | -4.50 | -669 | -4.76 | -6.63 | -4.68 | -5.74 | -6.23 |
| RNFL | -13.98 | -12.11 | -18.43 | -17.79 | -10.74 | -17.01 | -19.50 | -8.41 | -12.90 | -14.15 | -19.08 | -13.53 | -21.65 | -14.85 |
| GCL | -1.10 | -5.36 | -10.03 | -12.43 | -5.80 | -9.86 | -16.18 | -5.40 | -3.30 | -6.57 | -8.63 | -12.79 | -10.55 | -14.74 |

FIG. 29a

FIG. 29b

**FIG. 29c**

EP 4 084 609 B1

| MsDexaFibro ALL SECTORS AVERAGE LOSS RATE (µm)/mmHg/day | | | | | | |
|---|---|---|---|---|---|---|
| TIME | RETINA | | RNFL | | GCL | |
| | RE | LE | RE | LE | RE | LE |
| 2w | -0.223 | -0.295 | -0.137 | -0.122 | -0.005 | -0.035 |
| 4w | -0.129 | -0.174 | -0.046 | -0.082 | -0.0093 | -0.008 |
| 6w | -0.113 | -0.094 | -0.057 | -0.071 | -0.014 | -0.013 |
| 8w | -0.115 | -0.066 | -0.049 | -0.044 | -0.015 | -0.008 |
| 12w | -0.004 | -0.011 | -0.006 | -0.007 | -0.001 | -0.003 |
| 18w | -0.020 | -0.018 | -0.010 | -0.016 | -0.002 | -0.003 |
| 24w | -0.010 | -0.009 | -0.005 | -0.005 | -0.002 | -0.002 |
| AVERAGE | **-0.087** | **-0.095** | **-0.042** | **-0.049** | **-0.006** | **-0.010** |

**FIG. 30**

**FIG. 31c**

**FIG. 31d**

FIG. 31a

FIG. 31b

**FIG. 31e**

**FIG. 31f**

FIG. 32a

FIG. 32b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VIDAL-SANZ et al.** *Prog. Brain. Res.*, 2015, vol. 220, 1-35
- **PEASE et al.** *Invest. Ophtalmol. Vis. Sci.*, 2000, vol. 41 (3), 764-774
- **RITCH et al.** *Ophtalmol. Clin. North Am.*, 2005, vol. 18 (4), 597-609
- **RITCH et al.** *Ophthalmol. Clin. North Am.*, 2005, vol. 18 (4), 597-609
- **DEY et al.** *Cell Transplant*, February 2018, vol. 27 (2), 213-229
- **MUKAI et al.** *PLoS ONE*, 2019, vol. 14 (1), e02087132019
- **NADAL-NICOLÁS et al.** *Invest. Ophtalmol. Vis. Sci.*, 2016, vol. 57 (3), 1183-92
- **ZENG et al.** *Current Eye Research*, 2019
- **MORRISON et al.** *Exp. Eye Res.*, 1997, vol. 64, 84-96
- **VECINO et al.** Glaucoma Basic and Clinical Concepts. 2011, 319-334
- **CHEN et al.** *Invest. Ophtalmol. Vis. Sci.*, 2011, vol. 52 (1), 5-16
- **STRUEBING**. *Prog. Mol. Biol. Trans. Sci.*, 2015
- **BOUHENNI et al.** *J. Biomed. Biotech.*, 2012
- **AGARWAL et al.** *Expert Opin Drug Discov*, 2017
- **BISWAS ; WAN ACTA**. *Ophthalmol*, 2019
- **GARCIA-CABALLERO et al.** *Eur J Pharm Sci*, 2017, vol. 103, 19-26